# EUROPÄISCHE PATENTSCHRIFT

(11) **EP 1 758 864 B1**
(45) Veröffentlichungstag und Bekanntmachung des Hinweises auf die Patenterteilung: **10.04.2013**
(21) Anmeldenummer: 05754335.7
(22) Anmeldetag: 01.06.2005
(51) Int. Cl.: C07D 217/24, C07D 417/14, C07D 401/12, C07D 405/04, C07D 409/04, C07D 401/04, C07D 401/14, C07D 413/14, C07D 405/14, A61K 31/472, A61K 31/4725, A61P 9/00

(54) **Substituierte Tetrahydro-2H-isochinolin-1-on-Derivate, Verfahren zu ihrer Herstellung und ihre Verwendung als Medikamente**
Substituted tetrahydro-2H-isoquinolin-1-one derivatives, method for their preparation, and their use as medicaments
Dérivés substitués de tetrahydro-2H-isoquinolein-1-one, leur procédé de préparation et leur utilisation en tant que médicaments

(30) Priorität: 16.06.2004 DE 102004028973
(43) Veröffentlichungstag der Anmeldung: 07.03.2007
(73) Patentinhaber: Sanofi-Aventis Deutschland GmbH, 65929 Frankfurt am Main (DE)
(72) Erfinder: PEUKERT, Stefan, Arlington, MA 02476 (US); GUESSREGEN, Stefan, 65205 Wiesbaden (DE); HOFMEISTER, Armin, 55278 Dexheim (DE); SCHREUDER, Herman, 65719 Hofheim-Lorsbach (DE); SCHWAHN, Uwe, 65843 Sulzbach (DE)
(86) Internationale Anmeldenummer: PCT/EP2005/005871
(87) Internationale Veröffentlichungsnummer: WO 2005/123687

(56) Entgegenhaltungen:
- EP-A- 0 355 750
- WO-A-99/11624
- WO-A-02/090334
- WO-A1-2005/097750
- US-A- 3 594 380
- J. H. RIGBY ET AL: "Nucleophilic Addition of Silyl Enol Ethers to Aromatic Nitro Compounds: A Facile Synthesis of alpha-Nitroaryl Carbonyl Compounds" JOURNAL OF ORGANIC CHEMISTRY, Bd. 49, Nr. 23, 1984, Seiten 4569-4571, XP002349380 in der Anmeldung erwähnt
- J. H. RIGBY ET AL: "Preparation of Highly Substituted 2-Pyridones by Reaction of Vinyl Isocyanates and Enamines" JOURNAL OF ORGANIC CHEMISTRY, Bd. 54, Nr. 1, 1989, Seiten 224-228, XP002349381

## Beschreibung

Die Erfindung betrifft Verbindungen gemäß der allgemeinen Formel (I), wobei die Definitionen der Substituenten R1, R2, Ar und X im nachfolgenden Text aufgeführt sind, sowie deren physiologisch verträgliche Salze, Verfahren zur Herstellung dieser Verbindungen und deren Verwendung als Arzneimittel.

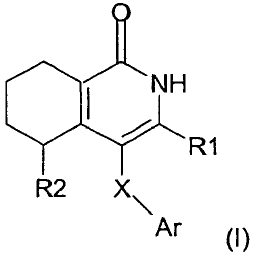

Diese Verbindungen sind Inhibitoren von Poly(ADP-ribose)polymerase (PARP).

### inhibitorische Wirkung gegenüber PARP

Poly(adenosin 5'-diphosphatribose)polymerase [Poly(ADP-ribose)polymerase, PARP], die auch als Poly(ADP-ribose)synthetase (PARS) bekannt ist, ist ein Chromatingebundenes nukleäres Enzym von eukaryoten Zellen, das mit ungefähr 2x10⁵ Molekülen pro Kern vorhanden ist. PARP spielt gemäß jüngsten Forschungsergebnissen in der Pathogenese verschiedener Erkrankungen eine Rolle und kann somit die Inhibition der PARP-Enzymaktivität den Verlauf von Erkrankungen in präklinischen Tiermodellen positiv beeinflussen (Cristina Cosi, Expert Opin. Ther. Patents, 2002, 12, 1047-1071 und L. Virag und C. Szabo, Pharmacol. Rev., 2002, 54, 1-54). Die Poly(ADP-ribose)polymerase kommt in allen eukaryontischen Organismen mit Ausnahme der Hefe vor und ist Teil des Genomüberwachungsnetzwerkes zum Schutz der Erbinformation gegen genotoxische Einflüsse. DNS-Schädigungen induzieren die enzymatische Aktivität der Poly(ADP-ribose)polymerase, was unter physiologischen Bedingungen zur Reparatur der vom Enzym erkannten Fehler der DNS führt. Unter pathologischen Umständen kann es jedoch zu einer starken Aktivierung der Poly(ADP-ribose)polymerase durch radikale Sauerstoffspezies kommen - wie bei der Ischämie, Hypoxie, Reperfusion oder bei Entzündungsprozessen der Fall - wodurch das Enzym große Mengen seines Substrats NAD verbraucht. Diese Verarmung an NAD ist mit ein Grund für den im betroffenen Gewebe zu beobachtenden Untergang von Zellen (die sogenannte Energiekrisen-Hypothese). Der therapeutische Nutzen von PARP-Inhibitoren liegt in der Verhinderung bzw. Minderung dieser NAD-Verarmung im Gewebe. Außer der hier beschriebenen Rolle in der Signalübertragung von oxidativem Stress in Zellen hin zur NAD-Verarmung werden in der aktuellen Literatur weitere zelluläre Funktionen der PARP diskutiert, die ebenfalls eine Rolle für den molekularen Wirkmechanismus von PARP-Inhibitoren unter pathologischen Umständen spielen könnten (A. Chiarugi, Trends Pharmacol. Sci., 2002, 23, 122-129). Unabhängig von dieser offenen Diskussion um den molekularen Wirkmechanismus konnte in mehreren präklinischen Tiermodellen die therapeutische Effektivität von verschiedenen PARP-Inhibitoren gezeigt werden: So zum Beispiel für den akuten Myokardinfarkt, akutes Nierenversagen, cerebrale Ischämie (Schlaganfall), neurodegenerative Erkrankungen (z.B. ein Modell der Parkinsonschen Krankheit), Diabetes, Xenobiotika-induzierte Hepatotoxizität, Arthritis, Schocklunge, septischen Schock und als Sensitivierer in der Chemotherapie neoplastischer Erkrankungen (Zusammengefasst in L. Virag und C. Szabo, Pharmacol. Rev., 2002, 54, 1-54).

Im Einzelnen konnte gezeigt werden, dass PARP-Inhibitoren nicht nur im akuten Herzinfarkt morphologische und funktionelle Verbesserungen bewirken (J. Bowes et al., Eur. J. Pharmacol., 1998, 359, 143-150; L. Liaudet et al., Br. J. Pharmacol., 2001, 133,1424-1430; N. Wayman et al., Eur. J Pharmacol., 2001, 430, 93-100) sondern auch bei der chronischen Herzinsuffizienz sind unter PARP-Inhibitorbehandlung signifikant bessere Herzleistungen gemessen worden (P. Pacher, J. Am. Coll. Cardiol., 2002, 40, 1006-1016). Die Minderdurchblutung, wie sie im infarzierten Herzen Einbußen in der Leistung des Organs durch den Untergang von Zellen bewirkt, steht auch beim Schlaganfall am Anfang der Ereigniskette, die zum Verlusten oder Komplettausfall einzelner Organbereiche und damit -funktionen führt. Demzufolge konnte die Wirksamkeit von PARP-Inhibitoren - neben der genetischen Ablation des PARP-1 Gens (M.J.L. Eliasson et al., Nat. Med., 1997, 10, 1089-1095) - auch in Modellen der cerebralen Ischämie (K. Takahashi et al., L. Cereb. Blood Flow Metab., 1997,11, 1137-1142), der MPTP-induzierten Neurotoxizität (C.Cosi et al., Brain Res., 1996, 729, 264-269) und neuronaler Erregungstoxizität (A.S. Mandir et al., J. Neurosci., 2000, 21, 8005-8011) gezeigt werden. Ein weiterer im Zusammenhang von Herz-Kreislauf-Erkrankungen sehr wichtiger Befund ist die Wirksamkeit von PARP-Inhibition in der ischämisch geschädigten Niere, wo ebenfalls Verbesserungen der Filtrationsleistung des Organs bei PARP-Inhibitor-behandelten im Vergleich zu Plazebo-behandelten Tieren festgestellt werden konnten (D.R. Martin et al., Am. J. Physiol. Regulatory Integrative Comp. Physiol., 2000, 279, R1834-R1840). Im Gegensatz zu den akuten ischämischen Insulten der oben aufgeführten Erkrankungen kommt es in diversen Pathologien zur chronischen PARP-Aktivierung, wie z.B. im Diabetes. Sowohl in präklinischen Modellen das Typ-I-Diabetes (W.L. Suarez-Pinzon et al., Diabetes, 2003, 52, 1683-1688) als auch in solchen des Typ-II-Diabetes konnte die Wirksamkeit von PARP-Inhibitoren nachgewiesen werden (F.G. Soriano et al., Nat. Med., 2001, 7, 108-113; F.G. Soriano et al., Circulation, 2001, 89, 684-691). Die positive Wirkung von PARP-Inhibitoren im Typ-I-Diabetes ist auf deren antiinflammatorische Eigenschaften zurückzuführen, die auch in weiteren präklinischen Modellen, wie der chronischen Colitis (H.B. Jijon et al., Am. J. Physiol. Gastrointest. Liver Physiol., 2000, 279,G641-G651), der Kollagen-induzierten Arthritis (H. Kröger et al., Inflammation, 1996, 20, 203-215) und im septischen Schock (B. Zingarelli et al., Shock, 1996, 5, 258-264) gezeigt werden konnten. Daneben wirken PARP-Inhibitoren sensitivierend auf Tumoren in,der Chemotherapie an Mäusen (L. Tentori et al., Blood, 2002, 99, 2241-2244).

### Stand der Technik

Aus der Literatur ist bereits bekannt (beispielsweise C. Cosi, Expert Opin. Ther. patents, 2002, 12, 1047-1071; Southan et. al., Current Medicinal Chemistry, 2003, 10, 321 - 340), dass viele unterschiedliche Klassen von chemischen Verbindungen als PARP-Inhibitoren verwendet werden können, wie beispielsweise Derivate von Indolen, Benzimidazolen, Isochinolinolen oder Chinazolinonen. Viele der bereits bekannten PARP-Inhibitoren sind Derivate eines bi- oder polycyclischen Grundgerüstes.

Die Verwendung von Isochinolinon-Derivaten als PARP-Inhibitoren wird beispielsweise in WO 02/090334 beschrieben. Die darin beschriebenen Isochinolinon-Derivate basieren ausnahmslos auf einem Grundgerüst, bei dem der zweite Ring des Bicyclus (mit den Kohlenstoffatomen 5, 6, 7 und 8) als Aromat vorliegt, während der die Amidgruppe aufweisende Ring des Bicyclus in 3 und 4-Position gegebenenfalls hydriert sein kann. Auf dem selben aromatischen Grundgerüst basieren die in EP-A 0 355 750 offenbarten Isochinolinon-Derivate, die ebenfalls als PARP-Inhibitoren. verwendet werden können.

Eine Vielzahl von PARP-Inhibitoren lässt sich aus der in WO 99/11624 offenbarten allgemeinen Formel (I) ableiten. Unter anderem ist darin ein Tetrahydro-2H-isochinolin-1-on-Derivat offenbart, das in Position 4 einen Aminoethyl-Substituenten aufweist. Weiterhin kann in Formel (I) der Rest R⁶ auch Aryl sein, wobei nicht erwähnt ist, dass dieser Arylrest gegebenenfalls über weitere Substituenten verfügt. Ein Arylrest für R⁶ ist in WO 99/11624 konkret nur für Beispiele beschrieben, bei denen der über Y definierte Ring des Grundgerüstes sowohl ungesättigt ist als auch Heteroatome aufweist. Die konkrete Kombination eines 5,6,7,8-Tetrahydro-2H-isochinolin-1-on-Derivats, das in Position 4 entweder direkt oder über einen Linker mit einem Arylbeziehungsweise Heteroaryl-Rest substituiert ist, wird jedoch in WO 99/11624 weder offenbart noch nahegelegt. Somit ist es offensichtlich, dass die erfindungsgemäßen Verbindungen nicht durch WO 99/11624 offenbart sind. Verbindungen als solche, die durch WO 99/11624 explizit offenbart sind, sind nicht Gegenstand der vorliegenden Erfindung.

In WO 2005/097750 A1 werden substituierte Pyridone als PARP-Inhibitoren offenbart.

In J. Rigby et al., J. Org. Chem. 1984, 4569-4571 wird im Rahmen eines allgemeinen Syntheseverfahrens für die Cyclisierung von Vinylisocyanaten die Darstellung von 4-Phenyl-5,6,7,8-tetrahydro-2H-isochinolin-1-on durch Cyclokondensation eines Enamins und eines Vinylisocyanats beschrieben. Die in J. Rigby et al. beschriebenen Verbindungen werden nicht mit einer etwaigen Verwendung als Arzneimittel in Verbindung gebracht. Die von J. Rigby et al. explizit offenbarten Verbindungen als solche sind nicht Gegenstand der vorliegenden Erfindung.

Da Krankheiten wie Myokardinfarkt, die durch die Inhibierung von PARP behandelt werden können, eine ernstzunehmende Gefahr für die Gesundheit von Menschen und anderen Säugern darstellt, besteht ein großer Bedarf nach neuen Arzneimitteln, die über ein vorteilhaftes therapeutisches Profil zur Behandlung von solchen Krankenheiten verfügen. Der vorliegenden Erfindung liegt deshalb die Aufgabe zugrunde, neue Verbindungen bereitzustellen, die einen inhibitorischen Effekt auf PARP haben.

Die Aufgabe wird durch die Tetrahydro-2H-isochinolin-1-on-Derivate gemäß der nachstehenden allgemeinen Formel (I) gelöst. 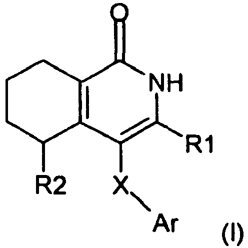 worin bedeuten:
- X: ist eine Einfachbindung, O, S, NH oder N(C₁-C₃-Alkyl);
- R1: ist Wasserstoff, Fluor, Chlor, -CN, Methoxy, -OCF₃ oder C₁-C₃-Alkyl, das gegebenenfalls mit Hydroxy, Chlor, Methoxy oder einem, zwei oder drei Fluoratomen substituiert ist;
- R2: ist Wasserstoff, Fluor, -CN, Hydroxy, Methoxy, -OCF₃, -NH₂, -NH(C₁-C₃-Alkyl), -N(C₁-C₃-Alkyl)₂ oder C₁-C₃-Alkyl, das gegebenenfalls mit Hydroxy, Chlor, Methoxy oder einem, zwei oder drei Fluoratomen substituiert ist;
- R3: ist -(C₁-C₃-Alkyl)-NR4R5, -SO₂NR4R5, -C(O)NR4R5, -C(H)=N-OR9, -C(O)R6, - NHC(O)R6, -(C₁-C₃-Alkyl)-NHC(O)R6, -NHSO₂R6, -(C₁-C₃-Alkyl)-NHSO₂R6 oder -CH(OH)R7;
- R4 und R5: sind unabhängig voneinander ausgewählt aus der Gruppe bestehend aus: Wasserstoff; unsubstituiertes oder zumindest monosubstitutiertes C₁-C₁₀-Alkyl, C₂-C₆-Alkenyl, C₂-C₆-Alkinyl, Aryl, Heteroaryl und Heterocyclyl,
wobei die Substituenten ausgewählt sind aus der Gruppe bestehend aus: Aryl, Heteroaryl, Heterocyclyl, -O-Aryl, Fluor, Chlor, Brom, -CF₃, -OCF₃, -NO₂, -CN, -C(O)R8, -NHC(O)(C₁-C₃-Alkyl), -NH₂, Hydroxy, C₁-C₆-Alkyl, C₁-C₃-Alkoxy, -NH(C₁-C₃-Alkyl), -N(C₁-C₃-Alkyl)₂, -NH-Aryl, -NH-Heteroaryl, -NH-C(O)-Heteroaryl, -SO₂NH₂, -SO₂(C₁-C₃-Alkyl) und -NH-SO₂(C₁-C₃-Alkyl),
und die Aryl-, Heteroaryl- und Heterocyclyl-Fragmente dieser Substituenten können wiederum mit Fluor; Chlor, Brom, Oxo, -CF₃, -OCF₃, -NO₂, -CN, Aryl, Heteroaryl, -NHC(O)(C₁-C₃-Alkyl), -COOH, Hydroxy, C₁-C₃-Alkyl, C₁-C₃-Alkoxy, -SO₂NH₂, -SO₂NH(C₁-C₃-Alkyl), -SO₂N(C₁-C₃-Alkyl)₂, -C(O)NH₂, -C(O)NH(C₁-C₃-Alkyl), -C(O)N(C₁-C₃-Alkyl)₂, -SO₂(C₁-C₃-Alkyl), -NH₂, -NH(C₁-C₃-Alkyl) oder -N(C₁-C₃-Alkyl)₂ zumindest monosubstituiert sein; oder
R4 und R5 bilden zusammen mit dem Stickstoffatom, an das sie gebunden sind, unsubstitutiertes oder zumindest monosubstitutiertes Heterocyclyl,
wobei die Substituenten ausgewählt sind aus der Gruppe bestehend aus: Aryl, Heteroaryl, Heterocyclyl, Oxo, Fluor, Chlor, Brom, -CF₃, -OCF₃, -NO₂, -CN, - C(O)R8, -NHC(O)(C₁-C₃-Alkyl), -NH₂, Hydroxy, C₁-C₃-Alkyl, C₁-c₃-Alkoxy, - NH(C₁-C₃-Alkyl), -N(C₁-C₃-Alkyl)₂, -SO₂NH₂, -SO₂(C₁-C₃-Alkyl) und -NH-SO₂(C₁-C₃-Alkyl),
und von diesen Substituenten können Aryl, Heterocyclyl und Heteroaryl wiederum mit Fluor, Chlor, Brom, Hydroxy, C₁-C₃-Alkyl oder C₁-C₃-Alkoxy zumindest monosubstituiert sein;
- R6: ist unsubstituiertes oder zumindest monosubstituiertes C₁-C₆-Alkyl, Phenyl, Heteroaryl oder Heterocyclyl,
wobei die Substituenten ausgewählt sind aus der Gruppe bestehend aus: Fluor, Chlor, Brom, Aryl, Heterocyclyl, Heteroaryl, -CF₃, -OCF₃, -NO₂, -CN, - C(O)R8, -NHC(O)(C₁-C₃-Alkyl), -NH₂, Hydroxy, C₁-C₃-Alkyl, C₂-C₃-Alkoxy, -O-Heteroaryl, -O-Aryl, -NH(C₁-C₃-Alkyl), -N(C₁-C₃-Alkyl)₂, -SO₂NH₂, -SO₂(C₁-C₃-Alkyl) und -NH-SO₂(C₁-C₃-Alkyl),
und die Aryl-, Heterocyclyl- und Heteroaryl-Fragmente dieser Substituenten können wiederum mit Fluor, Chlor, Brom, Hydroxy, C₁-C₃-Alkyl oder C₁-C₃-Alkoxy zumindest monosubstituiert sein;
- R7: ist ausgewählt aus der Gruppe bestehend aus: Wasserstoff; unsubstituiertes oder zumindest monosubstituiertes C₁-C₆-Alkyl, Phenyl und Heteroaryl,
wobei die Substituenten ausgewählt sind aus der Gruppe bestehend aus: Fluor, Chlor, Brom, -CF₃, -OCF₃, -NO₂, -CN, -C(O)R8, -NHC(O)(C₁-C₃-Alkyl), - NH₂, Hydroxy, C₁-C₃-Alkyl, C₁-C₃-Alkoxy, -NH(C₁-C₃-Alkyl), -N(C₁-C₃-Alkyl)₂, - SO₂NH₂, -SO₂(C₁-C₃-Alkyl) und -NH-SO₂(C₁-C₃-Alkyl);
- R8: ist C₁-C₃-Alkoxy, -O-Phenyl, C₁-C₃-Alkyl, -NH₂, -NH(C₁-C₃-Alkyl), -N(C₁-C₃-Alkyl)₂ oder Phenyl,
und die vorstehenden Phenyl-Fragmente können wiederum mit Fluor, Chlor, Brom, Oxo, -CF₃, -OCF₃, -NO₂, -CN, Aryl, Heteroaryl, -NHC(O)(C₁-C₃-Alkyl), -COOH, Hydroxy, C₁-C₃-Alkyl, C₁-C₃-Alkoxy, -SO₂NH₂, -SO₂NH(C₁-C₃-Alkyl), -SO₂N(C₁-C₃-Alkyl)₂, -C(O)NH₂, -C(O)NH(C₁-C₃-Alkyl), -C(O)N(C₁-C₃-Alkyl)₂, -SO₂(C₁-C₃-Alkyl), -NH₂, -NH(C₁-C₃-Alkyl) oder -N(C₁-C₃-Alkyl)₂ zumindest monosubstituiert sein;
- R9: ist ausgewählt aus der Gruppe bestehend aus:
Wasserstoff; unsubstituiertes oder zumindest monosubstituiertes C₁-C₆-Alkyl und Phenyl,
wobei die Substituenten ausgewählt sind aus der Gruppe bestehend aus: Fluor, Chlor, Brom, Aryl, Heterocyclyl, Heteroaryl, -CF₃, -OCF₃, -NO₂, -CN, - C(O)R8, -NHC(O)(C₁-C₃-Alkyl), C₁-C₃-Alkyl, C₁-C₃-Alkoxy, -NH(C₁-C₃-Alkyl), - N(C₁-C₃-Alkyl)₂, -SO₂NH₂, -SO₂(C₁-C₃-Alkyl) und -NH-SO₂(C₁-C₃-Alkyl), und von diesen Substituenten können Aryl, Heterocyclyl und Heteroaryl wiederum mit Fluor, Chlor, Brom, C₁-C₃-Alkyl oder C₁-C₃-Alkoxy zumindest monosubstituiert sein;
- Ar: ist unsubstituiertes oder zumindest monosubstituiertes Aryl oder Heteroaryl, wobei die Substituenten ausgewählt sind aus der Gruppe bestehend aus: Fluor, Chlor, Brom, -CF₃, -OCF₃, -NO₂, -CN, -C(O)R8, -NH₂, -NHC(O)(C₁-C₆-Alkyl), Hydroxy, C₁-C₆-Alkyl, C₁-C₆-Alkoxy, -CH₂-CH₂-CH₂-, -CH₂-O-C(O)-, -CH₂-C(O)-O-, -CH₂-NH-C(O)-, -CH₂-N(CH₃)-C(O)-, -CH₂-C(O)-NH-, -NH(C₁-C₆-Alkyl), - N(C₁-C₆-Alkyl)₂, -SO₂(C₁-C₆-Alkyl), Heterocyclyl, Heteroaryl, Aryl und R3,
und von diesen Substituenten können Heterocyclyl, Aryl und Heteroaryl wiederum mit C₁-C₆-Alkyl, C₁-C₆-Alkoxy, Fluor, Chlor, Brom, Trifluormethyl, Trifluormethoxy oder OH zumindest monosubstituiert sein;
Heteroaryl ist ein 5 bis 1 0-gliedriger, aromatischer, mono- oder bicyclischer Heterocyclus, der ein oder mehrere Heteroatome ausgewählt aus N, O und S enthält;
Aryl ist ein 5 bis 10-gliedriger, aromatischer, Mono- oder Bicyclus;
Heterocyclyl ist ein 5 bis 10-gliedriger, nicht-aromatischer, mono- oder bicyclischer Heterocyclus, der ein oder mehrere Heteroatome ausgewählt aus N, O und S enthält;
oder ein physiologisch verträgliches Salz davon;
unter der Voraussetzung, dass Ar nicht unsubstituiertes Phenyl ist, wenn X eine Einfachbindung ist und der weiteren Voraussetzung, dass die Verbindung 3-(3methyl-1-oxo-1,2,5,6,7,8-hexahydroisoquinolin-4-yl)benzonitril ausgenommen ist.

Die vorstehenden Bedeutungen der Substituenten R¹ bis R⁸, X, Ar, Heteroaryl, Heterocyclyl und Aryl sind die Grundbedeutungen (Definitionen) der jeweiligen Substituenten.

Die erfindungsgemäßen Tetrahydro-2H-isochinolin-1-on-Derivate gemäß Formel (I) unterscheiden sich in vorteilhafter Weise von bereits bekannten PARP-Inhibitoren auf Isochinolin-1-on-Basis, weil einerseits der zweite Ring des Isochinolinon-Grundgerüsts in hydrierter Form vorliegt und andererseits in Position 4 entweder direkt oder über den Linker X ein Aryl- oder Heteroaryl-Substituent am Isochinolinon-Grundgerüst vorhanden ist. Die allermeisten der bisher bekannten PARP-Inhibitoren auf Isochinolin-1-on-Basis weisen (nahezu) planare, aromatische Grundgerüste auf. Viele dieser planaren PARP-Inhibitoren können DNA-bindende oder DNA-interkalierende Eigenschaften haben, die für das suboptimale Sicherheitsprofil verantwortlich sind (vgl. Southan et al., Current Medicinal Chemistry 2003, 10, 321-340). Da bei den erfindungsgemäßen Verbindungen das Isochinolinon-Grundgerüst in der Position 5,6,7 und 8 aufgrund dessen Sättigung nicht mehr planar ist, weisen diese Moleküle gegenüber den planaren aromatischen Grundgerüsten ein vorteilhaftes Sicherheitsprofil auf. Das Vorhandensein eines Aryl- oder Heteroaryl-Substituenten in Position 4 des Grundgerüstes wirkt sich zusätzlich günstig auf die inhibitorische Wirkung der erfindungsgemäßen Verbindungen gegenüber PARP aus.

Sofern in den Verbindungen gemäß der Formel (I) Gruppen, Fragmente, Reste oder Substituenten wie beispielsweise Aryl, Heteroaryl, Alkyl, Alkoxy usw. mehr als einfach vorhanden sind, haben sie alle unabhängig voneinander die oben aufgeführten Bedeutungen und können somit in jedem (Einzel)-Fall entweder eine identische oder eine voneinander unabhängige Bedeutung haben. Die nachfolgenden Ausführungen gelten für (beispielsweise) Aryl sowie jeden beliebigen anderen Rest unabhängig von dessen Bezeichnung als Arylgruppe, -substituent, -fragment oder -rest. Ein weiteres Beispiel ist die -N(C₁-C₃-alkyl)₂ Gruppe, in der die beiden Alkylsubstituenten entweder identisch oder unterschiedlich sein können(beispielsweise zweimal Ethyl oder einmal Propyl und einmal Methyl).

Sofern in den vorstehenden Definitionen für Verbindungen gemäß Formel (I) ein Substituent, beispielsweise Aryl, unsubstituiert oder zumindest monosubstituiert mit einer Gruppe von weiteren Substituenten, beispielsweise C₁-C₆-Alkyl, C₁-C₆-Alkoxy, Halogen usw., sein kann, so gilt für diejenigen Fälle, in denen eine Mehrfachsubstitution von Aryl vorliegt, dass die Auswahl aus der Reihe von weiteren Substituenten unabhängig voneinander erfolgt. Somit sind beispielsweise bei einer Zweifachsubstitution von Aryl sämtliche Kombinationen der weiteren Substituenten mit umfasst. Aryl kann somit beispielsweise zweifach-substituiert mit Ethyl sein, Aryl kann jeweils monosubstituiert mit Methyl und Ethoxy sein, Aryl kann jeweils monosubstituiert mit Ethyl und Fluor sein, Aryl kann zweifach-substituiert mit Methoxy sein, usw..

Alkylreste können entweder linear oder verzweigt, acyclisch oder cyclisch sein. Dies trifft auch zu, sofern sie Teil einer anderen Gruppe wie beispielsweise Alkoxygruppen (C₁-C₁₀-Alkyl-O-), Alkoxycarbonylgruppen oder Aminogruppen sind oder wenn sie substituiert sind.

Beispiele für Alkylgruppen sind: Methyl, Ethyl, Propyl, Butyl, Pentyl, Hexyl, Heptyl, Octyl, Nonyl oder Decyl. Dabei sind sowohl die n-Isomere dieser Reste als auch Isopropyl, Isobutyl, Isopentyl, sec-Butyl, tert-Butyl, Neopentyl, 3,3-Dimethylbutyl usw. mit umfasst. Sofern nicht anders beschrieben, beinhaltet der Begriff Alkyl darüber hinaus auch Alkylreste, die unsubstituiert oder gegebenenfalls mit einem oder mehreren weiteren Resten substituiert sind, beispielsweise 1, 2, 3 oder 4 identische oder unterschiedliche Reste, wie beispielsweise Aryl, Heteroaryl, Alkoxy oder Halogen. Dabei können die zusätzlichen Substituenten in jeder beliebigen Position des Alkylrestes auftreten. Weiterhin umfasst der Begriff Alkyl auch Cycloalkyl sowie Cycloalkyl-alkyl- (Alkyl, das wiederum mit Cycloalkyl substituiert ist), wobei Cycloalkyl mindestens 3 Kohlenstoffatome aufweist. Beispiele für Cycloalkylreste sind: Cyclopropyl, Cyclobutyl, Cyclopentyl, Cyclohexyl, Cycloheptyl, Cyclooctyl, Cyclononyl und Cyclodecyl. Gegebenenfalls kann es sich auch um polycyclische Ringsysteme handeln, wie Decalinyl, Norbornanyl, Bornanyl oder Adamantanyl. Die Cycloalkylreste können unsubstituiert oder gegebenenfalls mit einem oder mehreren weiteren Resten substituiert sein, wie vorstehend bei den Alkylresten beispielhaft aufgeführt.

Beispiele für Alkenyl- und Alkinylgruppen sind: Vinyl, 1-Propenyl, 2-Propenyl (Allyl), 2-Butenyl, 2-Methyl-2-propenyl, 3-Methyl-2-butenyl, Ethinyl, 2-Propinyl (Propargyl), 2-Butinyl oder 3-Butinyl. Der Begriff Alkenyl umfasst hier ausdrücklich auch Cycloalkenylreste sowie Cycloalkenyl-Alkylreste (Alkyl, das mit Cycloalkenyl substituiert ist), die mindestens drei Kohlenstoffatome enthalten. Beispiele für Cycloalkenyl sind: Cyclopentenyl, Cyclohexenyl, Cycloheptenyl und Cyclooctenyl.

Die Alkenylreste können ein bis drei konjugierte oder nicht konjugierte Doppelbindungen (also auch Alk-dienyl- sowie Alk-trienylreste), vorzugsweise eine Doppelbindung in einer geraden oder verzweigten Kette aufweise, für Alkinylreste gilt das gleiche für die Dreifachbindungen. Die Alkenyl- und Alkinylreste können unsubstituiert oder gegebenenfalls mit einem oder mehreren weiteren Resten substituiert sein, wie vorstehend bei den Alkylresten beispielhaft aufgeführt.

Sofern nicht anders ausgeführt, können die vorgenannten Aryl-, Heteroaryl- und Heterocyclylreste sowohl unsubstituiert als auch einen oder mehrere, beispielsweise 1, 2, 3 oder 4 weitere, der vorgenannten Substituenten in jeder beliebigen Position aufweisen. Beispielsweise kann in monosubstituierten Phenylresten der Substituent in der Position 2, 3 oder 4, in disubstituierten Phenylresten können die Substituenten in der 2,3-Position, 2,4-Position, 2,5-Position, 2,6-Position, 3,4-Position oder 3,5-Position sein. In dreifach substituierten Phenylresten können die Substituenten in der 2,3,4-Position, 2,3,5-Position, 2,3,6-Position, 2,4,5-Position, 2,4,6-Position oder 3,4,5-Position sein. In vierfach substituierten Phenylresten können die Substituenten in der 2,3,4,5-Position, der 2,3,4,6-Position oder in der 2,3,5,6-Position sein.

Die vorgenannten beziehungsweise die nachfolgenden Definitionen, die sich auf einwertige Reste beziehen, gelten genauso für zweiwertige Reste wie Phenylen, Naphthylen oder Heteroarylen. Diese zweiwertigen Reste (Fragmente) können mit dem benachbarten Gruppen für jedes beliebige Ring-Kohlenstoffatom verknüpft sein. Im Falle von Phenylenresten kann dies in der 1,2-Position (Ortho-Phenylen), 1,3-Position (meta-Phenylen) oder 1,4-Position (Para-Phenylen) sein. Im Falle eines 5-gliedrigen Aromaten, der ein Heteroatom enthält, wie beispielsweise Thiophen oder Furan können die beiden freien Bindungen in der 2,3-Position, 2,4-Position, 2,5-Position oder der 3,4-Position sein. Ein zweiwertiger Rest, der von einem 6-gliedrigen Aromaten mit einem Heteroatom abgeleitet ist, wie beispielsweise Pyridin, kann ein 2,3-, 2,4-, 2,5-, 2,6-, 3,4- oder 3,5-Pyridindiylrest sein. Im Falle von unsymmetrischen zweiwertigen Resten beinhaltet die vorliegende Erfindung auch alle Positionsisomere, d.h. im Falle von beispielsweise einem 2,3-Pyridindiylrest ist die Verbindung, in der die eine benachbarte Gruppe sich in der Position 2 und die andere benachbarte Gruppe in der Position 3 sich befindet, genauso wie die Verbindung, in der die eine benachbarte Gruppe in der Position 3 und die andere benachbarte Gruppe sich in der Position 2 befindet, mit umfasst.

Soweit nicht anders aufgeführt, sind Heteroarylreste, Heteroarylenreste, Hetetrocyclylreste und Heterocyclylenreste sowie Ringe, die durch zwei an Stickstoff gebundene Gruppen gebildet werden, vorzugsweise abgeleitet von vollständig gesättigten, teilweise oder ganz ungesättigten Heterocyclen (d.h. Heterocycloalkane, Heterocycloalkene, Heteroaromaten), die 1, 2, 3 oder 4 Heteroatome enthalten, die sowohl unterschiedlich als auch gleich sein können. Vorzugsweise sind sie von Heterocyclen abgeleitet, die 1, 2 oder 3, besonders bevorzugt 1 oder 2, Heteroatome enthalten, die gleich oder unterschiedlich sein können. So lange nicht anders aufgeführt, sind die Heterocyclen mono- oder polycyclisch, zum Beispiel monocyclisch, bicyclisch oder tricyclisch. Vorzugsweise sind sie monocyclisch oder bicyclisch. Vorzugsweise sind es 5-gliedrige, 6-gliedrige oder 7-gliedrige Ringe, besonders bevorzugt 5-gliedriger oder 6-gliedrige Ringe. Im Fall von polycyclischen Heterocyclen mit 2 und mehr Heteroatomen können diese alle im gleichen Ring vorkommen oder auf mehrere Ringe verteilt sein.

Als Heteroaryl werden in der vorliegenden Erfindung Reste bezeichnet, die von monocyclischen oder bicyclischen, aromatischen Heterocyclen abgeleitet sind. Beispiele für Heteroaryl sind: Pyrrolyl, Furanyl (=Furyl), Thiophenyl (=Thienyl), Imidazolyl, Pyrazolyl, 1,2,3-Triazolyl, 1,2,4-Triazolyl, 1,3-Oxazolyl (=Oxazolyl), 1,2-Oxazolyl (=Isoxazolyl), Oxadiazolyl, 1,3-Thiazolyl (=Thiazolyl), 1,2-Thiazolyl (=Isothiazolyl), Tetrazolyl, Pyridinyl (=Pyridyl) Pyridazinyl, Pyrimidinyl, Pyrazinyl, 1,2,3-Triazinyl, 1,2,4-Triazinyl, 1,3,5-Triazinyl, 1,2,4,5-Tetrazinyl, Indazolyl, Indolyl, Benzothiophenyl, Benzofuranyl, Benzothiazolyl, Benzimidazolyl, Chinolinyl, Isochinolinyl, Chinazolinyl, Chinoxalinyl, Phthalazinyl, Thienothiophenyl, 1,8-Naphthyridinyl, andere Naphthyridinyle, Pteridinyl oder Thiazolo[3,2-b][1,2,4]-tiazolyl. Sofern es sich nicht um monocyclische Systeme handelt, ist bei jedem der vorgenannten Heteroaryle für den zweiten Ring auch die gesättigte Form (Perhydroform) oder die teilweise ungesättigte Form (beispielsweise die Dihydroform oder Tetrahydroform) oder die maximal ungesättigte (nicht-aromatische) Form mit umfasst, sofern die jeweiligen Formen bekannt und stabil sind. Die Bezeichnung Heteroaryl umfasst somit in der vorliegenden Erfindung beispielsweise auch bicyclische Reste, in denen sowohl beide Ringe aromatisch sind als auch bicyclische Reste, in denen nur ein Ring aromatisch ist. Solche Beispiele für Heteroaryl sind: 3H-Indolinyl, 2(1H)-Chinolinonyl, 4-Oxo-1,4-dihydrochinolinyl, 2H-1-Oxoisochinolyl, 1,2-Dihydrochinolinyl, 3,4-Dihydrochinolinyl, 1,2-Dihydroisochinolinyl, 3,4-Dihydroisochinolinyl, Chromonyl, Chromanyl, 1,3-Benzodioxolyl, Oxindolyl, 1,2,3,4-Tetrahydroisochinolinyl, 1,2,3,4-Tetrahydrochinolinyl, 5,6-Dihydrochinolyl, 5,6-Dihydroisochinolyl, 5,6,7,8-Tetrahydrochinolinyl oder 5,6,7,8-Tetrahydroisochinolyl.

Als Heterocyclyl werden in der vorliegenden Erfindung Reste bezeichnet, die von monocyclischen oder bicyclischen, nicht-aromatischen Heterocyclen abgeleitet sind. Unter nicht-aromatischen Heterocyclen werden nachfolgend insbesondere Heterocycloalkane (vollständig gesättigte Heterocyclen) sowie Heterocycloalkene (teilweise ungesättigte Heterocyclen) verstanden. Im Fall der Heterocycloalkene werden auch Verbindungen mit zwei oder mehreren Doppelbindungen mit umfasst, die gegebenenfalls auch miteinander konjugiert sein können. Beispiele für Heterocyclyl sind: Pyrrolidinyl, Piperidinyl, Piperazinyl, Imidazolidinyl, Pyrazolidinyl, Isothiazolidinyl, Thiazolidinyl, Isoxazolidinyl, Oxazolidinyl, Tetrahydrofuranyl, Tetrahydrothiophenyl, 1,3-Dioxolanyl, 1,4-Dioxinyl, Pyranyl, Thiopyranyl, Tetrahydro-1,2-Oxazinyl, Tetrahydro-1,3-Oxazinyl, Morpholinyl, Thiomorpholinyl, 1,2-Thiazinyl, 1,3-Thiazinyl, 1,4-Thiazinyl, Azepinyl, 1,2-Diazepinyl, 1,3-Diazepinyl, 1,4-Diazepinyl, 1,3-Oxazepinyl, 1,3-Thiazepinyl, Azepanyl, 2-Oxo-azepanyl, 1,2,3,4- Tetrahydropyridinyl, 1,2-Dihydropyridinyl, 1,4-Dihydropyridinyl, 1,2,3,6-Tetrahydropyridinyl, 4(3H)-pyrimidonyl, 1,4,5,6-Tetrahydropyrimidinyl, 2-Pyrrolinyl, 3-Pyrrolinyl, 2-Imidazolinyl, 2-Pyrazolinyl, 3,4-Dihydro-2H-pyranyl, Dihydrofuranyl, 7-Oxabicyclo[2.2.1]heptenyl, Dihydrothiophenyl und Dihydrothiopyranyl. Der Grad der Sättigung von heterocyclischen Gruppen ist in der jeweiligen Definition angezeigt.

Substituenten, die von diesen Heterocyclen abgeleitet sind, können über jedes geeignete Kohlenstoffatom verknüpft sowie mit weiteren Substituenten versehen sein. Reste, die von stickstoffhaltigen Heterocyclen abgeleitet sind, können am entsprechenden Stickstoffatom ein Wasserstoffatom oder einen anderen Substituenten aufweisen. Beispiele umschließen Pyrrol, Imidazol, Pyrrolidin, Morpholin, Piperazinreste usw.. Diese stickstoffhaltigen heterocyclischen Reste können auch über das Ring-Stickstoffatom gebunden sein, insbesondere wenn der entsprechende heterocyclische Rest an ein Kohlenstoffatom gebunden ist. Beispielsweise kann ein Thienylrest als 2-Thienyl oder 3-Thienyl vorliegen, ein Piperidinylrest als 1-Piperidinyl (Piperidino), 2-Piperidinyl, 3-Piperidinyl oder 4-Piperidinyl. Geeignete stickstoffhaltige Heterocyclen können auch als N-Oxide oder als quarternäre Salze, die ein Gegenion aufweisen, das von einer physiologisch annehmbaren Säure abgeleitet ist, vorliegen. Beispielsweise können Pyridylreste als Pyridin-N-oxide vorliegen. Geeignete schwefelhaltige Heterocyclen können auch als S-Oxid oder S-S-Dioxid vorliegen.

Als Aryl werden in der vorliegenden Erfindung Reste bezeichnet, die von monocyclischen oder bicyclischen Aromaten abgeleitet sind, die keine Ringheteroatome enthalten. Sofern es sich nicht um monocyclische Systeme handelt, ist bei der Bezeichnung Aryl für den zweiten Ring auch die gesättigte Form (Perhydroform) oder die teilweise ungesättigte Form (beispielsweise die Dihydroform oder Tetrahydroform), sofern die jeweiligen Formen bekannt und stabil sind. Die Bezeichnung Aryl umfasst in der vorliegenden Erfindung auch beispielsweise bicyclische Reste, in denen sowohl beide Ringe aromatisch sind als auch bicyclische Reste, in denen nur ein Ring aromatisch ist. Beispiele für Aryl sind: Phenyl, Naphthyl, Indanyl, 1,2-Dihydronaphthenyl, 1,4-Dihydronaphthenyl, Indenyl oder 1,2,3,4-Tetrahydronaphthyl.

Arylalkyl (wie Aryl-(C₁-C₆-alkyl)-) bedeutet, dass ein Alkylrest (wie C₁-C₆-Alkyl) wiederum mit einem Arylrest substituiert ist. Heteroarylalkyl (wie Heteroaryl-(C₁-C₆-alkyl)-) bedeutet, dass ein Alkylrest (wie C₁-C₆-Alkyl) wiederum mit einem Heteroarylrest substituiert ist. Heterocyclylalkyl (wie Heterocyclyl-(C₁-C₆-alkyl)-) bedeutet, dass ein Alkylrest (wie C₁-C₆-Alkyl) wiederum mit einem Heterocyclylrest substituiert ist. Für die Definitionen und Substitutionsmöglichkeiten von Alkyl, Heteroaryl, Heterocyclyl und Aryl wird auf die vorstehenden Definitionen verwiesen.

Sofern in der vorliegenden Erfindung bivalente Substituenten definiert sind, bei denen sich die beiden freien Valenzen nicht am (Kohlenstoff)-Atom befinden, wie beispielsweise -CH₂-CH₂-CH₂- (Propylen) oder -CH₂-O-C(O)- im Fall von Ar, so bedeutet dies, dass dieser bivalente Substituent mit beiden freien Valenzen an denselben Rest (beispielsweise Ar) gebunden ist und dabei die Bildung eines (weiteren) Cyclus bewirkt. Der bivalente Substituent ist in der Regel an verschiedene Atome des entsprechenden Restes gebunden, kann gegebenenfalls aber - sofern dies möglich ist - auch mit den beiden freien Valenzen an dasselbe Atom des Restes gebunden sein.

Halogen ist Fluor, Chlor, Brom oder Iod, ist bevorzugt Fluor, Chlor, oder Brom, ist besonders bevorzugt Fluor oder Chlor.

Die vorliegende Erfindung schließt alle stereo-isomeren Formen von Verbindungen gemäß der Formel (I) mit ein. Asymmetrische Kohlenstoffatome in Verbindungen gemäß der Formel (I) können unabhängig voneinander S-Konfigurationen oder R-Konfigurationen aufweisen. Die Erfindung schließt alle möglichen Enantiomere und Diastereomere und Mischungen aus zwei oder mehr Stereo-Isomeren, zum Beispiel Mischungen aus Enantiomeren und/oder Diastereomeren, in allen Mengen und Verhältnissen mit ein. Somit können Verbindungen der vorliegenden Erfindung, die als Enantiomere existieren, in Enantiomeren-Reinform, sowohl als rechtsdrehende als auch linksdrehende Antipoden, in Form von Racematen und in Form von Mischungen der zwei Enantiomeren in allen Verhältnissen vorliegen. Im Fall von Cis/Trans-Isomeren schließt die Erfindung sowohl die Cis-Form, als auch die Trans-Form sowie Mischungen von diesen Formen in allen Verhältnissen mit ein. All diese Formen sind Gegenstand der vorliegenden Erfindung. Die Herstellung der einzelnen Stereo-Isomeren kann, falls gewünscht, durch Trennung eines Gemisches mit herkömmlichen Methoden, zum Beispiel durch Chromatographie oder Kristallisation, durch die Verwendung von stereochemisch einheitlichen Ausgangsmaterialien zur Synthese oder durch stereoselektive Synthese erfolgen. Alternativ kann auch einer Derivatisierung vor der Trennung der Stereo-Isomeren durchgeführt werden. Die Trennung eines Gemisches aus Stereo-Isomeren kann mit den Verbindungen der Formel (I) oder mit den entsprechenden Zwischenprodukten während der Synthese durchgeführt werden. Weiterhin umfasst die vorliegende Erfindung auch alle tautomeren Formen von Verbindungen gemäß Formel (I), insbesondere Keto-/Enoltautomerie, d.h. die entsprechenden Verbindungen können entweder in ihrer Keto-Form oder in ihrer Enolform oder in Mischungen davon in allen Verhältnissen vorliegen.

Sofern die Verbindungen gemäß Formel (I) eine oder mehrere saure oder basische Gruppen enthalten, umfasst die vorliegende Erfindung auch die entsprechend physiologisch oder toxikologisch verträglichen Salze.

Physiologisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein physiologisch verträgliches Anion oder Kation aufweisen. Geeignete physiologisch verträgliche Säureadditionssalze der erfindungsgemäßen Verbindungen sind Salze anorganischer Säuren, wie Salzsäure, Bromwasserstoff-, Phosphor-, Metaphosphor-, Salpeter-, Sulfon- und Schwefelsäure sowie organischer Säuren, wie z.B. Essigsäure, Theophyllinessigsäure, Methylen-bis-b-oxynaphthon-, Benzolsulfon-, Benzoe-, Zitronen-, Ethansulfon-, Salicyl-, Fumar-, Glucon-, Glykol-, Isethion-, Milch-, Lactobion-, Malein-, Äpfel-, Methansulfon-, Bernstein-, p-Toluolsulfon-, Wein- und Trifluoressigsäure. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze).

Salze mit einem nicht pharmazeutisch verträglichen Anion gehören ebenfalls in den Rahmen der Erfindung als nützliche Zwischenprodukte für die Herstellung oder Reinigung pharmazeutisch verträglicher Salze und/oder für die Verwendung in nichttherapeutischen, zum Beispiel in-vitro-Anwendungen.

Sofern die Verbindungen der Formel (I) gleichzeitig saure und basische Gruppen in demselben Molekül enthalten, schließt die vorliegende Erfindung - zusätzlich zu den vorher aufgeführten Salzformen - auch innere Salze oder Betaine (Zwitterionen) mit ein.

Die entsprechenden Salze der Verbindungen gemäß Formel (I) können durch herkömmliche Methoden, die dem Fachmann bekannt sind, erhalten werden, beispielsweise durch Umsetzung mit einer organischen oder anorganischen Säure oder Base in einem Lösungsmittel oder Dispergiermittel, oder durch Anionen- der Kationenaustausch mit anderen Salzen.

Die vorliegende Erfindung schließt darüber hinaus alle Solvate von Verbindungen gemäß Formel (I) mit ein, beispielsweise Hydrate oder Addukte mit Alkohol, aktive Metaboliten von Verbindungen gemäß Formel (I) sowie Derivate, die eine physiologisch annehmbare und abspaltbare Gruppe enthalten, beispielsweise Ester oder Amide.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen. Solche Prodrugs können in vivo zu einer erfindungsgemäßen Verbindung metabolisiert werden. Diese Prodrugs können selbst wirksam sein oder nicht und sind ebenfalls Gegenstand der vorliegenden Erfindung.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Bevorzugte Verbindungen der allgemeinen Formel (I) sind diejenigen Verbindungen, in denen einer, mehrere oder alle der vorstehend aufgeführten Substituenten R¹ bis R⁸, X, Ar, Heteroaryl, Heterocyclyl und Aryl unabhängig voneinander die nachstehend aufgeführten Bedeutungen (Definitionen) haben, wobei alle möglichen Kombinationen von bevorzugten; mehr bevorzugten, viel mehr bevorzugten, noch viel mehr bevorzugten und besonders bevorzugten Bedeutungen (Definitionen), ebenso in Kombination mit den Substituenten in ihrer Grundbedeutung, Gegenstand der vorliegenden Erfindung sind.
- X: ist bevorzugt eine Einfachbindung, NH oder N(C₁-C₃-Alkyl);
- X: ist besonders bevorzugt eine Einfachbindung oder NH;
- R1: ist bevorzugt Wasserstoff oder C₁-C₃-Alkyl, das gegebenenfalls mit Hydroxy, Chlor, Methoxy oder einem, zwei oder drei Fluoratomen substituiert ist;
- R1: ist besonders bevorzugt Wasserstoff oder C₁-C₃-Alkyl;
- R2: ist bevorzugt Wasserstoff, Fluor, -OCF₃, Hydroxy, Methoxy, -NH₂ oder C₁-C₃-Alkyl;
- R2: ist besonders bevorzugt Wasserstoff;
- Ar: ist bevorzugt unsubstituiertes oder zumindest monosubstituiertes Phenyl oder Heteroaryl,
wobei die Substituenten ausgewählt sind aus der Gruppe bestehend aus: Fluor, Chlor, -CF₃, -OCF₃, -C(O)(C₁-C₃-Alkyl), -NH₂, -NHC(O)(C₁-C₃-Alkyl), Hydroxy, C₁-C₃-Alkyl, C₁-C₃-Alkoxy, -CH₂-CH₂-CH₂-, -CH₂-O-C(O)-, -CH₂-C(O)-O-, -CH₂-NH-C(O)-, -CH₂-N(CH₃)-C(O)-, -CH₂-C(O)-NH-, -NH(C₁-C₃-Alkyl), -N(C₁-C₃-Alkyl)₂, -SO₂(C₁-C₃-Alkyl), Heterocyclyl, Heteroaryl und R3, und von diesen Substituenten können Heterocyclyl und Heteroaryl wiederum mit C₁-C₃-Alkyl, C₁-C₃-Alkoxy, Fluor, Chlor, Brom, Trifluormethyl, Trifluormethoxy oder OH zumindest monosubstituiert sein;
- Ar: ist mehr bevorzugt unsubstituiertes oder zumindest monosubstituiertes Phenyl, Thienyl, Furanyl oder Pyridinyl,
wobei die Substituenten ausgewählt sind aus der Gruppe bestehend aus: Fluor, Chlor, -CF₃, -OCF₃, -C(O)(C₁-C₃-Alkyl), -NH₂, -NHC(O)(C₁-C₃-Alkyl), Hydroxy, C₁-C₃-Alkyl, C₁-C₃-Alkoxy, -CH₂-CH₂-CH₂-, -CH₂-O-C(O)-, -CH₂-C(O)-O-, -CH₂-NH-C(O)-, -CH₂-N(CH₃)-C(O)-, -CH₂-C(O)-NH-, -NH(C₁-C₃-Alkyl), -N(C₁-C₃-Alkyl)₂, -SO₂(C₁-C₃-Alkyl) und R3;
- Ar: ist viel mehr bevorzugt monosubstituiertes Phenyl, Thienyl, Furanyl oder Pyridinyl,
wobei der Substituent ausgewählt ist aus der Gruppe bestehend aus: Fluor, Chlor, -CF₃, -OCF₃, -C(O)(C₁-C₃-Alkyl), -NH₂, -NHC(O)(C₁-C₃-Alkyl), Hydroxy, C₁-C₃-Alkyl, C₁-C₃-Alkoxy, -NH(C₁-C₃-Alkyl), -N(C₁-C₃-Alkyl)₂, - SO₂(C₁-C₃-Alkyl) und R3;
und der Substituent und X in meta-Position zueinander stehen; dies ist dahingehend zu verstehen, dass das Fragment Ar der Verbindung gemäß Formel (I) - mit den vorstehenden Definitionen für Ar - mit einem Substituenten substituiert ist und in meta-Position zu diesem Substituenten ist Ar wiederum mit X substituiert. Die meta-Substitution hängt nicht von der Position des Heteroatoms von Ar ab.
- Ar: ist noch viel mehr bevorzugt monosubstituiertes Phenyl, Thienyl, Furanyl oder Pyridinyl,
wobei der Substituent ausgewählt ist aus der Gruppe bestehend aus: Fluor, Chlor, -OCF₃, -C(O)CH₃, -NHC(O)CH₃, Hydroxy, -N(CH₃)₂, Ethöxy, - SO₂CH₃ und R3;
und der Substituent und X in meta-Position zueinander stehen;
- Ar: ist besonders bevorzugt Furanyl monosubstituiert mit R3, und R3 und X stehen in meta-Position zueinander.
- R3: ist bevorzugt -CH₂-NR4R5, -SO₂NR4R5, -C(O)NR4R5, -CH₂-NHC(O)R6, - CH₂-NHSO₂R6 oder -CH(OH)R7;
- R3: ist mehr bevorzugt -CH₂-NR4R5, -C(O)NR4R5, -CH₂-NHC(O)R6,-CH₂NHSO₂R6 oder -CH(OH)R7;
- R3: ist viel mehr bevorzugt -CH₂-NR4R5, -C(O)NR4R5 oder -CH(OH)R7;
- R3: ist besonders bevorzugt -CH₂-NR4R5;
- R4 und R5: sind unabhängig voneinander bevorzugt ausgewählt aus der Gruppe bestehend aus: Wasserstoff; unsubstituiertes oder zumindest monosubstituiertes C₁-C₁₀-Alkyl, C₂-C₆-Alkenyl, Phenyl, Indanyl, Heterocyclyl und Heteroaryl,
wobei die Substituenten ausgewählt sind aus der Gruppe bestehend aus: Phenyl, Heteroaryl, Heterocyclyl, -O-Phenyl, Fluor, -CN, -C(O)NH₂, -C(O)(C₁-C₃-Alkyl), - C(O)-Phenyl, -N(C₁-C₃-Alkyl)₂, -NH(C₁-C₃-Alkyl), -NH₂, -NH-Heteroaryl, -NH-C(O)-Heteroaryl, C₁-C₆-Alkyl, C₁-C₃-Alkoxy und Hydroxy,
und die Phenyl-, Heterocyclyl- und Heteroaryl-Fragmente dieser Substituenten können wiederum mit Fluor, Chlor, Brom, Oxo, -CF₃, -OCF₃, -NO₂, -CN, Phenyl, Pyridinyl, -NHC(O)(C₁-C₃-Alkyl), -COOH, Hydroxy, C₁-C₃-Alkyl, C₁-C₃-Alkoxy, - SO₂NH₂, -SO₂NH(C₁-C₃-Alkyl), -SO₂N(C₁-C₃-Alkyl)₂, -C(O)NH₂, -C(O)NH(C₁-C₃-Alkyl), -C(O)N(C₁-C₃-Alkyl)₂, -SO₂(C₁-C₃-Alkyl), -NH₂, -NH(C₁-C₃-Alkyl) oder - N(C₁-C₃-Alkyl)₂, zumindest monosubstituiert sein; oder
R4 und R5 bilden zusammen mit dem Stickstoffatom, an das sie gebunden sind, unsubstituiertes oder zumindest monosubstituiertes Heterocyclyl,
wobei die Substituenten ausgewählt sind aus der Gruppe bestehend aus: Phenyl, Heteroaryl, Heterocyclyl, Oxo, Fluor, Chlor, -C(O)(C₁-C₃-Alkyl), -C(O)-Phenyl und Hydroxy,
und die Phenyl-, Heterocyclyl- und Heteroaryl-Fragmente dieser Substituenten können wiederum mit Fluor oder C₁-C₃-Alkyl zumindest monosubstituiert sein;
- R4: ist mehr bevorzugt ausgewählt aus der Gruppe bestehend aus: Wasserstoff, unsubstituiertes oder zumindest monosubstituiertes C₁-C₁₀-Alkyl, C₂-C₆-Alkenyl, Phenyl, Indanyl, Heterocyclyl und Heteroaryl,
wobei die Substituenten ausgewählt sind aus der Gruppe bestehend aus: Phenyl, Heteroaryl, Heterocyclyl, -O-Phenyl, Fluor, -CN, -C(O)NH₂, -C(O)(C₁-C₃-Alkyl), - C(O)-Phenyl, -N(C₁-C₃-Alkyl)₂, -NH(C₁-C₃-Alkyl), -NH₂, -NH-Heteroaryl, -NH-C(O)-Heteroaryl, C₁-C₆-Alkyl, C₁-C₃-Alkoxy und Hydroxy,
und die Phenyl-, Heterocyclyl- und Heteroaryl-Fragmente dieser Substituenten können wiederum mit Fluor, Chlor, Brom, Oxo, -CF₃, -OCF₃, -NO₂, -CN, Phenyl, Pyridinyl, -NHC(O)(C₁-C₃-Alkyl), -COOH, Hydroxy, C₁-C₃-Alkyl, C₁-C₃-Alkoxy, - SO₂NH₂, -SO₂NH(C₁-C₃-Alkyl), -SO₂N(C₁-C₃-Alkyl)₂, -C(O)NH₂, -C(O)NH(C₁-C₃-Alkyl), -C(O)N(C₁-C₃-Alkylh, -SO₂(C₁-C₃-Alkyl), -NH₂, -NH(C₁-C₃-Alkyl) oder - N(C₁-C₃-Alkyl)₂ zumindest monosubstituiert sein ; und R5 ist Wasserstoff; oder
R4 und R5 bilden zusammen mit dem Stickstoffatom, an das sie gebunden sind, unsubstituiertes oder zumindest monosubstituiertes Heterocyclyl,
wobei die Substituenten ausgewählt sind aus der Gruppe bestehend aus: Phenyl, Heteroaryl, Heterocyclyl, Oxo, Fluor, Chlor, -C(O)(C₁-C₃-Alkyl), -C(O)-Phenyl und Hydroxy,
und die Phenyl-, Heterocyclyl- und Heteroaryl-Fragmente dieser Substituenten können wiederum mit Fluor oder C₁-C₃-Alkyl zumindest monosubstituiert sein;
- R4: ist viel mehr bevorzugt ausgewählt aus der Gruppe bestehend aus; Wasserstoff; unsubstituiertes oder zumindest monosubstituiertes C₁-C₁₀-Alkyl, Cyclohexenyl, Indanyl, Phenyl, Pyrrolidinyl, Pyrrolyl, Pyrazolyl, Furanyl und Piperidinyl,
wobei die Substituenten ausgewählt sind aus der Gruppe bestehend aus: Fluor, - CN, -C(O)NH₂, -O-Phenyl, -C(O)-Phenyl, -N(CH₃)₂, C₁-C₃-Alkyl, C₁-C₃-Alkoxy, Hydroxy, unsubstituiertes oder zumindest monosubstituiertes Phenyl, Pyridinyl, Thienyl, Pyrimidinyl, Imidazolyl, Furanyl, Indolyl, Benzimidazolyl, Pyrazolyl, Morpholinyl, Pyrrolidinyl, 1,3-Benzodioxolyl, Piperidinyl, Tetrahydropyranyl, Triazolyl, Thiazolyl, Thiazolidinyl, Isoxazolyl und Dihydroisoxazolyl, deren Substituenten sind wiederum ausgewählt aus der Gruppe bestehend aus: Fluor, Chlor, Oxo, CF₃, -OCF₃, -NO₂, Phenyl, Pyridinyl, -NHC(O)CH₃, -COOH, Hydroxy, C₁-C₃-Alkyl, C₁-C₃-Alkoxy, -SO₂NH₂, -C(O)NH₂ und -N(CH₃)₂; und R5 ist Wasserstoff; oder
- R4 und R5: bilden zusammen mit dem Stickstoffatom, an das sie gebunden sind, einen Rest ausgewählt aus der Gruppe bestehend aus: unsubstituiertes oder zumindest monosubstituiertes Piperidinyl, Pyrrolidinyl, Morpholinyl und Piperazinyl,
wobei die Substituenten ausgewählt sind aus der Gruppe bestehend aus: Fluor, - C(O)(C₁-C₃-Alkyl), Oxo, C₁-C₃-Alkyl, Hydroxy, unsubstituiertes oder zumindest monosubstituiertes Phenyl, imidazolyl, Pyridinyl, Pyrimidinyl, Piperidinyl und Pyrrolidinyl, deren Substituenten sind wiederum Fluor oder C₁-C₃-Alkyl;
- R4: ist besonders bevorzugt unsubstituiertes oder zumindest monosubstituiertes C₁-C₆-Alkyl,
wobei die Substituenten ausgewählt sind aus der Gruppe bestehend aus: -N(CH₃)₂, Hydroxy, unsubstituiertes oder zumindest monosubstituiertes Phenyl, Pyridinyl, Imidazolyl, Indolyl, Benzimidazolyl, Pyrazolyl und Pyrrolidinyl, deren Substituenten sind wiederum ausgewählt aus der Gruppe bestehend aus: - NHC(O)CH₃, C₁-C₃-Alkyl, C₁-C₃-Alkoxy, -SO₂NH₂ und -C(O)NH₂; und R5 ist Wasserstoff; oder
R4 und R5 bilden zusammen mit dem Stickstoffatom, an das sie gebunden sind, einen Rest ausgewählt aus der Gruppe bestehend aus: unsubstituiertes oder zumindest monosubstituiertes Pyrrolidinyl, Piperidinyl und Piperazinyl, wobei die Substituenten ausgewählt sind aus der Gruppe bestehend aus: C₁-C₃-Alkyl, Hydroxy und Pyrrolidinyl;
- R6: ist bevorzugt unsubstituiertes oder zumindest monosubstituiertes C₁-C₆-Alkyl, Phenyl oder Heteroaryl,
wobei die Substituenten ausgewählt sind aus der Gruppe bestehend aus: Fluor, Chlor, Brom, -CF₃, -OCF₃, -NHC(O)(C₁-C₃-Alkyl), Hydroxy, C₁-C₃-Alkyl, C₁-C₃-Alkoxy, -O-Heteroaryl, Phenyl, -NH₂, -NH(C₁-C₃-Alkyl), -N(C₁-C₃-Alkyl)₂ und Heterocyclyl,
und die Phenyl-, Heteroaryl- und Heterocyclyl-Fragmente dieser Substituenten können wiederum mit Fluor, Chlor, Brom, Hydroxy, C₁-C₃-Alkyl oder C₁-C₃-Alkoxy zumindest monosubstituiert sein;
- R6: ist mehr bevorzugt -CF₃ oder unsubstituiertes oder zumindest monosubstituiertes C₁-C₆-Alkyl, Pyridinyl, Furanyl oder Phenyl, wobei die Substituenten ausgewählt sind aus der Gruppe bestehend aus: Fluor, -NHC(O)(C₁-C₃-Alkyl), Hydroxy, C₁-C₃-Alkyl, C₁-C₃-Alkoxy und -O-Pyridinyl;
- R6: ist besonders bevorzugt C₁-C₆-Alkyl, Pyridinyl, -CF₃, Furanyl oder Phenyl, und Phenyl kann gegebenenfalls mit -NH(O)CH₃, -O-Pyridinyl oder Methoxy substituiert sein;
- R7: ist bevorzugt ausgewählt aus der Gruppe bestehend aus: Wasserstoff; unsubstituiertes oder zumindest monosubstituiertes C₁-C₆-Alkyl, Phenyl und Pyridinyl,
wobei die Substituenten ausgewählt sind aus der Gruppe bestehend aus: Fluor, Chlor, Brom, Hydroxy, C₁-C₃-Alkyl und C₁-C₃-Alkoxy;
- R7: ist besonders bevorzugt Wasserstoff;
- R9: ist bevorzugt Wasserstoff oder Pyrrolidinylethyl;
Aryl ist bevorzugt Phenyl, Indanyl oder Naphthyl;
Aryl ist mehr bevorzugt Phenyl oder Indanyl;
Aryl ist besonders bevorzugt Phenyl;
Heteroaryl ist bevorzugt Pyridinyl, Thienyl, Pyrimidinyl, Imidazolyl, Furanyl, Indolyl, Benzimidazolyl, Pyrazolyl, 1,3-Benzodioxolyl, Triazolyl, Thiazolyl, Isoxazolyl, Pyrrolyl, Pyrazinyl, Oxazolyl, Pyridazinyl, Chinolinyl, Isochinolyl, Benzofuranyl, 3-Oxo-1,3-dihydroisobenzofuranyl oder 4,5,6,7-Tetrahydrobenzothiazolyl;
Heteroaryl ist mehr bevorzugt Pyridinyl, Thienyl, Pyrimidinyl, Imidazolyl, Furanyl, Benzimidazolyl, Pyrazolyl, Thiazolyl, Isöxazolyl, Pyrrolyl, Pyrazinyl, 3-Oxo-1,3-dihydroisobenzofuranyl oder 4,5,6,7-Tetrahydrobenzothiazolyl
Heteroaryl ist besonders bevorzugt Pyridinyl, Thienyl, Pyrazolyl, Furanyl oder Benzimidazolyl;
Heterocyclyl ist bevorzugt Morpholinyl, Pyrrolidinyl, Piperidinyl, Tetrahydropyranyl, Thiazolidinyl, Dihydroisoxazolyl, Piperazinyl oder Tetrahydrofuranyl;
Heterocyclyl ist besonders bevorzugt Morpholinyl, Pyrrolidinyl, Piperidinyl oder Piperazinyl;
Beispiele für Ausführungsformen mit bevorzugten Verbindungen der allgemeinen Formel (I) unter Bezugnahme auf die vorstehend beschriebenen Bedeutungen (Definitionen) sind:
i) R1 bis R7, X, Ar, Heteroaryl und Heterocyclyl haben jeweils ihre bevorzugte Bedeutung; oder
ii) R1 hat seine bevorzugte Bedeutung und alle anderen Substituenten haben ihre Grundbedeutung; oder
iii) R2 hat seine bevorzugte Bedeutung und alle anderen Substituenten haben ihre Grundbedeutung; oder
iv) R3 hat seine bevorzugte Bedeutung und R1, R2, R4 bis R8, X, Ar, Heteroaryl, Heterocyclyl und Aryl haben ihre Grundbedeutung; oder
v) R4 und R5 haben jeweils ihre bevorzugte Bedeutung und alle anderen Substituenten haben ihre Grundbedeutung; oder
vi) R6 hat seine bevorzugte Bedeutung und alle anderen Substituenten haben ihre Grundbedeutung; oder
vii) R7 hat seine bevorzugte Bedeutung und alle anderen Substituenten haben ihre Grundbedeutung; oder
viii) R9 hat seine bevorzugte Bedeutung und alle anderen Substituenten haben ihre Grundbedeutung; oder
ix) X hat seine bevorzugte Bedeutung und alle anderen Substituenten haben ihre Grundbedeutung; oder
x) Ar hat seine bevorzugte Bedeutung und alle anderen Substituenten haben ihre Grundbedeutung; oder
xi) Aryl hat seine bevorzugte Bedeutung und alle anderen Substituenten haben ihre Grundbedeutung; oder
xii) Heteroaryl hat seine bevorzugte Bedeutung und alle anderen Substituenten haben ihre Grundbedeutung; oder
xiii) Heterocyclyl hat seine bevorzugte Bedeutung und alle anderen Substituenten haben ihre Grundbedeutung; oder
xiv) Aryl, Heterocyclyl und Heteroaryl haben jeweils ihre bevorzugte Bedeutung und alle anderen Substituenten haben ihre Grundbedeutung; oder
xv) R4 bis R7 und R9 haben jeweils ihre bevorzugte Bedeutung und alle anderen Substituenten haben ihre Grundbedeutung; oder
xvi) R6 und Ar haben jeweils ihre mehr bevorzugte Bedeutung und R1 bis R5, R7, X, Heteroaryl und Heterocyclyl haben jeweils ihre bevorzugte Bedeutung; oder
xvii) R1 und 2 haben jeweils ihre besonders bevorzugte Bedeutung, Ar hat seine viel mehr bevorzugte Bedeutung, R3, R4, R6 und Heteroaryl haben jeweils ihre mehr bevorzugte Bedeutung und R7, X und Heterocyclyl haben jeweils ihre bevorzugte Bedeutung; oder
xviii) R1, R2, R7 und X haben jeweils ihre besonders bevorzugte Bedeutung und R3, R4 und Ar haben jeweils ihre viel mehr bevorzugte Bedeutung; oder
xix) R1 bis R4 und X haben jeweils ihre besonders bevorzugte Bedeutung und Ar hat seine noch viel mehr bevorzugte Bedeutung; oder
xx) R1 bis R4, X und Ar haben jeweils ihre besonders bevorzugte Bedeutung; oder
xxi) R1 bis R3, X und Ar haben jeweils ihre besonders bevorzugte Bedeutung und R4 hat seine viel mehr bevorzugte Bedeutung; oder
xxii) R1, R2 und X haben jeweils ihre bevorzugte Bedeutung, R4 hat seine mehr bevorzugte Bedeutung, Ar hat seine noch viel mehr bevorzugte Bedeutung und R3, Heteroaryl und Heterocyclul haben jeweils ihre besonders bevorzugte Bedeutung; oder
xxiii) R1, R2, X, Heteroaryl und Heterocyclyl haben jeweils ihre besonders bevorzugte Bedeutung, R4 und Ar haben jeweils ihre mehr bevorzugte Bedeutung, R3 hat seine viel mehr bevorzugte Bedeutung und R7 hat seine bevorzugte Bedeutung; oder
xxiv) R1, R2 und R7 haben jeweils ihre besonders bevorzugte Bedeutung, R3 hat seine viel mehr bevorzugte Bedeutung, R4, R6, Ar und Heteroaryl haben jeweils ihre mehr bevorzugte Bedeutung und X und Heterocyclyl haben jeweils ihre bevorzugte Bedeutung; oder
xxv) R1, R2 und Ar haben jeweils ihre besonders bevorzugte Bedeutung, R3, R7, X und Heterocyclyl haben jeweils ihre bevorzugte Bedeutung und R4, R6 und Heteroaryl haben jeweils ihre mehr bevorzugte Bedeutung; oder
xxvi) R1, R2, R4, R5, R7, X, Heteroaryl und Heterocyclyl haben jeweils ihre bevorzugte Bedeutung, Ar hat seine viel mehr bevorzugte Bedeutung, R6 hat seine mehr bevorzugte Bedeutung und R3 und R9 haben ihre Grundbedeutung; oder
xxvii) R1, R2, X, Ar, Heteroaryl und Heterocyclyl haben jeweils ihre bevorzugte Bedeutung, R3, R4 und R6 haben jeweils ihre mehr bevorzugte Bedeutung und R7 hat seine besonders bevorzugte Bedeutung; oder
xxviii) R1 bis R7, X, Heteroaryl, Heterocyclyl und Aryl haben jeweils ihre bevorzugte Bedeutung und Ar hat seine Grundbedeutung; oder
xxix) R1, R2, X, Heteroaryl, Heterocyclyl und Aryl haben jeweils ihre bevorzugte Bedeutung, R3, R4 und R6 haben jeweils ihre mehr bevorzugte Bedeutung, R7 hat seine besonders bevorzugte Bedeutung und Ar hat seine Grundbedeutung; oder
xxx) R1, R2, R4 bis R7, R9, X, Heteroaryl und Heterocyclyl haben jeweils ihre bevorzugte Bedeutung, Ar hat seine mehr bevorzugte Bedeutung und R3 hat seine Grundbedeutung; oder
xxxi) R1, R2, R9, X, Heteroaryl und Heterocyclyl haben jeweils ihre bevorzugte Bedeutung, R4, R6 und Ar haben jeweils ihre mehr bevorzugte Bedeutung, R7 hat seine besonders bevorzugte Bedeutung und R3 hat seine Grundbedeutung; oder
xxxii) R3 bis R7, Ar, Heteroaryl und Heterocyclyl haben jeweils ihre bevorzugte Bedeutung und R1, R2 und X haben jeweils ihre Grundbedeutung; oder
xxxiii) Ar hat seine viel mehr bevorzugte Bedeutung, X, Aryl, Heteroaryl und Heterocyclyl haben jeweils ihre bevorzugte Bedeutung und alle anderen Substituenten haben ihre Grundbedeutung; oder
xxxiv) X, Aryl, Heteroaryl und Heterocyclyl haben jeweils ihre bevorzugte Bedeutung, R3, R4 und R6 haben jeweils ihre mehr bevorzugte Bedeutung, R7 hat seine besonders bevorzugte Bedeutung und R1, R2 und Ar haben jeweils ihre Grundbedeutung.

Wie oben aufgeführt, sind die bevorzugten Verbindungen der allgemeinen Formel (I) nicht beschränkt auf die vorgenannten Beispiele. Vielmehr sind alle Kombinationen der einzelnen Substituenten in ihrer Grundbedeutung mit den bevorzugten, mehr bevorzugten, viel mehr bevorzugten, noch viel mehr bevorzugten oder besonders bevorzugten Bedeutungen der übrigen Substituenten oder alle Kombinationen der bevorzugten, mehr bevorzugten, viel mehr bevorzugten, noch viel mehr bevorzugten oder besonders bevorzugten Bedeutungen der einzelnen Substituenten, die vorstehend nicht als Beispiel aufgeführt sind, auch Gegenstand dieser Erfindung. Dies trifft selbstverständlich nur dann zu, so weit die Definitionen der jeweiligen Substituenten eine solche Kombination zulässt.

Am meisten bevorzugte Verbindungen gemäß der allgemeinen Formel (I) sind ausgewählt aus der Gruppe bestehend aus:
4-(3-Methansulfonyl-phenylamino)-3-methyl-5,6,7,8-tetrahydro-2H-isochinolin-1-on, 4-(3-Acetyl-phenylamino)-3-methyl-5,6,7,8-tetrahydro-2H-isochinolin-1-on, 4-(5-Butylaminomethyl-furan-2-yl)-3-methyl-5,6,7,8-tetrahydro-2H-isochinolin-1-on, 3-Methyl-4-(5-pyrrolidin-1-ylmethyl-furan-2-yl)-5,6,7,8-tetrahydro-2H-isochinolin-1-on, 4-[5-(3-Hydroxy-pyrrolidin-1-ylmethyl)-furan-2-yl]-5,6,7,8-tetrahydro-2H-isochinolin-1- on, 4-(5-{[(4-Pyrrolidin-1-yl-piperidin-1-ylmethyl)-amino]-methyl}-furan-2-yl)-5,6,7,8-tetrahydro-2H-isochinolin-1-on, 4-{5-[(2-Dimethylamino-ethylamino)-methyl]-furan-2-yl}-5,6,7,8-tetrahydro-2H-isochinolin-1-on, 4-{5-[(2-Hydroxy-2-phenyl-ethylamino)-methyl]-furan-2-yl}-5,6,7,8-tetrahydro-2H- isochinolin-1-on, 4-(5-{[(4-Methyl-piperazin-1-ylmethyl)-amino]-methyl}-furan-2-yl)-5,6,7,8-tetrahydro-2H-isochinolin-1-on, 4-(5-{[(1-Methyl-1 H-pyrazol-4-ylmethyl)-amino]-methyl}-furan-2-yl)-5,6,7,8-tetrahydro- 2H-isochinolin-1-on, 4-(5-Butylaminomethyl-furan-2-yl)-5,6,7,8-tetrahydro-2H-isochinolin-1-on und 4-(5-Hydroxymethyl-furan-2-yl)-3-methyl-5,6,7,8-tetrahydro-2H-isochinolin-1-on.

Die Verbindungen der Formel I sind durch unterschiedliche chemische Verfahren herstellbar, die ebenfalls zu der vorliegenden Erfindung gehören. Einige typische Wege sind in den unten als Schemata 1 bis 3 bezeichneten Reaktionssequenzen skizziert. Substituenten R sind jeweils wie oben angegeben definiert, sofern nachfolgend nichts anderes angegeben ist. Die Ausgangsverbindungen sowie die Zwischenverbindungen (Intermediate) sind entweder kommerziell erhältlich oder können mit dem Fachmann bekannten Verfahren hergestellt werden.

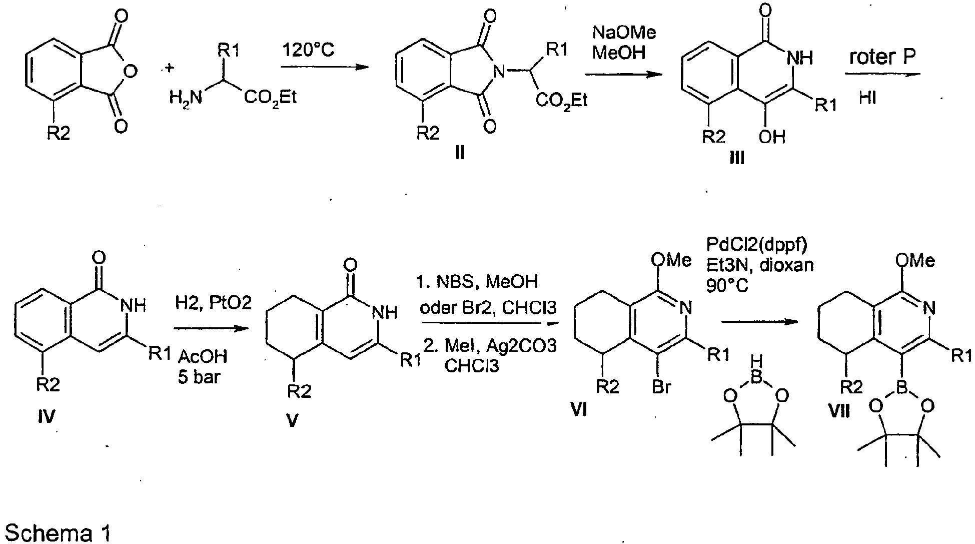

Intermediat II kann durch Erhitzen von Phtalsäureanhydrid mit Aminosäureestern erhalten werden. Anschließende Behandlung der Phtalimide II mit Natriummethanolat in Methanol unter Rückfluss ergibt Intermediate III die nach reduktiver Dehydroxylierung mit rotem Phosphor in lodwasserstoffsäure in die Isochinolinone IV umgewandelt werden. Partielle Hydrierung dieser Intermediate, die teilweise auch kommerziell erhältlich sind (z. Bsp. für R1 = H) bzw. auf anderem Weg hergestellt werden können, ergibt die Tetrahydroisochinolinone V. Intermediat VI kann daraus durch Bromierung (die z. Bsp. mit N-Bromsuccinimid in Methanol oder mit Brom in Chloroform als Lösungsmittel durchgeführt werden kann) und anschliessende O-Alkylierung (z. Bsp. durch Methyliodid in Chloroform mit Zusatz von Silbercarbonat) dargestellt werden. Aus dem Halogenid VI kann durch Palladium-katalysierte Borylierung (z. Bsp. durch Umsetzung mit 4,4,5,5-Tetramethyl-1,3,2-dioxaborolan mit Palladiumdichlorid-1,1'-bis(diphenylphosphino)ferrocen als Katalysator und Triethylamin als Base in 1,4-Dioxan als Lösungsmittel) Intermediat VII hergestellt werden. Beide Intermediate VI und VII sind für die weitere Umsetzung zu den Verbindungen I geeignet (siehe Schemata 2 und 3). Der Rest R2 kann gegebenenfalls während oder im Anschluss an einen beliebigen Reaktionschritt der Schemata 1 bis 3 mit dem Fachmann bekannten Mitteln modifiziert werden.

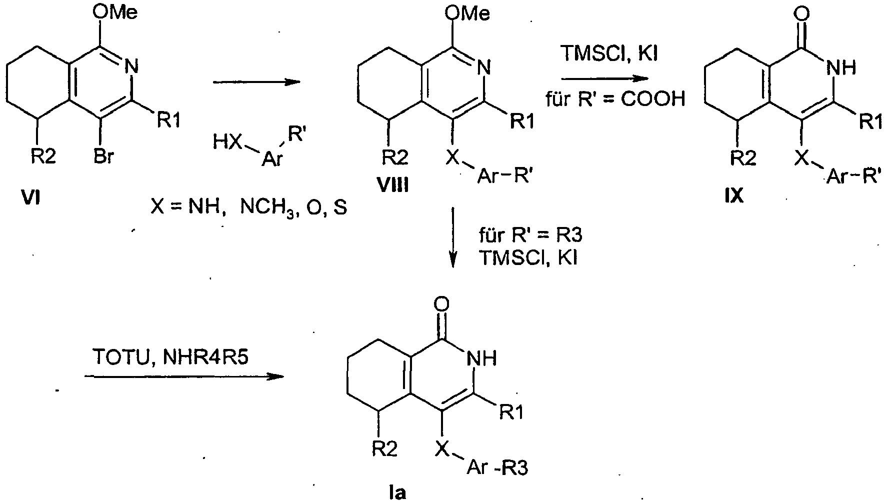

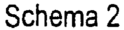

Verbindungen der Formel VIII mit X gleich NH oder N(C₁-C₃-Alkyl) können durch Hartwig-Buchwald Aminierung mit aromatischen Aminen unter Palladiumkatalyse (zum Beispiel mit Palladium-dibenzylidenaceton und Bis(tert-butyl)biphenylphosphin als Katalysator und Natrium- oder Kalium-tert-butanolat als Base in Toluol) dargestellt werden. Verbindungen der Formel VIII mit X = O, S können durch Umsetzung von Natrium- oder Kaliumphenolaten beziehungsweise Natrium- oder Kaliumthiophenolaten unter Kupferkatalyse (zum Beispiel Kupfer(I)chlorid, Kupfer(I)oxid oder Kupferpulver in hochsiedenden Lösungsmitteln wie DMF oder Collidin) dargestellt werden. Abspaltung der Methylgruppe aus den Verbindungen der Formel VIII (z. Bsp. durch Trimethylchlorsilan und Kaliumiodid in Acetonitril) ergibt Verbindungen der Formel Ia (Schema 2). Dabei kann das Heteroaryl/Aryl-Fragment (Ar) entweder unsubstituiert (R' = H) oder zumindest monosubstituiert sein, wobei die Substituenten die in der Grundbedeutung von Ar aufgeführten (wie R' = R³ oder Heteroaryl) oder auch R' = COOH sein können. Bei Vorliegen geeigneter funktioneller Gruppen, zum Beispiel wenn R' eine Carboxylatfunktion ist, können durch Amidbindungsbildung mit Kupplungsreagenzien wie O-[(Ethoxycarbonyl)-cyanmethylenamino]-N,N,N',N'- tetramethyluroniumtetra-fluoroborat (TOTU) weitere Verbindungen der Formel la dargestellt werden in denen R3 = -C(O)NR4R5 bedeutet.

Verbindungen der Formel X können durch Palladium-katalysierte Suzuki-Kupplung einer aromatischen Boronsäure oder eines Boronsäureesters und der Intermediate VI dargestellt werden (Schema 3). Zur Herstellung von Verbindungen der Formel X kann auch von Intermediat VII ausgegangen werden, das durch Palladium-katalysierte Suzuki-Kupplung (z.B. mit Palladiumdichlorid-1,1'-bis(diphenylphosphino)ferrocen als Katalysator und Kaliumcarbonat als Base in Dimethylformamid) mit aromatischen Halogeniden (z. Bsp. Br-Ar-R"; R" kann dabei zum Beispiel Wasserstoff, -CHO oder - COOH sein) gekuppelt wird (Schema 3).

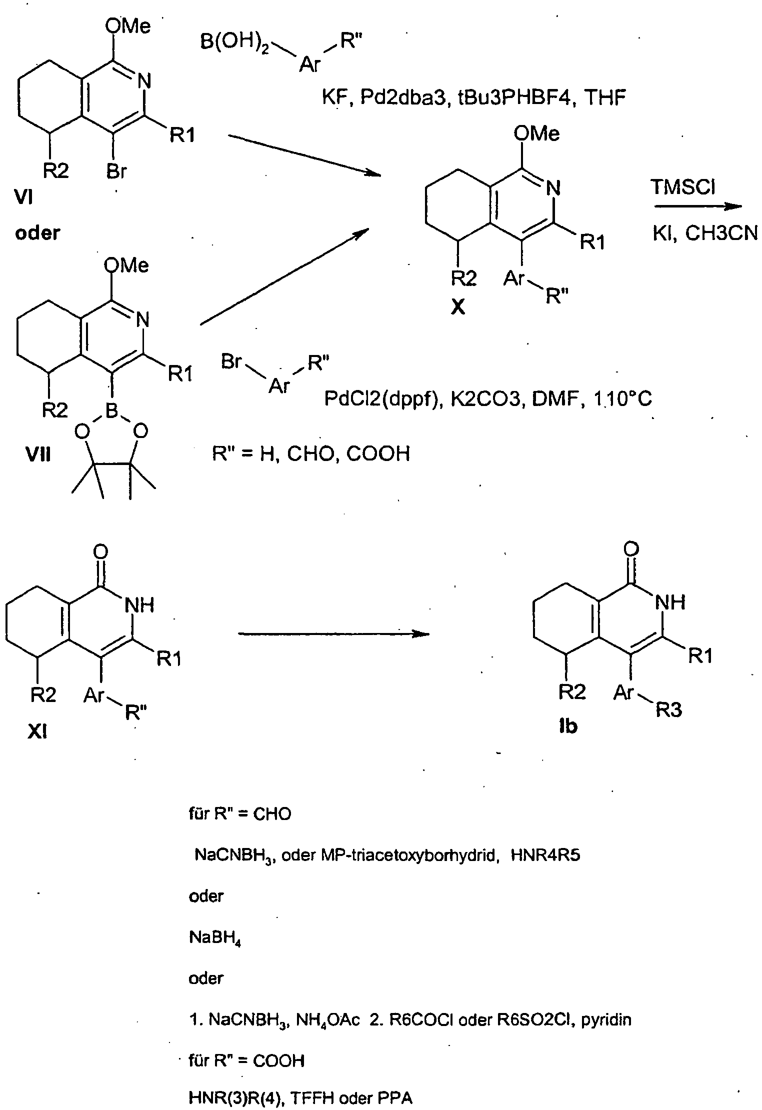

Abspaltung der Methylgruppe aus den Verbindungen der Formel X (z. Bsp. durch Trimethylchlorsilan und Kaliumiodid in Acetonitril) ergibt Tetrahydroisochinolinone der Formel XI. Zur Herstellung von Verbindungen der Formel Ib in denen R3 = - CH2NR4R5 bedeutet, werden Verbindungen XI mit R" = -CHO eingesetzt, die durch reduktive Aminierung mit Aminen NR4R5 umgesetzt werden. Dabei können Reduktionsmittel wie Natriumcyanoborhydrid oder festphasengebundenes Triacetoxyborhydrid verwendet werden.

Zur Herstellung von Verbindungen der Formel Ib, in denen R3 = -CH₂OH bedeutet, werden Verbindungen XI mit R" = -CHO eingesetzt, die durch Reduktion z.B. mit Natriumborhydrid umgesetzt werden.

Zur Herstellung von Verbindungen der Formel Ib, in denen R3 = -CH₂NHC(O)R6 oder - CH₂NHSO₂R6 bedeuten, wird zunächst eine reduktive Aminierung mit Ammoniumacetat durchgeführt und die so erhaltenen Aminomethylverbindungen mit entsprechenden Säurechloriden oder Säuren bzw. mit Sulfonylchloriden gekuppelt.

Zur Herstellung von Verbindungen der Formel Ib, in denen R3 = -C(O)NR4R5 bedeutet, werden Verbindungen der Formel XI mit R" = -COOH eingesetzt, die mit Aminen HNR4R5 gekuppelt werden. Dabei können verschiedene geeignete Kondensationsmittel wie Carbodiimide, TFFH oder Phosphonsäureanhydride (z. Bsp. PPA) verwendet werden.

Zur Herstellung von Verbindungen der Formel Ib, in denen R3 = -CHNOR9 bedeutet, werden Verbindungen XI mit R" = -CHO eingesetzt, die unter Oxim-Bildung mit Hydroxylaminen R9ONH₂ umgesetzt werden.

Zur Herstellung von Verbindungen der Formel Ib, in denen R3 = -SO₂NR4R5 bedeutet, werden Verbindungen XI mit R" = H eingesetzt, die durch Chlorsulfonierung (beispielsweise mit Chlorsulfonsäure und Phosphorpentachlorid in Chloroform) in die entsprechenden Sulfochloride, überführt werden und diese werden dann mit Aminen HNR4R5 umgesetzt.

Zur Herstellung von Verbindungen der Formel Ib, in denen R3 = -C(O)R6 bedeutet, werden Verbindungen XI mit R" = H eingesetzt, die lithiert und anschließend mit Weinreb-Amiden R6C(O)NMe(OMe) zu den gewünschten Verbindungen umgesetzt werden.

Zur Herstellung von Verbindungen der Formel Ib, in denen R3 = -CH(OH)R7 bedeutet, werden Verbindungen XI mit R" = H lithiert und dann mit Aldehyden R7CHO zur Reaktion gebracht.

Zur Herstellung von Verbindungen der Formel Ib, in denen R3 = -NHSO₂R6 bedeutet, werden Verbindungen XI mit R" = -NH₂ eingesetzt, die mit Sulfonylchlorideh R6SO₂Cl in Gegenwart einer Base umgesetzt werden.

Zur Herstellung von Verbindungen der Formel Ib, in denen R3 = -NHC(O)R6 bedeutet, werden Verbindungen der Formel XI mit R" = -NH₂ eingesetzt, die mit einem geeigneten Säurechlorid in Gegenwart einer Base oder mit einer geeigneten Säure in Gegenwart eines Kondensierungsmittels umgesetzt werden.

Zur Herstellung von Verbindungen der Formel Ib, in denen R3 = -(C₂-C₃-Alkyl)-NR4R5 bedeutet, werden Verbindungen XI mit R" = -CHO eingesetzt, die mit einer geeigneten Wittig-Reagenz umgesetzt werden, mit anschließender Schutzgruppenentfernung und reduktiver Aminierung des daraus erhaltenen Aldehyds mit einem geeigneten Amin. Vorzugsweise ist die Wittig-Reagenz Ph₃P=CHOR.

Zur Herstellung der Verbindungen der Formel Ib, in denen R3 = -(C₂-C₃-Alkyl)-NHC(O)R6 oder -(C₂-C₃-Alkyl)-NHSO₂R6 bedeuten, werden Verbindungen XI mit R" = -CHO eingesetzt, die mit einer geeigneten Wittig-Reagenz umgesetzt werden mit nachfolgender Entfernung der Schutzgruppen und reduktiver Aminierung des Aldehyds mit Ammoniumacetat zum Amin. Dieses Amin wird mit einem geeigneten Säurechlorid R6C(O)Cl oder mit einem geeigneten Sulfonylchlorid R6SO₂Cl in Gegenwart einer Base zu den oben erwähnten Verbindungen umgesetzt.

Bei allen Verfahrensweisen kann es angebracht sein, bei bestimmten Reaktionsvorschriften funktionelle Gruppen im Molekül zeitweilig zu schützen. Solche Schutzgruppen sind dem Fachmann geläufig. Die Auswahl einer Schutzgruppe für in Betracht kommende Gruppen und die Verfahren zu ihrer Einführung und Abspaltung sind in der Literatur beschrieben und können gegebenenfalls ohne Schwierigkeiten dem Einzelfall angepasst werden.
Gegenstand der vorliegenden Erfindung ist auch die Verwendung von Verbindungen gemäß der allgemeinen Formel (I) als Arzneimittel beziehungsweise Medikament. Hinsichtlich der Definitionen der Substituenten X, Ar, R¹ und R² (sowie der weiteren, über die vorgenannten Substituenten definierten Substituenten) werden auf die Ausführungen hinsichtlich der Verbindungen als solche verwiesen.

Die Verwendung von Verbindungen gemäß der allgemeinen Formel (I) als Arzneimittel, wobei einer, mehrere oder alle der vorgenannten Substituenten die oben aufgeführte bevorzugte, mehr bevorzugte, viel mehr bevorzugte, noch viel mehr bevorzugte oder besonders bevorzugte Bedeutung haben, inklusive sämtlicher Kombinationen untereinander, ist ebenfalls Gegenstand der vorliegenden Erfindung.

Die Verbindungen der allgemeinen Formel (I) sind PARP-Inhibitoren und eignen sich folglich zur Behandlung von Krankheiten, die zu PARP in Bezug stehen, dadurch verursacht werden, gefördert werden oder aus dessen Beteiligung resultieren.

Beispiele von Krankheiten, die mit den Verbindungen gemäß der vorliegenden Erfindung behandelt werden können, umfassen: Gewebeschaden resultierend aus Zellschädigung oder Zelltod aufgrund von Nekrose oder Apoptose, neuronal vermittelter Gewebeschaden oder Erkrankungen, zerebrale Ischämie, Kopftrauma, Schlaganfall, Reperfusionsschaden, neurologische Störungen und neurodegenerative Erkrankungen, vaskulärer Schlaganfall, Herz-Kreislauf-Störungen, Myokardinfarkt, Myokardischämie, experimentelle allergische Enzephalomyelitis (EAE), Multiple Sklerose (MS), Ischämie in Bezug zu Herzchirurgie, altersbezogene Makuladegeneration, Arthritis, Arterosklerose, Krebs, degenerative Erkrankungen des Skelettmuskels mit folgender replikativer Seneszenz, Diabetes und diabetische Herzmuskelerkrankungen.

Vorzugsweise werden die Verbindungen der vorliegenden Erfindung zur Behandlung von Krankheiten eingesetzt, die durch Ischämie oder Reperfusionsschäden verursacht werden. Krankheiten, die behandelt werden können, sind mehr bevorzugt ausgewählt aus der Gruppe bestehend aus: zerebraler Ischämie, Reperfusionsschaden, Herz-Kreislauf-Störungen, Myokardinfarkt, Myokardischämie und Ischämie in Bezug zu Herzchirurgie.
Insbesondere können die Verbindungen der vorliegenden Erfindung zur Behandlung eines Myokardinfarktes verwendet werden.

In den vorstehenden Ausführungen umfasst der Begriff Behandlung auch die Prophylaxe, Therapie oder Heilung der vorgenannten Krankheiten.

Nachfolgend beziehen sich alle Verweise auf "Verbindung(en) gemäß Formel (I)" auf Verbindung(en) der Formel (I) wie vorstehend beschrieben, sowie ihre Salze, Solvate und physiologisch funktionellen Derivate wie hierin beschrieben.

Die Verbindungen gemäß Formel (I) können Tieren und Menschen, bevorzugt Säugetieren und Menschen, besonders bevorzugt Menschen, verabreicht werden. Die Verbindungen gemäß Formel (I) können dabei selbst als Arzneimittel, in Mischungen miteinander oder in Mischungen mit anderen Arzneimitteln oder in Form von pharmazeutischen Zusammensetzungen verabreicht werden. Folglich sind die Verwendung von Verbindungen gemäß Formel (I) zur Herstellung eines oder mehrerer Medikamente zur Prophylaxe und/oder Behandlung der vorgenannten Krankheiten, pharmazeutische Zusammensetzungen enthaltend eine wirksame Menge von mindestens einer Verbindung gemäß Formel (I) sowie pharmazeutische Zusammensetzungen enthaltend eine wirksame Menge von mindestens einer Verbindung gemäß Formel (I) zur Prophylaxe und/oder Behandlung der vorgenannten Krankheiten ebenfalls Gegenstand der vorliegenden Erfindung

Die Menge einer Verbindung gemäß Formel (I), die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im Allgemeinen liegt die Tagesdosis im Bereich von 0,3 mg bis 100 mg (typischerweise von 3 mg bis 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Im Falle pharmazeutisch verträglicher Salze beziehen sich die vorgenannten Gewichtsangaben auf das Gewicht der dem Salz zugrunde liegenden freien Verbindung. Zur Prophylaxe oder Therapie der oben genannten Zustände können die Verbindungen gemäß Formel (I) selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist (physiologisch verträglich). Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel (I). Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Neben mindestens einer Verbindung gemäß Formel (I) sowie einem oder mehreren Trägerstoffen können die erfindungsgemäßen pharmazeutischen Zusammensetzungen auch Hilfsstoffe enthalten. Beispielsweise eignen sich als Hilfsbeziehungsweise Zusatzstoffe: Füllstoffe, Bindemittel, Gleitmittel, Netzmittel, Stabilisatoren, Emulgatoren, Dispergiermittel, Konservierungsmittel, Süßstoffe, Farbstoffe, Geschmacksstoffe, Aromastoffe, Verdickungsmittel, Verdünnungsmittel, Puffersubstanzen, Lösungsmittel, Lösungsvermittler, Mittel, mit denen eine Depotwirkung erzielt werden kann, Salze zur Veränderung des osmotischen Druckes, Beschichtungsmittel oder Antioxidantien.

Die erfindungsgemäßen pharmazeutischen Zusammensetzungen können beispielsweise in Form einer Pille, Tablette, beschichteten Tablette, Lutschtablette, Granulat, Kapsel, harten oder weichen Gelatinkapsel, wässrigen Lösung, alkoholischen Lösung, ölartigen Lösung, Sirup, Emulsion, Suspension, Zäpfchen, Pastille, Lösung zur Injektion oder Infusion, Salbe, Tinktur, Creme, Lotion, Puder, Spray, transdermalen therapeutischen Systems, Nasenspray, Aerosol, Aerosol-Mischung, Mikrokapsel, Implantat, Stab (rod) oder Pflaster vorliegen.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel (I) abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel (I) enthalten; als Pulver (Gelatinekapseln oder Beutel) oder Granulate; als Lösung oder Suspension in einer wässrigen oder nicht-wässrigen Flüssigkeit; oder als eine ÖI-in-Wasser- oder Wasser-in-ÖI-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden. Als Verdünnungsmittel eignen sich beispielsweise Stärke, Cellulose, Saccharose, Laktose oder Kieselgel. Weiterhin können die erfindungsgemäßen pharmazeutischen Zusammensetzungen auch Substanzen enthalten, bei denen es sich nicht um Verdünnungsmittel handelt, beispielsweise eine oder mehrere Gleitmittel, wie Magnesiumstearat oder Talkum, einen Farbstoff, eine Beschichtung (Dragees) oder einen Lack.

Pharmazeutische Zusammensetzungen, die für eine perorale (sublinguale) Verabreichung geeignet sind, umfassen Lutschtabletten, die eine Verbindung gemäß Formel (I) mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel (I), die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im Allgemeinen von 0,1 bis 5 Gew.-% der aktiven Verbindung.

Bei den sterilen Zusammensetzungen zur parenteralen Verabreichung kann es sich vorzugsweise um wässrige oder nicht wässrige Lösungen, um Suspensionen oder Emulsionen handeln. Als Lösungsmittel bzw. Vehikel lassen sich Wasser, Propylenglykol, Polyethylenglykol und Pflanzenöle, insbesondere Olivenöl, organische Ester für die Injektion, beispielsweise Ölsäureethylester, oder andere geeignete organische Lösungsmittel verwenden. Diese Zusammensetzungen können auch Adjuvantien, insbesondere Netzmittel, Mittel zur Einstellung der Isotonizität, Emulgatoren, Dispersionsmittel und Stabilisatoren enthalten. Die Sterilisierung kann auf mehrere Arten erfolgen, beispielsweise durch eine aseptische Filtration, durch Einbringen von Sterilisierungsmitteln in die Zusammensetzung, durch Bestrahlen öder durch Erhitzen. Die Zusammensetzungen können auch in Form sterilen festen Zusammensetzungen hergestellt werden, die bei der Verwendung in sterilem Wasser oder einem anderen sterilen Injektionsmedium gelöst werden.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel (I) mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im Allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1 % bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Die Erfindung bezieht sich weiterhin auf Zwischenverbindungen von Verbindungen gemäß der allgemeinen Formel (I). Zwischenverbindungen gemäß der allgemeinen Formel (XI), erhältlich mit den vorstehend beschriebenen Verfahren, sind auch Gegenstand der vorliegenden Erfindung. Die Zwischenverbindungen (XI) sind insbesondere dann von Bedeutung, wenn in Verbindungen der Formel (I) der Linker X eine einfache Bindung ist und der Substituent Ar mit R3 zumindest monosubstituiert ist. 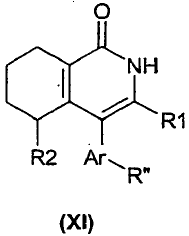 wobei
- R1: ist Wasserstoff, Fluor, Chlor, -CN, Methoxy, -OCF₃ oder C₁-C₃-Alkyl, das gegebenenfalls mit Hydroxy, Chlor, Methoxy oder einem, zwei oder drei Fluoratomen substituiert ist;
- R2: ist Wasserstoff, Fluor, -CN, Hydroxy, Methoxy, -OCF₃, -NH₂, -NH(C₁-C₃-Alkyl), -N(C₁-C₃-Alkyl)₂ oder C₁-C₃-Alkyl, das gegebenenfalls mit Hydroxy, Chlor, Methoxy oder einem, zwei oder drei Fluoratomen substituiert ist;
- R8: ist C₁-C₃-Alkoxy, -O-Phenyl, C₁-C₃-Alkyl, -NH₂, -NH(C₁-C₃-Alkyl), -N(C₁-C₃-Alkyl)₂ oder Phenyl,
und die vorstehenden Phenyl-Fragmente können wiederum mit Fluor, Chlor, Brom, Oxo, -CF₃, -OCF₃, -NO₂, -CN, Aryl, Heteroaryl, -NHC(O)(C₁-C₃-Alkyl), -COOH, Hydroxy, C₁-C₃-Alkyl, C₁-C₃-Alkoxy, -SO₂NH₂, -SO₂NH(C₁-C₃-Alkyl), -SO₂N(C₁-C₃-Alkyl)₂, -C(O)NH₂, -C(O)NH(C₁-C₃-Alkyl), -C(O)N(C₁-C₃-Alkyl)₂, -SO₂(C₁-C₃-Alkyl), -NH₂, -NH(C₁-C₃-Alkyl) oder -N(C₁-C₃-Alkyl)₂ zumindest monosubstituiert sein;
- Ar: ist Aryl oder Heteroaryl,
wobei dieses Aryl oder Heteroaryl gegebenenfalls mit zumindest einem Substituenten substituiert ist ausgewählt aus der Gruppe bestehend aus: Fluor, Chlor, Brom, -CF₃, -OCF₃, -NO₂, -CN, -C(O)R8, -NH₂, -NHC(O)(C₁-C₆-Alkyl), Hydroxy, C₁-C₆-Alkyl, C₁-C₆-Alkoxy, -CH₂-CH₂-CH₂-, -CH₂-O-C(O)-, - CH₂-C(O)-O-, -CH₂-NH-C(O)-, -CH₂-N(CH₃)-C(O)-, -CH₂-C(O)-NH-, -NH(C₁-C₆-Alkyl), -N(C₁-C₆-Alkyl)₂, -SO₂(C₁-C₆-Alkyl), Heterocyclyl, Heteroaryl und Aryl,
und von diesen Substituenten können Heterocyclyl, Aryl und Heteroaryl wiederum mit C₁-C₆-Alkyl, C₁-C₆-Alkoxy, Fluor, Chlor, Brom, Trifluormethyl, Trifluormethoxy oder ÖH zumindest monosubstituiert sein;
- R": ist -COOH, -CHO oder -SO₂Cl;
Heteroaryl ist ein 5 bis 10-gliedriger, aromatischer, mono- oder bicyclischer Heterocyclus, der ein oder mehrere Heteroatome ausgewählt aus N, O und S enthält;
Heterocyclyl ist ein 5 bis 10-gliedriger, nicht-aromatischer, mono- oder bicyclischer Heterocyclus, der ein oder mehrere Heteroatome ausgewählt aus N, O und S enthält;
Aryl ist ein 5 bis 10-gliedriger, aromatischer, Mono- oder Bicyclus.
Verbindungen der Formel (XI) sind bevorzugt, worin:
- R1: ist Wasserstoff oder C₁-C₃-Alkyl;
- R2: ist Wasserstoff;
- Ar: ist Phenyl, Thienyl, Furanyl oder Pyridinyl, wobei dieses Phenyl, Thienyl, Furanyl oder Pyridinyl gegebenenfalls mit zumindest einem Substituenten substituiert ist ausgewählt sind aus der Gruppe bestehend aus:Fluor, Chlor, -CF₃, -OCF₃, -C(O)(C₁-C₃-Alkyl), -NH₂ - NHC(O)(C₁-C₃-Alkyl), Hydroxy, C₁-C₃-Alkyl, C₁-C₃-Alkoxy, -CH₂-CH₂-CH₂-, - CH₂-O-C(O)-, -CH₂-C(O)-O-, -CH₂-NH-C(O)-, -CH₂-N(CH₃)-C(O)-, -CH₂-C(O)-NH-, -NH(C₁-C₃-Alkyl), -N(C₁-C₃-Alkyl)₂, und -SO₂(C₁-C₃-Alkyl);
- R": ist -COOH, -CHO oder -SO₂Cl;
Verbindungen der Formel (XI) sind besonders bevorzugt, worin
- R1: ist Wasserstoff oder C₁-C₃-Alkyl;
- R2: ist Wasserstoff;
- Ar: ist Phenyl, Furanyl, Thienyl oder Pyridinyl, wobei R" und das Tetrahydroisochinolinon-Fragment in meta-Position zueinander stehen;
- R": ist -CHO oder -COOH.

### Experimenteller Teil

### Liste der Abkürzungen

- ^{t}Bu: tert. Butyl
- dba: Dibenzylidenaceton
- DCM: Dichlormethan
- DMAP: 4-Dimethylaminopyridin
- DMF: N,N-Dimethylformamid
- D6-DMSO: deutereriertes Dimethylsulfoxid
- eq.: Moläquivalent
- MP: stärker quervernetztes makroporöses Polystyrol
- NBS: N-Bromsuccinimid
- PdCl₂(dppf): 1,1'Bis(diphenylphosphino)ferrocen-palladium(II)chlorid
- PPA: Propanphosphonsäureanhydrid
- RF: Rückfluss
- RT: Raumtemperatur
- RP-HPLC: Umkehrphasen-Hochleistungschromatographie
- SCX: Kationenaustauscher ('strong cation exchanger')
- TFFH: Tetramethylfluoroamindiniumhexafluorophosphat
- TFA: Trifluoressigsäure
- TFH: Tetrahydrofuran
- TMS: Trimethylsilyl
- TOTU: O-[(Ethoxycarbonyl)-cyanmethylenamino]-N,N,N',N'-tetramethyluroniumtetra-fluoroborat

### Synthese der Phtalimide der Formel II

Die Synthese gemäß Schema 1 wird anhand der Verbindung 1 demonstriert:
2-(1,3-Dioxo-1,3-dihydro-isoindol-2-yl)-propionsäureethylester (Verbindung 1) 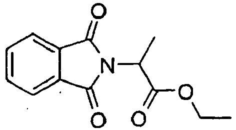 20,87 g (0,141 mol) Phtalsäureanhydrid wird zusammen mit 16,5 g (0,141 mol) Aminopropionsäureethylester im offenen Rundkolben 5 h auf 120°C erhitzt. Die Mischung wird mit 300 mL Cyclohexan versetzt und zum Rückfluss erhitzt: Die heisse Cyclohexanlösung wird vom rückständigen Öl abdekantiert und vollständig eingeengt. Man erhät ein zähes Öl das langsam erstarrt (31 g, 89% Ausbeute).
   MS: m/z = 248 (M+1)
   1 H-NMR (CDCl3): δ = 7,87 (2H, m); 7,74 (2H, m); 4,96 (1H, q, J=7,3Hz); 4,21 (2H, m); 1,70 (3H, d, J=7,3Hz); 1,24 (3H, t, J = 7,1 Hz).

### 4-Hydroxy-3-methyl-2H-isochinolin-1-on (Verbindung 2)

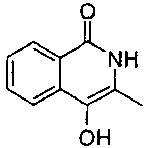

31,8 g (129 mmol) Verbindung 1 werden in 22 mL absolutem Methanol gelöst und mit 15,2 mL 28% Natriummethanolat-Lösung (257 mmol) versetzt und 3 h zum Rückfluss erhitzt. Die Lösung wird eingeengt und der Rückstand mit konz. Wässriger Ammoniaklösung versetzt. Nach 2 h wird der Feststoff abgesaugt und mit kaltem Wasser gewaschen. Man erhält 14,1 g (63% Ausbeute) weißen Feststoff.
MS: m/z =176 (M+1)
1 H-NMR (CD3OD): δ =8,21 (1H, d, J=8,8Hz); 8,14 (1H, d, J=8,3Hz); 7,65 (1H, m); 7,38 (1H, m); 2,30 (3H, s).

### 3-Methyl-2H-isochinolin-1-on (Verbindung 3)

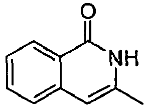

24,6 g (140 mmol) 4-Hydroxy-3-methyl-2H-isochinolin-1-on werden zusammen mit 9,13 g (294 mmol) rotem Phosphor 7 Tage in 55% lodwasserstoffsäure (130 mL) auf 160°C erhitzt. Die abgekühlte Mischung wird auf Wasser geschüttet und mit Dichlormethan extrahiert. Man erhält 10,2 g (46% Ausbeute) eines weißen Feststoffs.
MS: m/z =160 (M+1)
1H-NMR (CDCl3): δ = 10,55 (1H, s); 8,37 (1H, d, J=8,1 Hz); 7,62 (1H, m); 7,43 (2H, m); 6,31 (1H, s); 2,37 (3H, s).

### 3-Methyl-5,6,7,8-tetrahydro-2H-isochinolin-1-on (Verbindung 4)

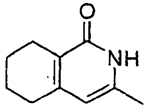

8,45 g (53 mmol) 3-Methyl-2H-isochinolin-1-on werden in 80 mL Eisessig gelöst, mit 168 mg (0,74 mmol) Platin(IV)oxid versetzt und bei 5 bar bei RT 8 h hydriert. Die Suspension wird filtriert, eingeengt und der Rückstand aus Methanol umkristallisiert. Man erhält 7,15 g (83%) eines farblosen Öls.
MS: m/z = 164 (M+1)
1 H-NMR (CDCl3): δ = 10,7 (1H, s); 5,78 (1H, s); 2,50 (4H, m); 2,22 (3H, s); 1,73 (4H, m).

### 5,6,7,8-Tetrahydro-2H-isochinolin-1-on (Verbindung 5)

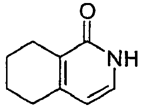

60 g (413 mmol) 2H-Isochinolin-1-on (=Isocarbostyril) werden in 1 L Eisessig gelöst, mit 2,3 g (10mmol) Platin(IV)oxid versetzt und bei 3 bar bei RT bis zum vollständigen Umsatz hydriert. Dabei wurde nach 3 Tagen und weiteren 5 Tagen der Katalysator gewechselt. Die Suspension wird filtriert, eingeengt und erneut nach Zusatz von Toluol eingeengt. Das Rohprodukt wird aus 1,6 L Wasser umkristallisiert. Lange Nadeln werden abfiltriert, die Mutterlauge wird eingeengt und getrocknet und an Kieselgel getrennt. Man erhält 36,8 g (60%) eines farblosen Öls.
MS: m/z = 150 (M+1)
1H-NMR (D6-DMSO): δ = 11,20 (1H, s); 7,08 (1H, d, J=6.8Hz); 5,90 (1H, d, J=6,8Hz); 2,47 (2H, m); 2,30 (2H, m); 1,64 (4H, m).

### 4-Brom-3-methyl-5,6,7,8-tetrahydro-2H-isochinolin-1-on (Verbindung 6)

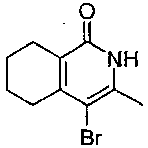

7,15 g (43,8 mmol) Verbindung 4 werden in 50 mL Methanol bei 0°C gelöst und portionsweise mit insgesamt 7,4 g (41,6 mmol) N-Bromsuccinimid versetzt. Nach 3 h Reaktionszeit bei 0° wird abgesaugt und der Rückstand mit wenig kaltem Wasser und Dichlormethan gewaschen. Man erhält 8,9 g (84% Ausbeute) des farblosen Bromids.
MS: m/z = 242/244 (M+1)
1H-NMR (CDCl3): δ = 12,4 (1H, s); 2,57 (4H, m); 2,43 (3H, s); 1,75 (4H, m).

### 4-Brom-5,6,7,8-tetrahydro-2H-isochinolin-1-on (Verbindung 7)

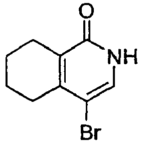

32,4 g (217 mmol) Tetrahydroisochinolinon (Verbindung 5) wird bei 5°C in 300 mL Chloroform gelöst und dann mit 11,2 mL (34,7 g, 217 mmol) Brom, gelöst in 150 mL Chloroform, tropfenweise innerhalb von 1 h versetzt. Es wird eine weitere Stunde unter Eiskühlung nachgerührt und dann mit 150 ml gesättigter NaHCO3-Lösung neutralisiert und abgesaugt. Die organische Phase des Filtrats wird mit Dichlormethan extrahiert und eingeengt. Der Rückstand wird mit Essigester versetzt, abgesaugt und mit dem ersten Rückstand zusammen im Vakuumtrockenschrank bei 45 °C getrocknet. Man erhält 47,7 g (96%) farbloses Bromid.
MS: m/z = 228/230 (M+1)
1H-NMR (CDCl3): δ = 9,50 (1H, s); 8,03 (1H, s); 2,28 (2H, m); 2,18 (2H, m); 1,85 (4H, m).

### 4-Bromo-1-methoxy-3-methyl-5,6,7,8-tetrahydro-isochinolin (Verbindung 8)

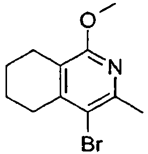

8,9 g (36,8 mmol) 4-Brom-3-methyl-5,6,7,8-tetrahydro-2H-isochinolin-1-on werden in 200 mL Chloroform gelöst, mit 13,7 g (49,6 mmol) Silber(I)carbonat und 16,2 mL (36,5 g, 257 mmol) Methyliodid versetzt und 3 h bei 50°C gerührt. Nach weiteren 24 h bei RT wird die Suspension über Celite filtriert, eingeengt und über Kieselgel mit n-Heptan unter Zusatz von 2% Essigester chromatographiert. Man erhält ein farbloses Öl (7,8 g, 83% Ausbeute).
MS: m/z = 256/258 (M+1)
1H-NMR (CDCl3): δ = 3,89 (3H, s); 2,65 (2H, m); 2,53 (2H, m); 2,51 (3H, s); 1,75 (4H, m).

### 4-Bromo-1-methoxy-5,6,7,8-tetrahydro-isochinolin (Verbindung 9)

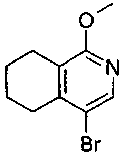

47,7 g (209 mmol) 4-Brom-5,6,7,8-tetrahydro-2H-isochinolin-1-on werden in 1 L Chloroform gelöst, mit 77,8 g (282 mmol) Silber(I)carbonat und 53,5 mL (118,7 g, 836 mmol) Methyliodid versetzt und 3 h bei 50°C gerührt. Nach dem Abkühlen wird die Suspension über Celite filtriert, eingeengt und über Kieselgel mit n-Heptan unter Zusatz von 2% Essigester chromatographiert. Man erhält ein farbloses Öl (42,5 g, 84% Ausbeute).
MS: m/z = 242/244 (M+1)
1H-NMR (CDCl3): δ = 8,03 (1H, s); 3,90 (3H, s); 2,66 (2H, m); 2,57 (2H, m); 1,78 (4H, m).

### 1-Methoxy-3-methyl-4-(4,4,5,5-tetramethyl-[1,3,2]dioxaborolan-2-yl)-5,6,7,8-tetrahydroisochinolin (Verbindung 10)

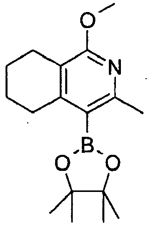

128 mg (0, 5 mmol) des Bromids 8, 218 µL (1,64 mmol) Triethylamin und 83 mg (0,65 mmol) 4,4,5,5-Tetramethyl-1,3,2-dioxaborolan werden in 2 mL Dioxan unter Argon gelöst, zuletzt werden 19 mg (0,027 mmol) PdCl2(dppf) zugegeben und die Mischung 60 h bei 90°C gerührt, anschließend weitere 2,5 h bei 150°C in der Mikrowelle. Die Mischung wird mit Wasser und Essigester verdünnt, die organische Phase mit Wasser gewaschen, eingeengt und über Kieselgel gereinigt. Man erhält einen weißen Feststoff (100 mg, 66% Ausbeute).
MS: m/z = 304 (M+1)
1H-NMR (CDCl3): δ = 4,08 (3H, s); 2,68 (2H, m); 2,58 (3H, s); 2,52 (2H, m); 1,75 (4H, m); 1,38 (12H, s).

### 1-Methoxy-4-(4,4,5,5-tetramethyl-[1,3,2]dioxaborolan-2-yl)-5,6,7,8-tetrahydroisochinolin (Verbindung 11)

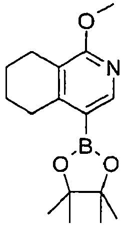

8,0 g (33 mmol) des Bromids 9, 14,4 mL (109 mmol) Triethylamin und 5,5 g (42,9 mmol) 4,4,5,5-Tetramethyl-1,3,2-dioxaborolan werden in 50 mL Dioxan unter Argon gelöst, zuletzt werden 1,28 g (1,75 mmol) PdCl2(dppf) zugegeben und die Mischung 18 h bei 90°C gerührt, Die abgekühlte Mischung wird mit Wasser versetzt, zweimal mit Essigester extrahiert, die organische Phase getrocknet, eingeengt und an Kieselgel chromatographiert. Man erhält einen weißen Feststoff (7,75 g, 81 % Ausbeute).
MS: m/z = 290 (M+1)
1H-NMR (CDCl3): δ = 8,01 (1H, s); 4,07 (3H, s); 2,66 (2H, m); 2,51 (2H, m); 1,75 (4H, m); 1,38 (12H, s).

Hartwig-Buchwald Aminierungen von Bromiden der Formel VI zu Verbindungen der Formel VIII.

### Allgemeine Arbeitsvorschrift:

1 eq. Bromid (Verbindung 8 oder 9), 1,5 eq. Anilin und 1.4 eq. NaOtBu werden in absolutem Toluol (2mL /mmol) unter Argon vorgelegt. Nach 10 min Rühren bei RT werden 0.05 eq. Tris-(dibenzylidenaceton)-di-palladium und 0.2 eq. Di-tert-butylphosphino-biphenyl zugesetzt und die Mischung 60 min bei 150°C in der Mikrowelle (CEM Discover) zur Reaktion gebracht. Die Reaktionsmischung wird mit Wasser und Essigester verdünnt, die organische Phase abgetrennt, eingeengt und per RP-HPLC gereinigt. Basische Verbindungen werden als Trifluoracetate isoliert.

### 3-(1-Methoxy-5,6,7,8-tetrahydro-isochinolin-4-ylamino)-benzoesäure-ethylester (Verbindung 12)

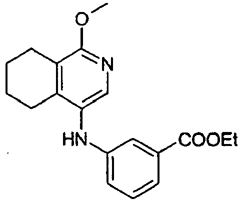

580 mg (4,9 mmol) 95%iges KO^{t}Bu werden in 20 ml abs. Toluol vorgelegt. Nachdem dreimal evakuiert und mit Argon belüftet wurde, werden 847 mg (3,5 mmol) 4-Bromo-1-methoxy-5,6,7,8-tetrahydro-isochinolin, sowie 868 mg (5,25 mmol) 3-Aminobenzoesäureethylester zugegeben. Nach der Zugabe von 160 mg (0,175 mmol) Tris-(dibenzyliden)-di-palladium, sowie 209 mg (0,7 mmol) Di-tert-butylphosphinobiphenyl wird drei Stunden auf 100 °C erhitzt. Zur Aufarbeitung wird eingeengt und der Rückstand zwischen H₂O und Essigester verteilt. Die Phasen werden getrennt und die wässrige Phase noch dreimal mit Essigester extrahiert. Die vereinigten organischen Phasen werden noch einmal mit H₂O gewaschen, mit Na₂SO₄ getrocknet und eingeengt. Die weitere Reinigung erfolgt durch Chromatographie an Kieselgel (CH₂Cl₂/MeOH 99:1), wobei 640 mg der Titelverbindung erhalten werden.
Ausbeute: 55%.

Entsprechend der allgemeinen Arbeitsvorschrift werden folgende weitere Verbindungen hergestellt:

| Verbindung | Name | |
|---|---|---|
| 13 | 3-(1-Methoxy-5,6,7,8-tetrahydro-isochinolin-4-ylamino)-phenol | 271 (M+1) |
| 14 | 3-(1-Methoxy-5,6,7,8-tetrahydro-isochinolin-4-ylamino)-N-methyl-benzamid | 312 (M+1) |
| 15 | (1-Methoxy-5,6,7,8-tetrahydro-isochinolin-4-yl)-(4-pyridin-4-yl-thiazol-2-yl)-amin | 339 (M+1) |
| 16 | (1-Methoxy-5,6,7,8-tetrahydro-isochinolin-4-yl)-(3,4,5,6-tetrahydro-2H- [1,2']bipyridinyl-5'-yl)-amin | 339 (M+1) |
| 17 | N-[3-(1-Methoxy-5,6,7,8-tetrahydro-isochinolin-4-ylamino)-phenyl]-acetamid | 312 (M+1) |
| 18 | (3-Methansulfonyl-phenyl)-(1-methoxy-5,6,7,8-tetrahydro-isochinolin-4-yl)-amin | 333 (M+1) |
| 19 | (3-Methansulfonyl-phenyl)-(1-methoxy-3-methyl-5,6,7,8-tetrahydro-isochinolin-4-yl)- amin | 337 (M+1) |
| 20 | [3-(1-Methoxy-5,6,7,8-tetrahydro-isochinolin-4-ylamino)-phenyl]-methanol | 285 (M+1) |
| 21 | (6-Methoxy-pyridin-3-yl)-(1-methoxy-5,6,7,8-tetrahydro-isochinolin-4-yl)-amin | 286 (M+1) |
| 22 | 6-(1-Methoxy-5,6,7,8-tetrahydro-isochinolin-4-ylamino)-3H-isobenzofuran-1-on | 311 (M+1) |
| 23 | (3-Ethoxy-phenyl)-(1-methoxy-5,6,7,8-tetrahydro-isochinolin-4-yl)-amin | 299 (M+1) |
| 24 | (6-Chloro-pyridin-3-yl)-(1-methoxy-5,6,7,8-tetrahydro-isochinolin-4-yl)-amin | 290 (M+1) |
| 25 | 3-(1-Methoxy-3-methyl-5,6,7,8-tetrahydro-isochinolin-4-ylamino)-N,N-dimethyl- benzamid | 340 (M+1) |
| 26 | (1-Methoxy-3-methyl-5,6,7,8-tetrahydro-isochinolin-4-yl)-(3-trifluoromethoxy-phenyl)- amin | 353 (M+1) |
| 27 | 1-[3-(1-Methoxy-5,6,7,8-tetrahydro-isochinolin-4-ylamino)-phenyl]-ethanon | 297 (M+1) |
| 28 | 1-[3-(1-Methoxy-3-methyl-5,6,7,8-tetrahydro-isochinolin-4-ylamino)-phenyl]-ethanon | 311 (M+1) |
| 29 | N-[3-(1-Methoxy-3-methyl-5,6,7,8-tetrahydro-isochinolin-4-ylamino)-phenyl]- acetamid | 326 (M+1) |

Synthese von Verbindungen der Formel X durch Suzuki-Kupplung mit Bromiden der Formel VI und Boronsäuren der Formel (OH)₂B-Ar-R"

### Allgemeine Arbeitsvorschrift

1 eq. Bromid (Verbindung 8 oder 9), 3,3 eq. Kaliumiodid, 1,2 eq. Boronsäure werden unter Argon in absolutem THF (3 ml/1 mmol) vorgelegt. Es wird 0.05 eq. Pd2dba3 und 0.1 eq. Tri-tert-butylphosphoniumterafluoroborat zugesetzt und die Mischung 15 h bei 60°C gerührt. Nach dem Abkühlen wird mit gesättigter NaHC03-Lösung neutralisiert und zweimal mit Essigester extrahiert und die organische Phasen eingeengt. Der Rückstand wird über Kieselgel chromatographiert.

### 1-Methoxy-3-methyl-4-thiophen-2-yl-5,6,7,8-tetrahydro-isochinolin (Verbindung 30)

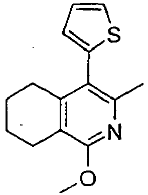

Entsprechend der allgemeinen Arbeitsvorschrift werden aus 70 mg Bromid 8 22 mg (31 % Ausbeute) der Verbindung 30 erhalten.
MS: m/z = 260 (M+1)
1H-NMR (CDCl3): δ = 7,37 (1H, dd, J=5.1, 1.1 Hz); 7,09 (1H, dd, J=5.1, 3.4 Hz); 6,79 (1H, dd, 3.4, 1.1 Hz); 3.96 (3H, s); 2,58 (2H, m); 2,40 (2H, m); 2,24 (3H, s); 1,70 (4H, m).

### 1-Methoxy-3-methyl-4-pyridin-3-yl-5,6,7,8-tetrahydro-isochinolin (Verbindung 31)

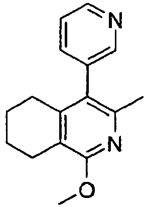

Entsprechend der allgemeinen Arbeitsvorschrift werden aus 100 mg Bromid 8 49 mg (49% Ausbeute) der Verbindung 31 erhalten.
MS: m/z = 260 (M+1)

### 5-(1-Methoxy-5,6,7,8-tetrahydro-isochinolin-4-yl)-furan-2-carbaldehyd (Verbindung 32)

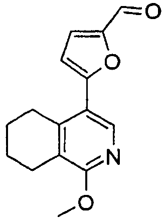

Entsprechend der allgemeinen Arbeitsvorschrift werden aus 4,32 g (17,9 mmol) Bromid 94,59 g (100% Ausbeute) der Verbindung 32 erhalten.
MS: m/z = 258 (M+1)

### 5-(1-Methoxy-3-methyl-5,6,7,8-tetrahydro-isochinolin-4-yl)-furan-2-carbaldehyd (Verbindung 33)

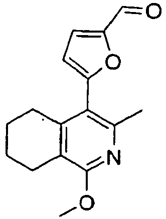

Entsprechend der allgemeinen Arbeitsvorschrift werden aus 2,0 g Bromid 8 1,86 g (79% Ausbeute) der Verbindung 33 erhalten.
MS: m/z = 272 (M+1)
1H-NMR (CDCl3): δ = 9,65 (1H, s); 7,34 (1H, d, J=3.4 Hz); 6,49 (1H, d, J=3.4 Hz); 3.96 (3H, s); 2,57 (2H, m); 2,46 (2H, m); 2,30 (3H, s); 1,71 (4H, m).

### Synthese von Verbindungen der Formel X durch Suzuki-Kupplung mit Boronsäurestern der Formel VII und Bromiden der Formel Br-Ar-R3

Die Synthese ist exemplarisch an folgender Verbindung gezeigt:

### 5-(1-Methoxy-5,6,7,8-tetrahydro-isochinolin-4-yl)-nicotinsäure (Verbindung 34)

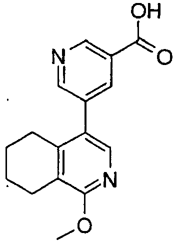

7,75 g (26,8 mmol) Boronsäureester 11, 14,82 g (107,2 mmol) Kaliumcarbonat und 980 mg (1,34 mmol) PdCl2(dppf) werden in 60 mL absolutem DMF unter Argon vorgelegt und 7 g (34,9 mmol) 5-Bromnicotinsäure zugesetzt. Die Mischung wird 18 h bei 110°C gerührt, mit Wasser versetzt und mit 2N HCL-Lösung auf pH4 eingestellt. Es wird zweimal mit DCM extrahiert, die organische Phase filtriert und eingeengt. Der Rückstand wird in 200 mL DCM aufgenommen, mit Aktivkohle versetzt und über Celite filtriert. Das Filtrat wird eingeengt und über Nacht im Vakuumtrockenschrank getrocknet. Man erhält 7,4 g (97% Ausbeute) dunkler Kristalle.
MS: m/z = 285 (M+1)

### Synthese der Verbindungen 35 - 39 (Zwischenprodukte) durch Entschützen der Methoxygruppe:

5-(1-oxo-1,2,5,6,7,8-hexahydro-isochinolin-4-yl)-furan-2-carbaldehyd (Verbindung 35)

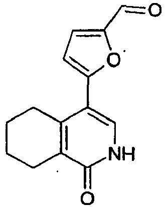

4,59 g (17,8 mmol) Verbindung 32 werden in 60 mL Acetonitril suspendiert. Nach Zugabe von 3,26 g (19,6 mmol) Kl und 2,13 g (19,6 mmol) Trimethylchlorsilan wird unter Argon 6 h auf 60°C behandelt. Die Mischung wird komplett eingeengt und der Rückstand mit wässriger NaHC03-Lösung behandlet. Der verbliebene Rückstand wird dann 3 x mit DCM extrahiert, die organischen Phasen getrocknet und eingeengt. Der Rückstand wird in 100 mL EtOH gelöst und mit Norit-Kohle versetzt. Es wird abfiltriert und eingeengt. Man erhält ca. 1g der Titelverbindung. Aus dem Kohlerückstand wird weiteres Rohprodukt durch Extraktion mit DCM isoliert und über Kieselgel gereinigt. Gesamtausbeute: 2,38 g (55%) weißer Feststoff.
MS: m/z = 244 (M+1) 1H-NMR (CDCl3): δ =12,6 (1H, s); 9,65 (1H, s); 7, 75 (1H, s); 7,29 (1H, d, J=3.4 Hz); 6,56 (1H, d, J=3.4 Hz); 2,75 (2H, m); 2,62 (2H, m); 1,70 (4H, m).

### 5-(3-Methyl-1-oxo-1,2,5,6,7,8-hexahydro-isochinolin-4-yl)-furan-2-carbaldehyd (Verbindung 36)

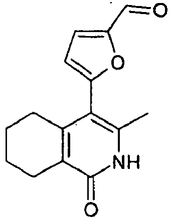

Analog zu Verbindung 35 werden aus 660 mg (2,43 mmol) Verbindung 33 172 mg (27% Ausbeute) der Titelverbindung nach Reinigung durch RP-HPLC erhalten.
MS: m/z = 258 (M+1)
1H-NMR (CDCl3): δ = 11,4 (1H, br s); 9,65 (1H, s); 7,35 (1H, d, J=3.4 Hz); 6,58 (1H, d, J=3.4 Hz); 2,61 (2H, m); 2,43 (2H, m); 2,35 (3H, s); 1,72 (4H, m).

### 5-(1-Oxo-1,2,5,6,7,8-hexahydro-isochinolin-4-yl)-nicotinsäure (Verbindung 37)

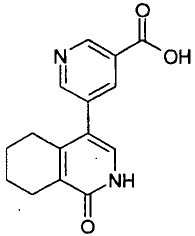

3,1 g (28,5 mmol) Verbindung 34 werden in 100 mL Acetonitril suspendiert. Nach Zugabe von 4,73 g (28,5 mmol) Kl und 3,1 g (28,5 mmol) Trimethylchlorsilan wird unter Argon 2 h auf 80°C erwärmt. Die Mischung wird auf RT abgekühlt, komplett eingeengt und in Essigester/MeOH 5:1 aufgenommen. Der Niederschlag (4,78 g) wird abgesaugt und im Vakuumtrockenschrank bei 45°C über Nacht getrocknet. Die Mutterlauge wird eingeengt und der Rückstand über Kieselgel gereinigt. Gesamtausbeute: 5,64 g (81%) beiger Feststoff.
MS: m/z = 271 (M+1)
1H-NMR (D6-DMSO): δ = 11,7 (1H, br s); 9,03 (1H, d, J=2.0Hz); 8,74 (1H, d, J=2.2 Hz); 8,12 (1H, t, J=2.2 Hz); 2,41 (2H, m); 2,32 (2H, m); 1,63 (4H, m).

### 3-(1-Oxo-1,2,5,6,7,8-hexahydro-isochinolin-4-ylamino)-benzoesäure-ethylester (Verbindung 38)

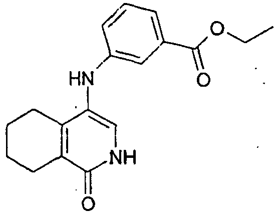

560 mg (1,7 mmol) 3-(1-Methoxy-5,6,7,8-tetrahydro-isochinolin-4-ylamino)-benzoesäure-ethylester (Verbindung 12) werden in 30 ml Chloroform gelöst und bei Raumtemperatur mit 413 mg (2,1 mmol) Trimethylsilyliodid versetzt. Danach wird zum Rückfluß erhitzt bis mittels LCMS vollständiger Umsatz erreicht ist. Eventuell wird weiteres Trimethylsilyliodod zugegeben. Zur Aufarbeitung wird zweimal mit H₂O gewaschen, mit Na₂SO₄ getrocknet und eingeengt. Chromatographie an Kieselgel (CH₂Cl₂/MeOH 95:5) liefert 216 mg der Titelverbindung. Ausbeute: 40 %.
MS: m/z = 313 (M+1)

### 3-(1-Oxo-1,2,5,6,7,8-hexahydro-isochinolin-4-ylamino)-benzoesäure (Verbindung 39)

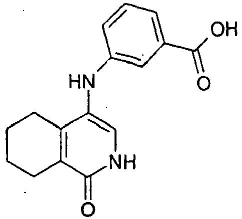

180 mg (0,58 mmol) 3-(1-Methoxy-5,6,7,8-tetrahydro-isochinolin-4-ylamino)-benzoesäure-ethylester (Verbindung 38) werden in 3 ml Ethanol vorgelegt und bei Raumtemperatur mit 3 ml 2N KOH versetzt. Nach zwei Stunden wird das Lösungsmittel i. Vak. entfernt, der Rückstand in 5 ml H₂O aufgenommen und mit 2N HCl angesäuert. Der entstandene Niederschlag wird abgesaugt und getrocknet. Ausbeute: 123 mg (75 %).
MS: m/z = 285 (M+1)

### Synthese von Tetrahydro-2H-isochinolinonen der Formel I durch Entschützen der 1-Methoxy-5,6,7,8-tetrahydroisochinoline der Formeln VIII und X.

### Allgemeine Arbeitsvorschrift

Zu einer Mischung des 1-Methoxy-5,6,7,8-tetrahydroisochinoline der Formel VIII und IX in wasserfreiem Acetonitril (3-5 mL/mmol) werden 2 eq. Kaliumiodid und 2 eq. Trimethylchlorsilan unter Argon gegeben und die trübe Mischung 1-3 h bei 60-80°C erhitzt. Die Mischung wird anschließend auf RT abgekühlt und eingeengt. Das Rohprodukt wird per RP-HPLC gereinigt, basische Verbindungen werden als Trifluoracetate isoliert.

Entsprechend der allgemeinen Vorschrift werden folgende Beispiele synthetisiert:

| **Beispiel** | **Name** | **Masse (ES+) m/z =** | **NMR (D6-DMSO) δ=** |
|---|---|---|---|
| 1 | 4-(3-Hydroxy-phenylamino)-5,6,7,8-tetrahydro-2H-isochinolin-1-on | 257 (M+1) | |
| 2 | N-Methyl-3-(1-oxo-1,2,5,6,7,8-hexahydro-isochinolin-4-ylamino)-benzamid | 298 (M+1) | |
| 3 | 4-(4-Pyridin-4-yl-thiazol-2-ylamino)-5,6,7,8-tetrahydro-2H-isochinolin-1-on | 325 (M+1) | |
| 4 | 4-(6-Morpholin-4-yl-pyridin-3-ylamino)-5,6,7,8-tetrahydro-2H-isochinolin-1-on | 325 (M+1) | |
| 5 | N-[3-(1-Oxo-1,2,5,6,7,8-hexahydro-isochinolin-4-ylamino)-phenyl]-acetamid | 298 (M+1) | |
| 6 | 4-(3-Methansulfonyl-phenylamino)-5,6,7,8-tetrahydro-2H-isochinolin-1-on | 319 (M+1) | |
| 7 | 4-(3-Methansulfonyl-phenylamino)-3-methyl-5,6,7,8-tetrahydro-2H-isochinolin-1-on | 333 (M+1) | 11.47 (1H, br s), 7.48 (1H, s), 7.32 (1H, m), 7.09 (1H, d, J=8.6 Hz), 6.95 (1H, br s), 6.67 (1H, br s), 3.13 (3H, s), 2.33 (4H, m), 2.01 (3H, s), 1.58 (4H, m) |
| 8 | 4-(3-Hydroxymethyl-phenylamino)-5,6,7,8-tetrahydro-2H-isochinolin-1-on | 271 (M+1) | |

### 4-(5-Hydroxymethyl-furan-2-yl)-3-methyl-5,6,7,8-tetrahydro-2H-isochinolin-1- on (Beispiel 9)

Die Titelverbindung wird bei der reduktiven Aminierung von 5-(3-Methyl-1-oxo-1,2,5,6,7,8-hexahydro-isochinolin-4-yl)-furan-2-carbaldehyd (Verbindung 36) als Nebenprodukt erhalten und per RP-HPLC isoliert.
MS: m/z = 260 (M+1)
1H-NMR (D6-DMSO): δ = 11,52 (1H, br s); 6,33 (1H, d, J=2.9 Hz); 6,27 (1H, d, J=2.9 Hz); 4,38 (2H, s); 2,33 (2H, m); 2,23 (2H, m); 2,03 (3H, s); 1,59 (4H, m).

Die nachfolgenden Beispiele 10 bis 19 werden entsprechend zu den Beispielen 1 bis 8 hergestellt.

| **Beispiel** | **Name** | **Masse (ES+) m/z =** | **NMR (D6-DMSO) δ=** |
|---|---|---|---|
| 10 | 4-(3-Oxo-1,3-dihydro-isobenzofuran-5-ylamino)-5,6,7,8-tetrahydro-2H-isochinolin-1-on | 297 (M+1) | |
| 11 | 4-(3-Ethoxy-phenylamino)-5,6,7,8-tetrahydro-2H-isochinolin-1-on | 285 (M+1) | |
| 12 | 4-(6-Chlor-pyridin-3-ylamino)-5,6,7,8-tetrahydro-2H-isochinolin-1-on | 276 (M+1) | |
| 13 | N,N-Dimethyl-3-(3-methyl-1-oxo-1,2,5,6,7,8-hexahydro-isochinolin-4-ylamino)-benzamid | 326 (M+1) | |
| 14 | 3-Methyl-4-(3-trifluormethoxy-phenylamino)-5,6,7,8-tetrahydro-2H-isochinolin-1-on | 339 (M+1) | |
| 15 | 4-(3-Acetyl-phenylamino)-5,6,7,8-tetrahydro-2H-isochinolin-1-on | 283 (M+1) | |
| 16 | 4-(3-Acetyl-phenylamino)-3-methyl-5,6,7,8-tetrahydro-2H-isochinolin-1-on | 297 (M+1) | 11.42 (1H, br s), 7.21 (2H, m), 7.18 (1H, m), 6.98 (1H, br s), 6.63 (1H, br s), 2.48 (3H, s), 2.32 (4H, m), 2.00 (3H, s), 1.57 (4H, m) |
| 17 | N-[3-(3-Methyl-1-oxo-1,2,5,6,7,8-hexahydro-isochinolin-4-ylamino)-phenyl]-acetamid | 312 (M+1) | |
| 18 | 3-Methyl-4-thiophen-2-yl-5,6,7,8-tetrahydro-2H-isochinolin-1-on | 246 (M+1) | |
| 19 | 3-Methyl-4-pyridin-3-yl-5,6,7,8-tetrahydro-2H-isochinolin-1-on | 241 (M+1) | |

### Synthese der Beispiele 20-24 durch Kupplung von Verbindung 39 mit Aminen

### Algemeine Arbeitsvorschrift

38 mg (0,13 mmol) 3-(1-Methoxy-5,6,7,8-tetrahydro-isochinolin-4-ylamino)-benzoesäure werden in 2 ml DMF vorgelegt und bei Raumtemperatur mit 20 µL (0,15 mmol) Triethylamin versetzt. Bei 0 °C werden 52 mg (0,16 mmol) TOTU zugegeben und noch 15 min bei 0 °C gerührt. Nach weiteren 30 Minuten bei Raumtemperatur wird diese Lösung zu einer zweiten Lösung, bestehend aus dem jeweiligen Amin (0,15 mmol), 20 µL (0,15 mmol) Triethylamin in 2 ml DMF, gegeben und bei Raumtemperatur gerührt bis mittels LCMS vollständiger Umsatz festgestellt wird. Zur Aufarbeitung wird vom Lösungsmittel befreit und an Kieselgel gereinigt.

Die folgenden Verbindungen werden nach der angegebenen allgemeinen Arbeitsvorschrift hergestellt:
N-Butyl-3-(1-oxo-1,2,5,6,7,8-hexahydro-isochinolin-4-ylamino)-benzamid (Beispiel 20)

Ausbeute nach Kieselgelchromatographie (CH₂Cl₂/MeOH 95:5) 31%.
Rₜ = 1,166min¹⁾; MS(M+H⁺) = 340,15; 500 MHz ¹H-NMR (DMSO-d₆)[ppm]: 11.30, 1H, s, NH; 7.20-7.00, m, 5H, 4 x aromat. H, NH; 6.62, dd, 1H, aromat. H; 3.20, m, 2H, CH₂; 2.33 m, 4H, 2 x CH₂; 1.62, m, 2H, CH₂; 1.58, m, 2H, CH₂; 1.46, m, 2H, CH₂, 1.30, m, 2H, CH₂; 0.88, t, 3H, CH₃.

### 4-[3-(Pyrrolidin-1-carbonyl)-phenylamino]-5,6,7,8-tetrahydro-2H-isochinolin-1-on

### (Beispiel 21)

Ausbeute nach Kieselgelchromatographie (CH₂Cl₂/MeOH 95:5) 74%.
Rₜ = 1,052 min¹⁾; MS(M+H⁺) = 338,15; 500 MHz ¹H-NMR (DMSO-d₆)[ppm]: 11.30, 1H, s, NH; 7.22-7.05, m, 3H, 2 x aromat. H, NH; 6.71, dd, 1H, aromat. H; 6.63-6.55, m, 2H, aromat. H; 3.08, m, 4H, 2 x CH₂; 2.33, m, 4H, 2 x CH₂; 1.90-1.73, m, 4H, 2 x CH₂; 1.68-1.52, m, 4H, 2 x CH₂.

### 4-[3-(4-Cyclopropylmethyl-piperazin-1-carbonyl)-phenylamino]-5,6,7,8-tetrahydro-2H-isochinolin-1-on (Beispiel 22)

Ausbeute nach Kieselgelchromatographie (CH₂Cl₂/MeOH 95:5) 67%.
Rₜ = 0,839 min¹⁾; MS(M+H⁺) = 407,25.

### 3-(1-Oxo-1,2,5,6,7,8-hexahydro-isochinolin-4-ylamino)-N-(2-pyridin-3-yl-ethyl)-benzamid (Beispiel 23)

Ausbeute nach Kieselgelchromatographie (Essigester/MeOH 10:1 → 4:1) 63%.
Rₜ = 1,03 min²⁾ ; MS(M+H⁺) = 389,11.

### 3-(1-Oxo-1,2,5,6,7,8-hexahydro-isochinolin-4-ylamino)-N-(2-pyrrolidin-1-yl-ethyl)-benzamid (Beispiel 24)

Ausbeute nach Kieselgelchromatographie (Essigester/MeOH 10:1 → MeOH) 66%.
Rₜ = 1,05 min²⁾; MS(M+H⁺) = 381,15.

### LCMS-Methode:

1)
   YMC J'sphere ODS H80 20x2, 4 µm;
   0 min 96% H2O(0,05% TFA) 2,0 min - 95% ACN; 95% ACN bis 2,4 min; 4% ACN 2,45 min;
   1 ml/min;
   30 °C.
2)
   Col YMC J'sphere 33x2, 4 µm;
   Grad ACN+0,05% TFA : H2O+0,05% TFA
   5:95 (0 min) to 95:5 (3,4 min) to 95:5 (4,4 min);
   1 ml/min;
   30 °C.

### Synthese der Beispiele 25-49 durch Kupplung von Verbindung 37 mit Aminen Allgemeine Arbeitsvorschrift

54 mg (0,2 mmol) 5-(1-Oxo-1,2,5,6,7,8-hexahydro-isochinolin-4-yl)-nicotinsäure, 0.24 mmol Amin, 0,02 mmol 4-DMAP, 0,8 mmol N-Methylmorpholin und 0,4 mmol PPA (50% Lösung in DMF) werden in 3 ml absolutem DCM bei RT vermischt und 18 h gerührt. Die Mischung wird mit etwas DCM verdünnt, mit gesättigter NaHC03-Lösung gewaschen, die organische Phase dannn eingeengt und der Rückstand per RP-HPLC gereinigt. Basische Verbindungen werden als Trilfluoracetate isoliert.

Entsprechend der allgemeinen Vorschrift werden folgende Beispiele dargestellt:

| **Beispiel** | **Name** | **Masse (ES+) m/z =** | **NMR (D6-DMSO) δ=** |
|---|---|---|---|
| 25 | 4-[5-(Pyrrol idin-1-carbonyl)-pyridin-3-yl]-5,6,7,8-tetrahydro-2H-isochinolin-1-on | 324 (M+1) | |
| 26 | 5-(1-Oxo-1,2,5,6,7,8-hexahydro-isochinolin-4-yl)-N-(2-oxo-2-phenyl-ethyl)-nicotinamid | 388 (M+1) | |
| 27 | 5-(1-Oxo-1,2,5,6,7,8-hexahydro-isochinolin-4-yl)-N-phenethyl-nicotinamid | 374 (M+1) | |
| 28 | N-(4-Nitro-benzyl)-5-(1-oxo-1,2,5,6,7,8-hexahydro-isochinolin-4-yl)-nicotinamid | 405 (M+1) | |
| 29 | N-[2-(4-Nitro-phenyl)-ethyl]-5-(1-oxo-1,2,5,6,7,8-hexahydro-isochinolin-4-yl)- nicotinamid | 417 (M+1) | |
| 30 | N-[(4-Nitro-benzoylamino)-methyl]-5-(1-oxo-1,2,5,6,7,8-hexahydro-isochinolin-4-yl)-nicotinamid | 433 (M+1) | |
| 31 | N-[2-(4-Pyridincarbamoylamino-ethyl)]-5-(1-oxo-1,2,5,6,7,8-hexahydro-isochinolin-4-yl)-nicotinamid | 418 (M+1) | |
| 32 | N-Methyl-N-(3-nitro-benzyl)-5-(1-oxo-1,2,5,6,7,8-hexahydro-isochinolin-4-yl)- nicotinamid | 419 (M+1) | |
| 33 | 5-(1-Oxo-1,2,5,6,7,8-hexahydro-isochinolin-4-yl)-N-[2-(pyrimidin-2-ylamino)-ethyl]-nicotinamid | 391 (M+1) | |
| 34 | N-(5-Methyl-isoxazol-3-ylmethyl)-5-(1-oxo-1,2,5,6,7,8-hexahydro-isochinolin-4-yl)-nicotinamid | 365 (M+1) | |
| 35 | 5-(1-Oxo-1,2,5,6,7,8-hexahydro-isochinolin-4-yl)-N-(1,3,5-trimethyl-1H-pyrazol-4-ylmethyl)-nicotinamid | 392 (M+1) | |
| 36 | 4-[5-(3-Pyridin-4-yl-pyrrolidin-1-carbonyl)-pyridin-3-yl]-5,6,7,8-tetrahydro-2H-isochinolin-1-on | 401 (M+1) | |
| 37 | N-(4-Acetylamino-benzyl)-5-(1-oxo-1,2,5,6,7,8-hexahydro-isochinolin-4-yl)- nicotinamid | 417 (M+1) | |
| 38 | N-Methyl-5-(1-oxo-1,2,5,6,7,8-hexahydro-isochinolin-4-yl)-N-pyridin-4-ylmethyl-nicotinamid | 375 (M+1) | |
| 39 | N-(5-Methyl-pyrazin-2-ylmethyl)-5-(1-oxo-1,2,5,6,7,8-hexahydro-isochinolin-4-yl)-nicotinamid | 376 (M+1) | |
| 40 | 5-(1-Oxo-1,2,5,6,7,8-hexahydro-isochinolin-4-yl)-N-(2-pyridin-3-yl-ethyl)-nicotinamid | 375 (M+1) | |
| 41 | 5-(1-Oxo-1,2,5,6,7,8-hexahydro-isochinolin-4-yl)-N-(3-phenyl-4,5-dihydro-isoxazol-5- ylmethyl)-nicotinamid | 429 (M+1) | |
| 42 | N-(4-Methansulfonyl-benzyl)-5-(1-oxo-1,2,5,6,7,8-hexahydro-isochinolin-4-yl)- nicotinamid | 438 (M+1) | |
| 43 | 5-(1-Oxo-1,2,5,6,7,8-hexahydro-isochinolin-4-yl)-N-(2-pyridin-4-yl-ethyl)-nicotinamid | 375 (M+1) | 11.60 (1H, br s), 8.88 (1H, d, J=2.1 Hz), 8.78 (1H, t, J=5.5 Hz), 8.74 (2H, d, J= 6.2 Hz), 8.65 (1H, d, J=2.1 Hz), 8.03 (1H, t, J=2.1 Hz), 7.80 (2H, d, J= 6.2 Hz), 7.18 (1H, s); 3.65 (2H, m), 3.10 (2H, t, J= 6.5 Hz); 2.41 (2H, m), 2.31 (2H, m), 1.69 (2H, m), 1.59 (2H, m). |
| 44 | 5-(1-Oxo-1,2,5,6,7,8-hexahydro-isochinolin-4-yl)-N-[2-(4-sulfamoyl-phenyl)-ethyl]-nicotinamid | 453 (M+1) | |
| 45 | N-(3-Methoxy-benzyl)-5-(1-oxo-1,2,5,6,7,8-hexahydro-isochinolin-4-yl)-nicotinamid | 390 (M+1) | 11.67 (1H, br s), 9.23 (1H, t, J=5.8 Hz), 9.00 (1H, d, J=1.9 Hz), 8.67 (1H, d, J=2.1 Hz), 8.18 (1H, t, J=2.0 Hz), 7.25 (1H, t, J=8.0 Hz), 7.20 (1H, s), 6.91 (2H, m); 6.82 1H, m), 4.49 (2H, d, J= 5.8 Hz), 3.74 (3H, s), 2.41 (2H, m), 2.34 (2H, m), 1.68 (2H, m), 1.58 (2H, m). |
| 46 | N-(2-Methoxy-benzyl)-5-(1-oxo-1,2,5,6,7,8-hexahydro-isochinolin-4-yl)-nicotinamid | 390 (M+1) | |
| 47 | N-(2,3-Dimethoxy-benzyl)-5-(1-oxo-1,2,5,6,7,8-hexahydro-isochinolin-4-yl)- nicotinamid | 420 (M+1) | |
| 48 | 5-(1-Oxo-1,2,5,6,7,8-hexahydro-isochinolin-4-yl)-N-pyridin-4-ylmethyl-nicotinamid | 361 (M+1) | |
| 49 | N-(1-Ethyl-pyrrolidin-2-ylmethyl)-5-(1-oxo-1,2,5,6,7,8-hexahydro-isochinolin-4-yl)-nicotinamid | 381 (M+1) | |

### 4-(5-Aminomethyl-furan-2-yl)-5,6,7,8-tetrahydro-2H-isochinolin-1-on (Beispiel 50)

2,18 g (9,0 mmol) Verbindung 35 werden zusammen mit 844 mg (13,4 mmol) NaCNBH4 und 55,3 g (717 mmol) Ammoniumacetat in 160 mL Methanol/THF (5/1) gelöst und 18 h bei RT zur Reaktion gebracht. Die gelbe Lösung wird komplett eingeengt und der Rückstand über Kieselgel mit Essigester/Methanol (1/1) chromatographiert. Die isolierte Verbindung die noch größere Mengen Salz enthält wird anschließend per RP-HPLC chromatographiert. Es werden 400 mg der Titelverbindung isoliert.
MS: m/z = 245 (M+1)

### Synthese der Beispiele 51 - 79 durch reduktive Aminierung der Verbindung 35 und 36 mit Aminen

### Allgemeine Arbeitsvorschrift

0,2 mmol 5-(3-Methyl-1-oxo-1,2,5,6,7,8-hexahydro-isochinolin-4-yl)-furan-2-carbaldehyd oder 5-(1-oxo-1,2,5,6,7,8-hexahydro-isochinolin-4-yl)-furan-2-carbaldehyd und 0,24 mmol Amin werden in 2 mL THF gelöst und mit ca. 0,5 mmol MP-Triacetoxyborohydride versetzt und 18 h bei RT geschüttelt. Die Lösung wird abfiltriert, das Harz zweimal mit je 4 mL THF gewaschen, die gesamte organische Phase eingeengt und per RP-HPLC gereinigt. Die Verbindungen werden als Trifluoracetate nach der Gefriertrocknung isoliert.

| **Beispiel** | **Name** | **Masse (ES+) m/z=** | **NMR (D6-DMSO) δ=** |
|---|---|---|---|
| 51 | 4-[5-(Benzylamino-methyl)-furan-2-yl]-3-methyl-5,6,7,8-tetrahydro-2H-isochinolin-1-on | 349 (M+1) | |
| 52 | 3-Methyl-4-(5-{[(1-methyl-1H-pyrazol-4-ylmethyl)-amino]-methyl}-furan-2-yl)-5,6,7,8-tetrahydro-2H-isochinolin-1-on | 353 (M+1) | |
| 53 | 4-(5-Butylaminomethyl-furan-2-yl)-3-methyl-5,6,7,8-tetrahydro-2H-isochinolin-1-on | 315 (M+1) | |
| 54 | 3-Methyl-4-(5-pyrrolidin-1-ylmethyl-furan-2-yl)-5,6,7,8-tetrahydro-2H-isochinolin-1-on | 313 (M+1) | 11.62 (1H, br s), 10.03 (1H, br s), 6.74 (1H, d, J= 3.4 Hz), 6.47 (1H, d, J=3.4 Hz), 4.46 (2H, d, J=5.2 Hz), 3.40 (2H, m), 3.11 (2H, m), 2.34 (2H, m), 2.23 (2H, m), 2.04 (3H, s), 1.86 (2H, m), 1.62 (4H, m). |
| 55 | 4-(5-{[(1-Methyl-1H-benzoimidazol-2-ylmethyl)-amino]-methyl}-furan-2-yl)-5,6,7,8-tetrahydro-2H-isochinolin-1-on | 389 (M+1) | |
| 56 | 4-[5-(4-Pyrimidin-2-yl-piperazin-1-ylmethyl)-furan-2-yl]-5,6,7,8-tetrahydro-2H-isochinolin-1-on | 392 (M+1) | |
| 57 | 4-{5-[(2-Pyrrolidin-1-yl-ethylamino)-methyl]-furan-2-yl}-5,6,7,8-tetrahydro-2H- isochinolin-1-on | 342 (M+1) | |
| 58 | 4-(5-{[(Pyridin-4-ylmethyl)-amino]-methyl}-furan-2-yl)-5,6,7,8-tetrahydro-2H- isochinolin-1-on | 336 (M+1) | |
| 59 | 4-(5-{[(Pyridin-3-ylmethyl)-amino]-methyl}-furan-2-yl)-5,6,7,8-tetrahydro-2H- isochinolin-1-on | 336 (M+1) | |
| 60 | 4-(5-{[2-(1-Methyl-pyrrolidin-2-yl)-ethylamino]-methyl}-furan-2-yl)-5,6,7,8-tetrahydro- 2H-isochinolin-1-on | 356 (M+1) | |
| 61 | 4-(5-Pyrrolidin-1-ylmethyl-furan-2-yl)-5,6,7,8-tetrahydro-2H-isochinolin-1-on | 299 (M+1) | |
| 62 | 4-(5-{[(Pyridin-2-ylmethyl)-amino]-methyl}-furan-2-yl)-5,6,7,8-tetrahydro-2H- isochinolin-1-on | 336 (M+1) | |
| 63 | 4-[5-(3-Hyd roxy-pyrrolidin-1-ylmethyl)-furan-2-yl]-5,6,7,8-tetrahydro-2H-isochinolin-1- on | 315 (M+1) | |
| 64 | 4-(5-{[(4-Pyrrolidin-1-yl-piperidin-1-ylmethyl)-amino]-methyl}-furan-2-yl)-5,6,7,8- tetrahydro-2H-isochinolin-1-on | 411 (M+1) | |
| 65 | 4-{5-[(2-Dimethylamino-ethylamino)-methyl]-furan-2-yl}-5,6,7,8-tetrahydro-2H- isochinolin-1-on | 316 (M+1) | |
| 66 | 4-(5-{[Methyl-(4,5,6,7-tetrahydro-benzthiazol-2-ylmethyl)-amino]-methyl}-furan-2-yl)- 5,6,7,8-tetrahydro-2H-isochinolin-1-on | 410 (M+1) | |
| 67 | 4-[5-(2-Pyridin-2-yl-pyrrolidin-1-ylmethyl)-furan-2-yl]-5,6,7,8-tetrahydro-2H- isochinolin-1-on | 376 (M+1) | |
| 68 | 4-(5-{[(1H-Benzimidazol-2-ylmethyl)-amino]-methyl}-furan-2-yl)-5,6,7,8-tetrahydro-2H-isochinolin-1-on | 375 (M+1) | |
| 69 | 4-(5-{[Benzyl-(1-methyl-1H-imidazol-2-ylmethyl)-amino]-methyl}-furan-2-yl)-5,6,7,8-tetrahydro-2H-isochinolin-1-on | 429 (M+1) | |
| 70 | 4-[5-(Indan-2-ylaminomethyl)-furan-2-yl]-5,6,7,8-tetrahydro-2H-isochinolin-1-one | 361 (M+1) | |
| 71 | 4-[5-({[4-(4-Fluoro-phenyl)-piperazin-1-ylmethyl]-amino}-methyl)-furan-2-yl]-5,6,7,8-tetrahydro-2H-isochinolin-1-on | 437 (M+1) | |
| 72 | 4-{5-[(2-Hydroxy-2-phenyl-ethylamino)-methyl]-furan-2-yl}-5,6,7,8-tetrahydro-2H- isochinolin-1-on | 365 (M+1) | 11.75 (1H, br s), 9.14 (2H, br s), 7.46 (1H, s), 7.41-7.31 (5H, m), 6.67 (1H, d, J=3.1 Hz), 6.52 (1H, d, J=3.1 Hz), 6.21 (1H, d, J= 3.4 Hz); 4.90 (1H, m), 4.30 (2H, br s), 3.11 (1H, m), 2.99 (1H, m), 2.56 (2H, m), 2.38 (2H, m), 1.65 (4H, m) |
| 73 | 4-(5-{[(4-Methyl-piperazin-1-ylmethyl)-amino]-methyl}-furan-2-yl)-5,6,7,8-tetrahydro-2H-isochinolin-1-on | 357 (M+1) | |
| 74 | 4-(5-{[(Morpholin-4-ylmethyl)-amino]-methyl}-furan-2-yl)-5,6,7,8-tetrahydro-2H-isochinolin-1-on | 344 (M+1) | |
| 75 | N-[4-({[5-(1-Oxo-1,2,5,6,7,8-hexahydro-isochinolin-4-yl)-furan-2-ylmethyl]-amino}- methyl)-phenyl]-acetamid | 392 (M+1) | |
| 76 | 4-(5-Thiazolidin-3-ylmethyl-furan-2-yl)-5,6,7,8-tetrahydro-2H-isochinolin-1-on | 317 (M+1) | |
| 77 | 4-(5-{[(1-Methyl-1H-pyrazol-4-ylmethyl)-amino]-methyl}-furan-2-yl)-5,6,7,8-tetrahydro- 2H-isochinolin-1-on | 339 (M+1) | (CDCl3) 7.68 (1H, s), 7.62 (1H, s), 7.16 (1H, s), 6.42 (1H, d, J=3.4 Hz), 6.27 (1H, d, J=3.4 Hz), 4.18 (2H, s), 4.09 (2H, s), 3.90 (3H, s), 2.55 (2H, m), 2.39 (2H, m), 1.68 (4H, m) |
| 78 | 4-(5-Butylaminomethyl-furan-2-yl)-5,6,7,8-tetrahydro-2H-isochinolin-1-on | 301 (M+1) | |
| 79 | 4-[5-(Benzylamino-methyl)-furan-2-yl]-5,6,7,8-tetrahydro-2H-isochinolin-1-on | 335 (M+1) | |

### Synthese der Beispiele 80 - 84 durch Acylierung von Beispiel 50 mit Carbonsäuren Allgemeine Arbeitsvorschrift

0,15 mmol 4-(5-Aminomethyl-furan-2-yl)-5,6,7,8-tetrahydro-2H-isochinolin-1-on werden zusammen mit 0,15 mmol Säurechlorid oder Säureanhydrid und 0,375 mmol Triethylamin in 5 mL DCM gelöst und bei 0°C 2 h gerührt. Der Reaktionsansatz wird mit Wasser versetzt, mit DCM extrahiert, die organische Phase eingeengt und per RP-HPLC gereinigt. Beispiel 84 wird als Trifluoracetat isoliert.
Entsprechend der allgemeinen Vorschrift werden folgende Verbindungen hergestellt:

| **Beispiel** | **Name** | **Masse (ES+) m/z =** | **NMR(D6-DMSO)** δ = |
|---|---|---|---|
| 80 | 2,2,2-Trifluor-N-[5-(1-oxo-1,2,5,6,7,8-hexahydro-isochinolin-4-yl)-furan-2-ylmethyl]- acetamid | 341 | 11.62 (1H, br s), 9.99 (1H, s), 7.34 (1H, s), 6.42 (1H, d, J=3.4 Hz), 6.37 (1H, d, J=3.4 Hz), 4.42 (2H, d, J=5.8 Hz), 2.55 (2H, m), 2.37 (2H, m), 1.65 (4H, m). |
| 81 | 3-Methyl-N-[5-(1-oxo-1,2,5,6,7,8-hexahydro-isochinolin-4-yl)-furan-2-ylmethyl]-butyramid | 329 | |
| 82 | Furan-2-carbonsäure [5-(1-oxo-1,2,5,6,7,8-hexahydro-isochinolin-4-yl)-furan-2-ylmethyl]-amid | 339 | |
| 83 | 4-Methoxy-N-[5-(1-oxo-1,2,5,6,7,8-hexahydro-isochinolin-4-yl)-furan-2-ylmethyl]- benzamid | 379 | 11,6 (1H, br s); 8,83 (1H, t, J=5.5 Hz); 7,86 (2H, d, J=8.9 Hz); 7,33 (1H, s); 7,00 (2H, d, J=8.9 Hz); 6,39 (1H, d, J= 3.1 Hz); 6,30 (1H, d, J=3.1 Hz); 4,46 (2H, d, J=5.5 Hz); 3,80 (3H, s); 2,55 (2H, m); 2,37 (2H, m); 1,64 (4H, m). |
| 84 | N-[5-(1-Oxo-1,2,5,6,7,8-hexahydro-isochinolin-4-yl)-furan-2-ylmethyl]-nicotinamid | 350 | |

### Synthese der Beispiele 85 - 87 durch Umsetzung von Beispiel 50 mit Sulfonylchloriden Allgemeine Arbeitsvorschrift

0,15 mmol 4-(5-Aminomethyl-furan-2-yl)-5,6,7,8-tetrahydro-2H-isochinolin-1-on werden zusammen mit 0,18 mmol Sulfonylchlorid und 0,375 mmol Kaliumcarbonat in 5 mL DMF gelöst und bei RT 18 h gerührt. Der Reaktionsansatz wird mit Wasser versetzt, mit DCM extrahiert, die organische Phase eingeengt und per RP-HPLC gereinigt. Beispiel 85 wird als Trifluoracetat isoliert.

| **Beispiel** | **Name** | **Masse (ES+) m/z =** | **NMR (D6-DMSO)** δ= |
|---|---|---|---|
| 85 | N-[5-(1-Oxo-1,2,5,6,7,8-hexahydro-isochinolin-4-yl)-furan-2-ylmethyl]-4-(pyridin-2-yloxy)-benzensulfonamid | 592 | |
| 86 | 3-Methoxy-N-[5-(1 -oxo-1,2,5,6,7,8-hexahydro-isochinolin-4-yl)-furan-2-ylmethyl]- benzensulfonamid | 415 | |
| 87 | N-(4-{[5-(1-Oxo-1,2,5,6,7,8-hexahydro-isochinolin-4-yl)-furan-2-ylmethyl]-sulfamoyl}-phenyl)-acetamid | 442 | 11,6 (1H, br s); 10,22 (1H, s); 8,06 (1H, t, J=5.8 Hz); 7,65 (4H, m); 7,24 (1H, s); 6,26 (1H, d, J= 3.1 Hz); 6,29 (1H, d, J=3.1 Hz); 4,02 (2H, d, J=5.8 Hz); 2,45 (2H, m); 2,36 (2H, m); 2,06 (3H, s); 1,63 (4H, m). |

### Pharmakologische Untersuchungen

### PARP Enzymassay

Zur Bestimmung der halbmaximalen Inhibitorkonzentration werden die zu testenden Substanzen mit dem durch DNA aktivierten, rekombinant exprimierten und gereinigten PARP-1 Enzym inkubiert. Im Einzelnen werden verschiedene Konzentrationen der Testsubstanz in 50µl Reaktionslösung, die 50mM Tris, 5mM MgCl₂, 1 mM DTT, 200µM NAD, 0,1mCi/ml Tritium-markiertes NAD, 0,1 mg/ml DNA, 0,1mg/ml Histone, 2µg/ml rekombinant exprimiertes humanes PARP-1 Enzym, pH=8,0 enthält, für 1 Stunde bei Raumtemperatur inkubiert. Die Reaktion wird durch Zugabe von 150µl 20%ige Trichloressigsäure gestoppt und die radioaktiv markierten Proteinbestandteile gefällt. Nach einer 10-minütigen Inkubation auf Eis werden die markierten, unlöslichen Bestandteile durch einen Glasfaserfilter abgetrennt und nach dreimaligem Waschen mit 20%iger Trichloressigsäure die durch das PARP-1 Enzym eingebaute Radioaktivität durch Radiolumineszenz gemessen. Betrachtet man die so bestimmten Einbauraten in Abhängigkeit von der Konzentration der Testsubstanz, erhält man die halbmaximale Inhibitorkonzentration (IC₅₀) als die Konzentration der Testsubstanz, die die Einbaurate auf die Hälfte des maximal erreichbaren Wertes (Inkubation ohne Inhibitor) reduziert.

Auf diese Weise wurden für die nachfolgenden Verbindungen IC-50 Werte bestimmt:

| **Bsp.** | **IC-50 [µM]** | **Bsp.** | **IC-50 [µM]** | **Bsp.** | **IC-50 [µM]** |
|---|---|---|---|---|---|
| 1 | 1.31 | 2 | 0.27 | 3 | 9.83 |
| 4 | 6.35 | 5 | 0.39 | 6 | 0.80 |
| 7 | 0.30 | 8 | 0.61 | 9 | 0.28 |
| 10 | 1.43 | 11 | 0.39 | 12 | 0.67 |
| 13 | 0.08 | 14 | 1.21 | 15 | 0.69 |
| 16 | 0.09 | 17 | 0.14 | 18 | 0.38 |
| 19 | 0.74 | 20 | 1.28 | 21 | 10.58 |
| 22 | 0.33 | 23 | 2.01 | 24 | 0.22 |
| 25 | 7.70 | 26 | 1.47 | 27 | 2.54 |
| 28 | 0.40 | 29 | 0.56 | 30 | 3.35 |
| 31 | 4.80 | 32 | 0.77 | 33 | 3.69 |
| 34 | 3.48 | 35 | 6.88 | 36 | 0.42 |
| 37 | 4.07 | 38 | 3.19 | 39 | 5.89 |
| 40 | 4.69 | 41 | 3.85 | 42 | 1.03 |
| 43 | 0.82 | 44 | 8.27 | 45 | 1.70 |
| 46 | 3.13 | 47 | 3.29 | 48 | 3.07 |
| 49 | 5.11 | 51 | 0.59 | 52 | 0.33 |
| 53 | 0.24 | 54 | 0.30 | 55 | 4.54 |
| 56 | 1.45 | 57 | 0.42 | 58 | 1.27 |
| 59 | 0.99 | 60 | 0.56 | 61 | 0.11 |
| 62 | 1.53 | 63 | 0.24 | 64 | 0.34 |
| 65 | 0.46 | 66 | 1.18 | 67 | 0.72 |
| 68 | 0.34 | 69 | 2.76 | 70 | 0.31 |
| 71 | 1.24 | 72 | 0.11 | 73 | 0.66 |
| 74 | 0.74 | 75 | 0.51 | 76 | 0.44 |
| 77 | 0.18 | 78 | 0.25 | 79 | 0.15 |
| 80 | 0.54 | 81 | 1.07 | 82 | 0.46 |
| 83 | 1.25 | 84 | 0.79 | 85 | 1.63 |
| 86 | 2.48 | 87 | 3.77 | | |

### ATP-Verbrauchsassay in Kardiomyoblasten

Die Wirksamkeit der Testsubstanzen in Zellen wird mittels eines ATP-Verbrauchsassays ermittelt. Hierzu werden Ratten Kardiomyoblasten (Zelllinie H9c2) in einer 96-Lochplatte (40.000 Zellen pro Loch; RPMI1640; 10%FCS) ausgesät und für 16 Stunden bei 37°C und 5% CO₂ gehalten. Die Zellen werden mit PBS gewaschen und für 15 min unter gleichen Bedingungen mit verschiedenen Konzentrationen der Testsubstanz in Medium inkubiert. Nach der Zugabe von 300µM H₂O₂ werden die Zellen eine weitere Stunde bei 37°C, 5% CO₂ gehalten, lysiert und der zelluläre Gehalt an ATP mittels einer Luziferasereaktion bestimmt. Die halbmaximale effektive Konzentration der Substanzen (EC50) wird als die Konzentration bestimmt, bei der der ATP-Gehalt der Zellen die Hälfte des Wertes erreicht hat, der bei einer maximalwirksamen Konzentration derselben Substanz gemessen werden kann.

### Enzyminhibitionsassay mit variablen Substratkonzentrationen

Apparente Kᵢ Werte der Testsubstanzen werden in einem enzymatischen Test unter Verwendung des gereinigten humanen PARP-1 Enzyms bestimmt. Im Einzelnen werden verschiedene Konzentrationen des Tritium-markierten Substrats NAD mit einer gleichen Konzentration der Testsubstanz in 50µl Reaktionslösung, die 50mM Tris, 5mM MgCl2, 1 mM DTT, 0,1 mg/ml DNA, 0,1mg/ml Histone, 2µg/ml rekombinant exprimiertes humanes PARP-1 Enzym, pH=8,0 enthält, für 10 min bei , Raumtemperatur inkubiert. Die Reaktion wird durch Zugabe von 150µl 20%ige Trichloressigsäure gestoppt und die radioaktiv markierten Proteinbestandteile gefällt. Nach einer 10-minütigen Inkubation auf Eis werden die markierten, unlöslichen Bestandteile durch einen Glasfaserfilter abgetrennt und nach dreimaligem Waschen mit 20%iger Trichloressigsäure die durch das PARP-1 Enzym eingebaute Radioaktivität durch Radiolumineszenz gemessen. Eine Auswertung der so bestimmten Einbauraten in Abhängigkeit von der Konzentration des Substrats NAD liefert die nach einer Michaelis-Menten-Kinetik apparenten Kᵢ-Werte, einen rein kompetitiven Mechanismus der Inhibition vorausgesetzt.

Auf diese Weise werden für die nachfolgenden ausgewählten Verbindungen EC-50 Werte und apparente Kᵢ Werte bestimmt:

| **Bsp.** | **EC-50 [µM]** | **app. Kᵢ [nM]** | **Bsp.** | **EC-50 [µM]** | **app. Kᵢ [nM]** |
|---|---|---|---|---|---|
| 9 | 1.57 | 16 | 54 | 0.33 | 15 |
| 16 | 0.2 | 54 | 72 | 0.38 | 24 |
| 24 | -- | 31 | 78 | 0.52 | 40 |

## Patentansprüche

1. Verbindung der allgemeinen Formel (I) 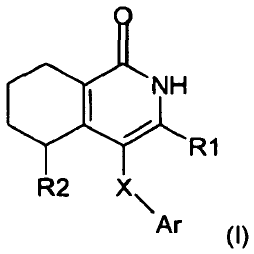 worin bedeuten:
X ist eine Einfachbindung, O, S, NH oder N(C₁-C₃-Alkyl);
R1 ist Wasserstoff, Fluor, Chlor, -CN, Methoxy, -OCF₃ oder C₁-C₃-Alkyl, das gegebenenfalls mit Hydroxy, Chlor, Methoxy oder einem, zwei oder drei Fluoratomen substituiert ist;
R2 ist Wasserstoff, Fluor, -CN, Hydroxy, Methoxy, -OCF₃, -NH₂, -NH(C₁-C₃-Alkyl), -N(C₁-C₃-Alkyl)₂ oder C₁-C₃-Alkyl, das gegebenenfalls mit Hydroxy, Chlor, Methoxy oder einem, zwei oder drei Fluoratomen substituiert ist;
R3 ist -(C₁-C₃-Alkyl)-NR4R5, -SO₂NR4R5, -C(O)NR4R5, -C(H)=N-OR9, -C(O)R6, - NHC(O)R6, -(C₁-C₃-Alkyl)-NHC(O)R6, -NHSO₂R6, -(C₁-C₃-Alkyl)-NHSO₂R6 oder -CH(OH)R7;
R4 und R5 sind unabhängig voneinander ausgewählt aus der Gruppe bestehend aus: Wasserstoff; unsubstituiertes oder zumindest monosubstitutiertes C₁-C₁₀-Alkyl, C₂-C₆-Alkenyl, C₂-C₆-Alkinyl, Aryl, Heteroaryl und Heterocyclyl, wobei die Substituenten ausgewählt sind aus der Gruppe bestehend aus: Aryl, Heteroaryl, Heterocyclyl, -O-Aryl, Fluor, Chlor, Brom, -CF₃, -OCF₃, -NO₂, -CN, -C(O)R8, -NHC(O)(C₁-C₃-Alkyl), -NH₂, Hydroxy, C₁-C₆-Alkyl, C₁-C₃-Alkoxy, -NH(C₁-C₃-Alkyl), -N(C₁-C₃-Alkyl)₂, -NH-Aryl, -NH-Heteroaryl, -NH-C(O)-Heteroaryl, -SO₂NH₂, -SO₂(C₁-C₃-Alkyl) und -NH-SO₂(C₁-C₃-Alkyl),
und die Aryl-, Heteroaryl- und Heterocyclyl-Fragmente dieser Substituenten können wiederum mit Fluor, Chlor, Brom, Oxo, -CF₃, -OCF₃, -NO₂, -CN, Aryl, Heteroaryl, -NHC(O)(C₁-C₃-Alkyl), -COOH, Hydroxy, C₁-C₃-Alkyl, C₁-C₃-Alkoxy, -SO₂NH₂, -SO₂NH(C₁-C₃-Alkyl), -SO₂N(C₁-C₃-Alkyl)₂, -C(O)NH₂, -C(O)NH(C₁-C₃-Alkyl), -C(O)N(C₁-C₃-Alkyl)₂, -SO₂(C₁-C₃-Alkyl), -NH₂, -NH(C₁-C₃-Alkyl) oder -N(C₁-C₃-Alkyl)₂ zumindest monosubstituiert sein; oder
R4 und R5 bilden zusammen mit dem Stickstoffatom, an das sie gebunden sind, unsubstitutiertes oder zumindest monosubstitutiertes Heterocyclyl,
wobei die Substituenten ausgewählt sind aus der Gruppe bestehend aus: Aryl, Heteroaryl, Heterocyclyl, Oxo, Fluor, Chlor, Brom, -CF₃, -OCF₃, -NO₂, -CN, - C(O)R8, -NHC(O)(C₁-C₃-Alkyl), -NH₂, Hydroxy, C₁-C₃-Alkyl, C₁-C₃-Alkoxy, - NH(C₁-C₃-Alkyl), -N(C₁-C₃-Alkyl)₂, -SO₂NH₂, -SO₂(C₁-C₃-Alkyl) und -NH-SO₂(C₁-C₃-Alkyl),
und von diesen Substituenten können Aryl, Heterocyclyl und Heteroaryl wiederum mit Fluor, Chlor, Brom, Hydroxy, C₁-C₃-Alkyl oder C₁-C₃-Alkoxy zumindest monosubstituiert sein;
R6 ist unsubstituiertes oder zumindest monosubstituiertes C₁-C₆-Alkyl, Phenyl, Heteroaryl oder Heterocyclyl,
wobei die Substituenten ausgewählt sind aus der Gruppe bestehend aus: Fluor, Chlor, Brom, Aryl, Heterocyclyl, Heteroaryl, -CF₃, -OCF₃, -NO₂, -CN, - C(O)R8, -NHC(O)(C₁-C₃-Alkyl), -NH₂, Hydroxy, C₁-C₃-Alkyl, C₁-C₃-Alkoxy, -O-Heteroaryl, -O-Aryl, -NH(C₁-C₃-Alkyl), -N(C₁-C₃-Alkyl)₂, -SO₂NH₂, -SO₂(C₁-C₃-Alkyl) und -NH-SO₂(C₁-C₃-Alkyl), und die Aryl-, Heterocyclyl- und Heteroaryl-Fragmente dieser Substituenten können wiederum mit Fluor, Chlor, Brom, Hydroxy, C₁-C₃-Alkyl oder C₁-C₃-Alkoxy zumindest monosubstituiert sein;
R7 ist ausgewählt aus der Gruppe bestehend aus: Wasserstoff; unsubstituiertes oder zumindest monosubstituiertes C₁-C₆-Alkyl, Phenyl und Heteroaryl,
wobei die Substituenten ausgewählt sind aus der Gruppe bestehend aus: Fluor, Chlor, Brom, -CF₃, -OCF₃, -NO₂, -CN, -C(O)R8, -NHC(O)(C₁-C₃-Alkyl), - NH₂, Hydroxy, C₁-C₃-Alkyl, C₁-C₃-Alkoxy, -NH(C₁-C₃-Alkyl), -N(C₁-C₃-Alkyl)₂, - SO₂NH₂, -SO₂(C₁-C₃-Alkyl) und -NH-SO₂(C₁-C₃-Alkyl);
R8 ist C₁-C₃-Alkoxy, -O-Phenyl, C₁-C₃-Alkyl, -NH₂, -NH(C₁-C₃-Alkyl), -N(C₁-C₃-Alkyl)₂ oder Phenyl,
und die vorstehenden Phenyl-Fragmente können wiederum mit Fluor, Chlor, Brom, Oxo, -CF₃, -OCF₃, -NO₂, -CN, Aryl, Heteroaryl, -NHC(O)(C₁-C₃-Alkyl), -COOH, Hydroxy, C₁-C₃-Alkyl, C₁-C₃-Alkoxy, -SO₂NH₂, -SO₂NH(C₁-C₃-Alkyl), -SO₂N(C₁-C₃-Alkyl)₂, -C(O)NH₂, -C(O)NH(C₁-C₃-Alkyl), -C(O)N(C₁-C₃-Alkyl)₂, -SO₂(C₁-C₃-Alkyl), -NH₂, -NH(C₁-C₃-Alkyl) oder -N(C₁-C₃-Alkyl)₂ zumindest monosubstituiert sein;
R9 ist ausgewählt aus der Gruppe bestehend aus: Wasserstoff; unsubstituiertes oder zumindest monosubstituiertes C₁-C₆-Alkyl und Phenyl,
wobei die Substituenten ausgewählt sind aus der Gruppe bestehend aus: Fluor, Chlor, Brom, Aryl, Heterocyclyl, Heteroaryl, -CF₃, -OCF₃, -NO₂, -CN, - C(O)R8, -NHC(O)(C₁-C₃-Alkyl), C₁-C₃-Alkyl, C₁-C₃-Alkoxy, -NH(C₁-C₃-Alkyl),-N(C₁-C₃-Alkyl)₂, -SO₂NH₂, -SO₂(C₁-C₃-Alkyl) und -NH-SO₂(C₁-C₃-Alkyl), und von diesen Substituenten können Aryl, Heterocyclyl und Heteroaryl wiederum mit Fluor, Chlor, Brom, C₁-C₃-Alkyl oder C₁-C₃-Alkoxy zumindest monosubstituiert sein;
Ar ist unsubstituiertes oder zumindest monosubstituiertes Aryl oder Heteroaryl, wobei die Substituenten ausgewählt sind aus der Gruppe bestehend aus: Fluor, Chlor, Brom, -CF₃, -OCF₃, -NO₂, -CN, -C(O)R8, -NH₂, -NHC(O)(C₁-C₆-Alkyl), Hydroxy, C₁-C₆-Alkyl, C₁-C₆-Alkoxy, -CH₂-CH₂-CH₂-, -CH₂-O-C(O)-, -CH₂-C(O)-O-, -CH₂-NH-C(O)-, -CH₂-N(CH₃)-C(O)-, -CH₂-C(O)-NH-, -NH(C₁-C₆-Alkyl), - N(C₁-C₆-Alkyl)₂, -SO₂(C₁-C₆-Alkyl), Heterocyclyl, Heteroaryl, Aryl und R3,
und von diesen Substituenten können Heterocyclyl, Aryl und Heteroaryl wiederum mit C₁-C₆-Alkyl, C₁-C₆-Alkoxy, Fluor, Chlor, Brom, Trifluormethyl, Trifluormethoxy oder OH zumindest monosubstituiert sein;
Heteroaryl ist ein 5 bis 10-gliedriger, aromatischer, mono- oder bicyclischer Heterocyclus, der ein oder mehrere Heteroatome ausgewählt aus N, O und S enthält;
Aryl ist ein 5 bis 10-gliedriger, aromatischer, Mono- oder Bicyclus.
Heterocyclyl ist ein 5 bis 10-gliedriger, nicht-aromatischer, mono- oder bicyclischer Heterocyclus, der ein oder mehrere Heteroatome ausgewählt aus N, O und S enthält;
oder ein physiologisch verträgliches Salz davon;
unter der Voraussetzung, dass Ar nicht unsubstituiertes Phenyl ist, wenn X eine Einfachbindung ist und der weiteren Voraussetzung, dass die Verbindung 3-(3methyl-1-oxo-1,2,5,6,7,8-hexahydroisoquinolin-4-yl)benzonitril ausgenommen ist.

2. Verbindung der allgemeinen Formel (I) nach Anspruch 1, worin R1, R2, R3 und X die in Anspruch 1 aufgeführten Bedeutungen haben und
Ar ist unsubstituiertes oder zumindest monosubstituiertes Phenyl oder Heteroaryl, wobei die Substituenten ausgewählt sind aus der Gruppe bestehend aus: Fluor, Chlor, -CF₃, -OCF₃, -C(O)(C₁-C₃-Alkyl), -NH₂, -NHC(O)(C₁-C₃-Alkyl), Hydroxy, C₁-C₃-Alkyl, C₁-C₃-Alkoxy, -CH₂-CH₂-CH₂-, -CH₂-O-C(O)-, -CH₂-C(O)-O-, -CH₂-NH-C(O)-, -CH₂-N(CH₃)-C(O)-, -CH₂-C(O)-NH-, -NH(C₁-C₃-Alkyl), -N(C₁-C₃-Alkyl)₂, -SO₂(C₁-C₃-Alkyl), Heterocyclyl, Heteroaryl und R3,
und von diesen Substituenten können Heterocyclyl und Heteroaryl wiederum mit C₁-C₃-Alkyl, C₁-C₃-Alkoxy, Fluor, Chlor, Brom, Trifluormethyl, Trifluormethoxy oder OH zumindest monosubstituiert sein;
oder ein physiologisch verträgliches Salz davon;
unter der Voraussetzung, dass Ar nicht unsubstituiertes Phenyl ist, wenn X eine Einfachbindung ist.

3. Verbindung nach Anspruch 1 oder 2, worin in der allgemeinen Formel (I) bedeuten:
X ist eine Einfachbindung, NH oder N(C₁-C₃-Alkyl);
R1 ist Wasserstoff oder C₁-C₃-Alkyl, das gegebenenfalls mit Hydroxy, Chlor, Methoxy oder einem, zwei oder drei Fluoratomen substituiert ist;
R2 ist Wasserstoff, Fluor, -OCF₃, Hydroxy, Methoxy, -NH₂ oder C₁-C₃-Alkyl;
R3 ist -CH₂-NR4R5, -SO₂NR4R5, -C(O)NR4R5, -CH₂-NHC(O)R6, -CH₂-NHSO₂R6 oder -CH(OH)R7;
R4 und R5 sind unabhängig voneinander ausgewählt aus der Gruppe bestehend aus: Wasserstoff; unsubstituiertes oder zumindest monosubstituiertes C₁-C₁₀-Alkyl, C₂-C₆-Alkenyl, Phenyl, Indanyl, Heterocyclyl und Heteroaryl,
wobei die Substituenten ausgewählt sind aus der Gruppe bestehend aus: Phenyl, Heteroaryl, Heterocyclyl, -O-Phenyl, Fluor, -CN, -C(O)NH₂, - C(O)(C₁-C₃-Alkyl), -C(O)-Phenyl, -N(C₁-C₃-Alkyl)₂, -NH(C₁-C₃-Alkyl), -NH₂, -NH-Heteroaryl, -NH-C(O)-Heteroaryl, C₁-C₆-Alkyl, C₁-C₃-Alkoxy und Hydroxy,
und die Phenyl-, Heterocyclyl- und Heteroaryl-Fragmente dieser Substituenten können wiederum mit Fluor, Chlor, Brom, Oxo, -CF₃, -OCF₃, -NO₂, -CN, Phenyl, Pyridinyl, -NHC(O)(C₁-C₃-Alkyl), -COOH, Hydroxy, C₁-C₃-Alkyl, C₁-C₃-Alkoxy, -SO₂NH₂, -SO₂NH(C₁-C₃-Alkyl), -SO₂N(C₁-C₃-Alkyl)₂, -C(O)NH₂, -C(O)NH(C₁-C₃-Alkyl), -C(O)N(C₁-C₃-Alkyl)₂, -SO₂(C₁-C₃-Alkyl), -NH₂, -NH(C₁-C₃-Alkyl) oder -N(C₁-C₃-Alkyl)₂, zumindest monosubstituiert sein; oder
R4 und R5 bilden zusammen mit dem Stickstoffatom, an das sie gebunden sind, unsubstituiertes oder zumindest monosubstituiertes Heterocyclyl,
wobei die Substituenten ausgewählt sind aus der Gruppe bestehend aus: Phenyl, Heteroaryl, Heterocyclyl, Oxo, Fluor, Chlor, -C(O)(C₁-C₃-Alkyl),-C(O)-Phenyl und Hydroxy,
und die Phenyl-, Heterocyclyl- und Heteroaryl-Fragmente dieser Substituenten können wiederum mit Fluor oder C₁-C₃-Alkyl zumindest monosubstituiert sein;
R6 ist unsubstituiertes oder zumindest monosubstituiertes C₁-C₆-Alkyl, Phenyl oder Heteroaryl,
wobei die Substituenten ausgewählt sind aus der Gruppe bestehend aus: Fluor, Chlor, Brom, -CF₃, -OCF₃, -NHC(O)(C₁-C₃-Alkyl), Hydroxy, C₁-C₃-Alkyl, C₁-C₃-Alkoxy, -O-Heteroaryl, Phenyl, -NH₂, -NH(C₁-C₃-Alkyl), -N(C₁-C₃-Alkyl)₂ und Heterocyclyl,
und die Phenyl-, Heteroaryl- und Heterocyclyl-Fragmente dieser Substituenten können wiederum mit Fluor, Chlor, Brom, Hydroxy, C₁-C₃-Alkyl oder C₁-C₃-Alkoxy zumindest monosubstituiert sein;
R7 ist ausgewählt aus der Gruppe bestehend aus: Wasserstoff; unsubstituiertes oder zumindest monosubstituiertes C₁-C₆-Alkyl, Phenyl und Pyridinyl,
wobei die Substituenten ausgewählt sind aus der Gruppe bestehend aus: Fluor, Chlor, Brom, Hydroxy, C₁-C₃-Alkyl und C₁-C₃-Alkoxy;
Ar ist unsubstituiertes oder zumindest monosubstituiertes Phenyl oder Heteroaryl,
wobei die Substituenten ausgewählt sind aus der Gruppe bestehend aus: Fluor, Chlor, -CF₃, -OCF₃, -C(O)(C₁-C₃-Alkyl), -NH₂, -NHC(O)(C₁-C₃-Alkyl), Hydroxy, C₁-C₃-Alkyl, C₁-C₃-Alkoxy, -CH₂-CH₂-CH₂-, -CH₂-O-C(O)-, -CH₂-C(O)-O-, -CH₂-NH-C(O)-, -CH₂-N(CH₃)-C(O)-, -CH₂-C(O)-NH-, -NH(C₁-C₃-Alkyl), -N(C₁-C₃-Alkyl)₂, -SO₂(C₁-C₃-Alkyl), Heterocyclyl, Heteroaryl und R3,
und von diesen Substituenten können Heterocyclyl und Heteroaryl wiederum mit C₁-C₃-Alkyl, C₁-C₃-Alkoxy, Fluor, Chlor, Brom, Trifluormethyl, Trifluormethoxy oder OH zumindest monosubstituiert sein;
Heteroaryl ist Pyridinyl, Thienyl, Pyrimidinyl, Imidazolyl, Furanyl, Indolyl, Benzimidazolyl, Pyrazolyl, 1,3-Benzodioxolyl, Triazolyl, Thiazolyl, Isoxazolyl, Pyrrolyl, Pyrazinyl, Oxazolyl, Pyridazinyl, Chinolinyl, Isochinolyl, Benzofuranyl, 3-Oxo-1,3-dihydroisobenzofuranyl oder 4,5,6,7-Tetrahydrobenzothiazolyl;
Heterocyclyl ist Morpholinyl, Pyrrolidinyl, Piperidinyl, Tetrahydropyranyl, Thiazolidinyl, Dihydroisoxazolyl, Piperazinyl oder Tetrahydrofuranyl;
oder ein physiologisch verträgliches Salz davon;
unter der Voraussetzung, dass Ar nicht unsubstituiertes Phenyl ist, wenn X eine Einfachbindung ist.

4. Verbindung nach Anspruch 1 bis 3, worin in der allgemeinen Formel (I) bedeuten:
X ist eine Einfachbindung, NH oder N(C₁-C₃-Alkyl);
R1 ist Wasserstoff oder C₁-C₃-Alkyl, das gegebenenfalls mit Hydroxy, Chlor, Methoxy oder einem, zwei oder drei Fluoratomen substituiert ist;
R2 ist Wasserstoff, Fluor, -OCF₃, Hydroxy, Methoxy, -NH₂ oder C₁-C₃-Alkyl-,
R3 ist -CH₂-NR4R5, -SO₂-NR4R5, -C(O)NR4R5, -CH₂-NHC(O)R6, - CH₂NHSO₂R6 oder -CH(OH)R7;
R4 und R5 sind unabhängig voneinander ausgewählt aus der Gruppe bestehend aus: Wasserstoff; unsubstituiertes oder zumindest monosubstituiertes C₁-C₁₀-Alkyl, C₂-C₆-Alkenyl, Phenyl, Indanyl, Heterocyclyl und Heteroaryl,
wobei die Substituenten ausgewählt sind aus der Gruppe bestehend aus: Phenyl, Heteroaryl, Heterocyclyl, -O-Phenyl, Fluor, -CN, -C(O)NH₂, - C(O)(C₁-C₃-Alkyl), -C(O)-Phenyl, -N(C₁-C₃-Alkyl)₂, -NH(C₁-C₃-Alkyl), -NH₂, -NH-Heteroaryl, -NH-C(O)-Heteroaryl, C₁-C₆-Alkyl, C₁-C₃-Alkoxy und Hydroxy,
und die Phenyl-, Heterocyclyl- und Heteroaryl-Fragmente dieser Substituenten können wiederum mit Fluor, Chlor, Brom, Oxo, -CF₃, -OCF₃, -NO₂, -CN, Phenyl, Pyridinyl, -NHC(O)(C₁-C₃-Alkyl), -COOH, Hydroxy, C₁-C₃-Alkyl, C₁-C₃-Alkoxy, -SO₂NH₂, -SO₂NH(C₁-C₃-Alkyl), -SO₂N(C₁-C₃-Alkyl)₂, -C(O)NH₂, -C(O)NH(C₁-C₃-Alkyl), -C(O)N(C₁-C₃-Alkyl)₂, -SO₂(C₁-C₃-Alkyl), -NH₂, -NH(C₁-C₃-Alkyl) oder -N(C₁-C₃-Alkyl)₂, zumindest monosubstituiert sein; oder
R4 und R5 bilden zusammen mit dem Stickstoffatom, an das sie gebunden sind, unsubstituiertes oder zumindest monosubstituiertes Heterocyclyl,
wobei die Substituenten ausgewählt sind aus der Gruppe bestehend aus: Phenyl, Heteroaryl, Heterocyclyl, Oxo, Fluor, Chlor, -C(O)(C₁-C₃-Alkyl), - C(O)-Phenyl und Hydroxy,
und die Phenyl, Heterocyclyl- und Heteroaryl-Fragmente dieser Substituenten können wiederum mit Fluor oder C₁-C₃-Alkyl zumindest monosubstituiert sein;
R6 ist CF₃ oder unsubstituiertes oder zumindest monosubstituiertes C₁-C₆-Alkyl, Pyridinyl, Furanyl oder Phenyl,
wobei die Substituenten ausgewählt sind aus der Gruppe bestehend aus: Fluor, -NHC(O)(C₁-C₃-Alkyl), Hydroxy, C₁-C₃-Alkyl, C₁-C₃-Alkoxy und -O-Pyridinyl;
R7 ist ausgewählt aus der Gruppe bestehend aus: Wasserstoff; unsubstituiertes oder zumindest monosubstituiertes C₁-C₆-Alkyl, Phenyl und Pyridinyl,
wobei die Substituenten ausgewählt sind aus der Gruppe bestehend aus: Fluor, Chlor, Brom, Hydroxy, C₁-C₃-Alkyl und C₁-C₃-Alkoxy;
Ar ist unsubstituiertes oder zumindest monosubstituiertes Phenyl, Thienyl, Furanyl oder Pyridinyl,
wobei die Substituenten ausgewählt sind aus der Gruppe bestehend aus: Fluor, Chlor, -CF₃, -OCF₃, -C(O)(C₁-C₃-Alkyl), -NH₂, -NHC(O)(C₁-C₃-Alkyl), Hydroxy, C₁-C₃-Alkyl, C₁-C₃-Alkoxy, -CH₂-CH₂-CH₂-, -CH₂-O-C(O)-, -CH₂-C(O)-O-, -CH₂-NH-C(O)-, -CH₂-N(CH₃)-C(O)-, -CH₂-C(O)-NH-, -NH(C₁-C₃-Alkyl), -N(C₁-C₃-Alkyl)₂, -SO₂(C₁-C₃-Alkyl) und R3;
Heteroaryl ist Pyridinyl, Thienyl, Pyrimidinyl, Imidazolyl, Furanyl, Indolyl, Benzimidazolyl, Pyrazolyl, 1,3-Benzodioxolyl, Triazolyl, Thiazolyl, Isoxazolyl, Pyrrolyl, Pyrazinyl, Oxazolyl, Pyridazinyl, Chinolinyl, Isochinolyl, Benzofuranyl, 3-Oxo-1,3-dihydroisobenzofuranyl oder 4,5,6,7-Tetrahydrobenzothiazolyl;
Heterocyclyl ist Morpholinyl, Pyrrolidinyl, Piperidinyl, Tetrahydropyranyl, Thiazolidinyl, Dihydroisoxazolyl, Piperazinyl oder Tetrahydrofuranyl;
oder ein physiologisch verträgliches Salz davon;
unter der Voraussetzung, dass Ar nicht unsubstituiertes Phenyl ist, wenn X eine Einfachbindung ist.

5. Verbindung nach einem der Ansprüche 1 bis 4, worin in der allgemeinen Formel (I) bedeuten:
X ist eine Einfachbindung, NH oder N(C₁-C₃-Alkyl);
R1 ist Wasserstoff oder C₁-C₃-Alkyl;
R2 ist Wasserstoff;
R3 ist -CH₂-NR4R5, -CH₂-NHC(O)R6, -CH₂-NHSO₂R6, -C(O)NR4R5 oder - CH(OH)R7;
R4 ist ausgewählt aus der Gruppe bestehend aus: Wasserstoff, unsubstituiertes oder zumindest monosubstituiertes C₁-C₁₀-Alkyl, C₁-C₆-Alkenyl, Phenyl, Indanyl, Heterocyclyl und Heteroaryl,
wobei die Substituenten ausgewählt sind aus der Gruppe bestehend aus: Phenyl, Heteroaryl, Heterocyclyl, -O-Phenyl, Fluor, -CN, -C(O)NH₂, - C(O)(C₁-C₃-Alkyl), -C(O)-Phenyl, -N(C₁-C₃-Alkyl)₂, -NH(C₁-C₃-Alkyl), -NH₂, -NH-Heteroaryl, -NH-C(O)-Heteroaryl, C₁-C₆-Alkyl, C₁-C₃-Alkoxy und Hydroxy,
und die Phenyl-, Heterocyclyl- und Heteroaryl-Fragmente dieser Substituenten können wiederum mit Fluor, Chlor, Brom, Oxo, -CF₃, -OCF₃, -NO₂, -CN, Phenyl, Pyridinyl, -NHC(O)(C₁-C₃-Alkyl), -COOH, Hydroxy, C₁-C₃-Alkyl, C₁-C₃-Alkoxy, -SO₂NH₂, -SO₂NH(C₁-C₃-Alkyl), -SO₂N(C₁-C₃-Alkyl)₂, -C(O)NH₂, -C(O)NH(C₁-C₃-Alkyl), -C(O)N(C₁-C₃-Alkyl)₂, -SO₂(C₁-C₃-Alkyl), -NH₂, -NH(C₁-C₃-Alkyl) oder-N(C₁-C₃-Alkyl)₂ zumindest monosbstuiert sein; und R5 ist Wasserstoff; oder
R4 und R5 bilden zusammen mit dem Stickstoffatom, an das sie gebunden sind. unsubstituiertes oder zumindest monosubstituiertes Heterocyclyl,
wobei die Substituenten ausgewählt sind aus der Gruppe bestehend aus: Phenyl, Heteroaryl, Heterocyclyl, Oxo, Fluor, Chlor, -C(O)(C₁-C₃-Alkyl), - C(O)-Phenyl und Hydroxy,
und die Phenyl-, Heterocyclyl- und Heteroaryl-Fragmente dieser Substituenten können wiederum mit Fluor oder C₁-C₃-Alkyl zumindest monosubstituiert sein;
R6 ist CF₃ oder unsubstituiertes oder zumindest monosubstituiertes C₁-C₆-Alkyl, Pyridinyl, Furanyl oder Phenyl,
wobei die Substituenten ausgewählt sind aus der Gruppe bestehend aus: Fluor, -NHC(O)(C₁-C₃-Alkyl), Hydroxy, C₁-C₃-Alkyl, C₁-C₃-Alkoxy und -O-Pyridinyl;
R7 ist ausgewählt aus der Gruppe bestehend aus: Wasserstoff; unsubstituiertes oder zumindest monosubstituiertes C₁-C₆-Alkyl, Phenyl und Pyridinyl,
wobei die Substituenten ausgewählt sind aus der Gruppe bestehend aus: Fluor, Chlor, Brom, Hydroxy, C₁-C₃-Alkyl und C₁-C₃-Alkoxy;
Ar ist monosubstituiertes Phenyl, Thienyl, Furanyl oder Pyridinyl, wobei der Substituent ausgewählt ist aus der Gruppe bestehend aus: Fluor, Chlor, -CF₃, -OCF₃, -C(O)(C₁-C₃-Alkyl), -NH₂, -NHC(O)(C₁-C₃-Alkyl), Hydroxy, C₁-C₃-Alkyl, C₁-C₃-Alkoxy, -NH(C₁-C₃-Alkyl), -N(C₁-C₃-Alkyl)₂, - SI₂(C₁-C₃-Alkyl) und R3; und der Substituent und X in meta-Position zueinander stehen;
Heteroaryl ist Pyridinyl, Thienyl, Pyrimidinyl, Imidazolyl, Furanyl, Benzimidazolyl, Pyrazolyl, Thiazolyl, Isoxazolyl, Pyrrolyl, Pyrazinyl, 3-Oxo-1,3-dihydroisobenzofuranyl oder 4,5,6,7-Tetrahydrobenzothiazolyl;
Heterocyclyl ist Morpholinyl, Pyrrolidinyl, Piperidinyl, Tetrahydropyranyl, Thiazolidinyl, Dihydroisoxazolyl, Piperazinyl oder Tetrahydrofuranyl;
oder ein physiologisch verträgliches Salz davon.

6. Verbindung nach einem der Ansprüche 1 bis 5, worin in der allgemeinen Formel (I) bedeuten:
X ist eine Einfachbindung oder NH;
R1 ist Wasserstoff oder C₁-C₃-Alkyl;
R2 ist Wasserstoff;
R3 ist -CH₂-NR4R5, -C(O)NR4R5 oder -CH(OH)R7;
R4 ist ausgewählt aus der Gruppe bestehend aus; Wasserstoff; unsubstituiertes oder zumindest monosubstituiertes C₁-C₁₀-Alkyl, Cyclohexenyl, Indanyl, Phenyl, Pyrrolidinyl, Pyrrolyl, Pyrazolyl, Furanyl und Piperidinyl,
wobei die Substituenten ausgewählt sind aus der Gruppe bestehend aus: Fluor, -CN, -C(O)NH₂, -O-Phenyl, -C(O)-Phenyl, -N(CH₃)₂, C₁-C₃-Alkyl, C₁-C₃-Alkoxy, Hydroxy, unsubstituiertes oder zumindest monosubstituiertes Phenyl, Pyridinyl, Thienyl, Pyrimidinyl, Imidazolyl, Furanyl, Indolyl, Benzimidazolyl, Pyrazolyl, Morpholinyl, Pyrrolidinyl, 1,3-Benzodioxolyl, Piperidinyl, Tetrahydropyranyl, Triazolyl, Thiazolyl, Thiazolidinyl, Isoxazolyl und Dihydroisoxazolyl, deren Substituenten sind wiederum ausgewählt aus der Gruppe bestehend aus: Fluor, Chlor, Oxo, CF₃, -OCF₃, -NO₂, Phenyl, Pyridinyl, -NHC(O)CH₃, -COOH, Hydroxy, C₁-C₃-Alkyl, C₁-C₃-Alkoxy, -SO₂NH₂, -C(O)NH₂ und -N(CH₃)₂; und R5 ist Wasserstoff; oder
R4 und R5 bilden zusammen mit dem Stickstoffatom, an das sie gebunden sind, einen Rest ausgewählt aus der Gruppe bestehend aus: unsubstituiertes oder zumindest monosubstituiertes Piperidinyl, Pyrrolidinyl, Morpholinyl und Piperazinyl,
wobei die Substituenten ausgewählt sind aus der Gruppe bestehend aus: Fluor, -C(O)(C₁-C₃-Alkyl), Oxo, C₁-C₃-Alkyl, Hydroxy, unsubstituiertes oder zumindest monosubstituiertes Phenyl, Imidazolyl, Pyridinyl, Pyrimidinyl, Piperidinyl und Pyrrolidinyl, deren Substituenten sind wiederum Fluor oder C₁-C₃-Alkyl-,
R7 ist Wasserstoff;
Ar ist monosubstituiertes Phenyl, Thienyl, Furanyl oder Pyridinyl, wobei der Substituent ausgewählt ist aus der Gruppe bestehend aus: Fluor, Chlor, -CF₃, -OCF₃, -C(O)(C₁-C₃-Alkyl), -NH₂, -NHC(O)(C₁-C₃-Alkyl), Hydroxy, C₁-C₃-Alkyl, C₁-C₃-Alkoxy, -NH(C₁-C₃-Alkyl), -N(C₁-C₃-Alkyl)₂, - SO₂(C₁-C₃-Alkyl) und R3; und der Substituent und X in meta-Position zueinander stehen;
oder ein physiologisch verträgliches Salz davon.

7. Verbindung nach einem der Ansprüche 1 bis 6, worin in der allgemeinen Formel (I) bedeuten:
X ist eine Einfachbindung oder NH;
R1 ist Wasserstoff oder C₁-C₃-Alkyl;
R2 ist Wasserstoff;
R3 ist -CH₂-NR4R5;
R4 ist unsubstituiertes oder zumindest monosubstituiertes C₁-C₆-Alkyl,
wobei die Substituenten ausgewählt sind aus der Gruppe bestehend aus: -N(CH₃)₂, Hydroxy, unsubstituiertes oder zumindest monosubstituiertes Phenyl, Pyridinyl, Imidazolyl, Indolyl, Benzimidazolyl, Pyrazolyl und Pyrrolidinyl, deren Substituenten sind wiederum ausgewählt aus der Gruppe bestehend aus: -NHC(O)CH₃, C₁-C₃-Alkyl, C₁-C₃-Alkoxy, -SO₂NH₂ und -C(O)NH₂; und R5 ist Wasserstoff; oder
R4 und R5 bilden zusammen mit dem Stickstoffatom, an das sie gebunden sind, einen Rest ausgewählt aus der Gruppe bestehend aus: unsubstituiertes oder zumindest monosubstituiertes Pyrrolidinyl, Piperidinyl und Piperazinyl,
wobei die Substituenten ausgewählt sind aus der Gruppe bestehend aus: C₁-C₃-Alkyl, Hydroxy und Pyrrolidinyl;
Ar ist monosubstituiertes Phenyl, Thienyl, Furanyl oder Pyridinyl, wobei der Substituent ausgewählt ist aus der Gruppe bestehend aus: Fluor, Chlor, -OCF₃, -C(O)CH₃, -NHC(O)CH₃, Hydroxy, -N(CH₃)₂, Ethoxy, - SO₂CH₃ und R3;
und der Substituent und X in meta-Position zueinander stehen;
oder ein physiologisch verträgliches Salz davon;.

8. Verbindung nach einem der Ansprüche 1 bis 6, ausgewählt aus der Gruppe bestehend aus:
4-(3-Methansulfonyl-phenylamino)-3-methyl-5,6,7,8-tetrahydro-2H-isochinolin-1-on, 4-(3-Acetyl-phenylamino)-3-methyl-5,6,7,8-tetrahydro-2H-isochinolin-1-on, 4-(5-Butylaminomethyl-furan-2-yl)-3-methyl-5,6,7,8-tetrahydro-2H-isochinolin-1-on, 3-Methyl-4-(5-pyrrolidin-1-ylmethyl-furan-2-yl)-5,6,7,8-tetrahydro-2H-isochinolin-1-on, 4-[5-(3-Hydroxy-pyrrolidin-1-ylmethyl)-furan-2-yl]-5,6,7,8-tetrahydro-2H-isochinolin-1- on, 4-(5-{[(4-Pyrrolidin-1-yl-piperidin-1-ylmethyl)-amino]-methyl}-furan-2-yl)-5,6,7,8- tetrahydro-2H-isochinolin-1-on, 4-{5-[(2-Dimethylamino-ethylamino)-methyl]-furan-2-yl}-5,6,7,8-tetrahydro-2H-isochinolin-1-on, 4-{5-[(2-Hydroxy-2-phenyl-ethylamino)-methyl]-furan-2-yl}-5,6,7,8-tetrahydro-2H- isochinolin-1-on, 4-(5-{[(4-Methyl-piperazin-1-ylmethyl)-amino]-methyl}-furan-2-yl)-5,6,7,8-tetrahydro-2H-isochinolin-1-on, 4-(5-{[(1-Methyl-1H-pyrazol-4-ylmethyl)-amino]-methyl}-furan-2-yl)-5,6,7,8-tetrahydro-2H-isochinolin-1-on, 4-(5-Butylaminomethyl-furan-2-yl)-5,6,7,8-tetrahydro-2H-isochinolin-1-on und 4-(5-Hydroxymethyl-furan-2-yl)-3-methyl-5,6,7,8-tetrahydro-2H-isochinolin-1-on;
oder ein physiologisch verträgliches Salz davon.

9. Verbindung nach einem der Ansprüche 1 bis 8 oder ein physiologisch verträgliches Salz davon zur Verwendung als Arzneimittel.

10. Verwendung einer Verbindung nach einen der Ansprüche 1 bis 8 oder einem physiologisch verträglichen Salz davon zur Herstellung von einem Medikament zur Prophylaxe und/oder Behandlung einer Krankheit, die ausgewählt ist aus der Gruppe bestehend aus: Gewebeschaden resultierend aus Zellschädigung oder Zelltod aufgrund von Nekrose oder Apoptose, neuronal vermittelter Gewebeschaden oder Erkrankungen, zerebrale Ischämie, Kopftrauma, Schlaganfall, Reperfusionsschaden, neurologische Störungen und neurodegenerative Erkrankungen, vaskulärer Schlaganfall, Herz-Kreislauf-Störungen, Myokardinfarkt, Myokardischämie, experimentelle allergische Enzephalomyelitis (EAE), Multiple Sklerose (MS), Ischämie in Bezug zu Herzchirurgie, altersbezogene Makuladegeneration, Arthritis, Arterosklerose, Krebs, degenerative Erkrankungen des Skelettmuskels mit folgender replikativer Seneszenz, Diabetes und diabetische Herzmuskelerkrankungen.

11. Verwendung gemäß Anspruch 10, **dadurch gekennzeichnet, dass** die Krankheit ausgewählt ist aus der Gruppe bestehend aus: zerebrale Ischämie, Reperfusionsschaden, Herz-Kreislauf-Störungen, Myokardinfarkt, Myokardischämie und Ischämie in Bezug zu Herzchirurgie.

12. Verwendung gemäß Anspruch 10 oder 11, **dadurch gekennzeichnet, dass** die Krankheit Myokardinfarkt ist.

13. Pharmazeutische Zusammensetzung umfassend eine wirksame Menge von mindestens einer Verbindung oder einem physiologisch verträglichen Salz davon gemäß einem der Ansprüche 1 bis 8 und einen physiologisch verträglichen Träger.

14. Pharmazeutische Zusammensetzung gemäß Anspruch 13, wobei die pharmazeutische Zusammensetzung in Form einer Pille, Tablette, beschichteten Tablette, Lutschtablette, Granulat, Kapsel, harten oder weichen Gelatinkapsel, wässrigen Lösungen, alkoholischen Lösung, ölartigen Lösung, Sirup, Emulsion, Suspension. Pastille, Lösung zur Injektion oder Infusion, Salbe, Tinktur, Creme, Lotion, Puder, Spray, transdermalen therapeutischen Systems, Nasenspray, Aerosol, Aerosol-Mischung, Mikrokapsel, Implantat, Stab oder Pflaster vorliegt.

15. Verfahren zur Herstellung einer Verbindung der Formel (I) gemäß einem der Ansprüche 1 bis 8, wobei, unter der Voraussetzung, dass in der Formel (I) X eine Einfachbindung und der Substituent Ar mit R3 zumindest monosubstituiert ist (Formel Ib), 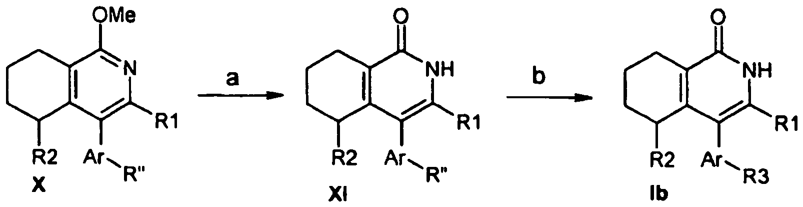
a) eine Verbindung der Formel (X) mit einer geeigneten Reagenz unter Abspaltung der Methylgruppe zu der Verbindung der Formel (XI) umgesetzt wird und
b) die Verbindung der Formel (XI)
1. mit einem geeigneten Amin in Gegenwart eines Reduktionsmittels umgesetzt wird, wenn R" = -CHO und R3 = -CH₂-NR4R5 sind, oder
2. mit einem geeigneten Reduktionsmittel reduziert wird, wenn R" = - CHO und R3 = -CH₂OH sind, oder
3. durch reduktive Aminierung mit Ammoniumacetat umgesetzt wird mit nachfolgender Kupplungsreaktion mit einem geeigneten Säurechlorid, einer geeigneten Säure oder einem geeigneten Sulfonylchlorid, wenn R" = -CHO und R3 = -CH₂NHC(O)R6 oder - CH₂NHSO₂R6 sind, oder
4. mit einem geeigneten Amin in Gegenwart eines Kondensationsmittels umgesetzt wird, wenn R" = -COOH und R3 = -C(O)NR4R5 sind.

16. Verbindung der allgemeinen Formel (XI), 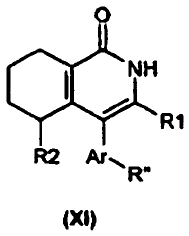 wobei
R1 ist Wasserstoff, Fluor, Chlor, -CN, Methoxy, -OCF₃ oder C₁-C₃-Alkyl, das gegebenenfalls mit Hydroxy, Chlor, Methoxy oder einem, zwei oder drei Fluoratomen substituiert ist;
R2 ist Wasserstoff, Fluor, -CN, Hydroxy, Methoxy, -OCF₃, -NH₂, -NH(C₁-C₃-Alkyl), -N(C₁-C₃-Alkyl)₂ oder C₁-C₃-Alkyl, das gegebenenfalls mit Hydroxy, Chlor, Methoxy oder einem, zwei oder drei Fluoratomen substituiert ist;
R8 ist C₁-C₃-Alkoxy, -O-Phenyl, C₁-C₃-Alkyl, -NH₂. -NH(C₁-C₃-Alkyl), -N(C₁-C₃-Alkyl)₂ oder Phenyl,
und die vorstehenden Phenyl-Fragmente können wiederum mit Fluor, Chlor, Brom, Oxo, -CF₃, -OCF₃, -NO₂, -CN, Aryl, Heteroaryl, -NHC(O)(C₁-C₃-Alkyl), -COOH, Hydroxy, C₁-C₃-Alkyl, C₁-C₃-Alkoxy, -SO₂NH₂, - SO₂NH(C₁-C₃-Alkyl), -SO₂N(C₁-C₃-Alkyl)₂, -C(O)NH₂, -C(O)NH(C₁-C₃-Alkyl), -C(O)N(C₁-C₃-Alkyl)₂, -SO₂(C₁-C₃-Alkyl), -NH₂, -NH(C₁-C₃-Alkyl) oder -N(C₁-C₃-Alkyl)₂ zumindest monosubstituiert sein:
Ar ist Aryl oder Heteroaryl,
wobei dieses Aryl oder Heteroaryl gegebenenfalls mit zumindest einem Substituenten substituiert ist ausgewählt aus der Gruppe bestehend aus: Fluor, Chlor, Brom, -CF₃, -OCF₃, -NO₂, -CN, -C(O)R8, -NH₂, - NHC(O)(C₁-C₆-Alkyl), Hydroxy, C₁-C₆-Alkyl, C₁-C₆-Alkoxy, -CH₂-CH₂-CH₂-, -CH₂-O-C(O)-, -CH₂-C(O)-O-, -CH₂-NH-C(O)-, -CH₂-N(CH₃)-C(O)-, -CH₂-C(O)-NH-, -NH(C₁-C₆-Alkyl), -N(C₁-C₆-Alkyl)₂, -SO₂(C₁-C₆-Alkyl), Heterocyclyl, Heteroaryl und Aryl,
und von diesen Substituenten können Heterocyclyl, Aryl und Heteroaryl wiederum mit C₁-C₆-Alkyl, C₁-C₆-Alkoxy, Fluor, Chlor, Brom, Trifluormethyl, Tritluomnethoxy oder OH zumindest monosubstituiert sein;
R" ist -COOH, -CHO oder -SO₂Cl;
Heteroaryl ist ein 5 bis 10-gliedriger, aromatischer, mono- oder bicyclischer Heterocyclus, der ein oder mehrere Heteroatome ausgewählt aus N, O und S enthält;
Heterocyclyl ist ein 5 bis 10-gliedriger, nicht-aromatischer, mono- oder bicyclischer Heterocyclus, der ein oder mehrere Heteroatome ausgewählt aus N, O und S enthält;
Aryl ist ein 5 bis 10-gliedriger, aromatischer. Mono- oder Bicyclus. 17. Verbindung nach Anspruch 16, worin in der allgemeinen Formel (XI) bedeuten:
R1 ist Wasserstoff oder C₁-C₃-Alkyl;
R2 ist Wasserstoff,
Ar ist Phenyl, Furanyl, Thienyl oder Pyridinyl, wobei R" und das Tetrahydroisochinolinon-Fragment in meta-Position zueinander stehen;
R" ist -CHO oder -COOH.

## Claims

1. A compound of the general formula (I) 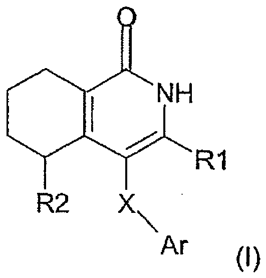 in which the meanings are:
X is a single bond, 0, S, NH or N(C₁-C₃-alkyl);
R1 is hydrogen, fluorine, chlorine, -CN, methoxy, - OCF₃ or C₁-C₃-alkyl which is optionally substituted by hydroxy, chlorine, methoxy or one, two or three fluorine atoms;
R2 is hydrogen, fluorine, -CN, hydroxy, methoxy, - OCF₃, -NH₂, -NH (C₁-C₃-alkyl) , -N (C₁-C₃-alkyl)₂ or C₁-C₃-alkyl which is optionally substituted by hydroxy, chlorine, methoxy or one, two or three fluorine atoms;
R3 is - (C₁-C₃-alkyl) -NR4R5, -SO₂NR4R5, -C(O)NR4R5, - C(H)=N-OR9, -C(O)R6, -NHC(O)R6, - (C₁-C₃-alkyl) - NHC(O)R6, -NHSO₂R6, -(C1-C3-alkyl) -NHSO2R6 or - CH(OH)R7;
R4 and R5 are independently of one another selected from the group consisting of: hydrogen; unsubstituted or at least monosubstituted C₁-C₁₀-alkyl, C₂-C₆-alkenyl, C₂-C₆-alkynyl, aryl, heteroaryl and heterocyclyl,
where the substituents are selected from the group consisting of: aryl, heteroaryl, heterocyclyl, -0-aryl, fluorine, chlorine, bromine, -CF₃, -OCF₃, - NO₂, -CN, -C(O)R8, -NHC(O) (C₁-C₃-alkyl), -NH₂, hydroxy, C₁-C₆-alkyl, C₁-C₃-alkoxy, -NH(C₁-C₃-alkyl), -N(C₁-C₃-alkyl)₂, -NH-aryl, -NH-heteroaryl, -NH-C(O)-heteroaryl, -SO₂NH₂, -SO2(C₁-C₃-alkyl) and -NH-SO₂(C₁-C₃-alkyl),
and the aryl, heteroaryl and heterocyclyl fragments of these substituents may in turn be at least monosubstituted by fluorine, chlorine, bromine, oxo, -CF₃, -OCF₃, -NO₂, -CN, aryl, heteroaryl, -NHC(O)(C₁-C₃-alkyl), -COOH, hydroxy, C₁-C₃-alkyl, C₁-C₃-alkoxy, -SO₂NH₂, -SO₂NH (C₁-C₃-alkyl), -SO₂N(C₁-C₃-alkyl)₂, -C(O)NH2, -C(O)NH(C₁-C₃-alkyl), -C(O)N (C₁-C₃-alkyl)₂, -SO₂ (C₁-C₃-alkyl), -NH₂, -NH(C₁-C₃-alkyl) or -N(C₁-C₃-alkyl)₂; or
R4 and R5 form together with the nitrogen atom to which they are bonded unsubstituted or at least monosubstituted heterocyclyl,
where the substituents are selected from the group consisting of: aryl, heteroaryl, heterocyclyl, oxo, fluorine, chlorine, bromine, -CF₃, -OCF₃, - NO₂, -CN, -C(O)R8, -NHC(O) (C₁-C₃-alkyl), -NH₂, hydroxy, C₁-C₃-alkyl, C₁-C₃-alkoxy, -NH(C₁-C₃-alkyl), -N (C₁-C₃-alkyl)₂, -SO₂NH₂, -SO₂(C₁-C₃-alkyl) and -NH-SO₂(C₁-C₃-alkyl), and, of these substituents, aryl, heterocyclyl and heteroaryl in turn may be at least monosubstituted by fluorine, chlorine, bromine, hydroxy,- C₁-C₃-alkyl or C₁-C₃-alkoxy;
R6 is unsubstituted or at least monosubstituted C₁-C₆-alkyl, phenyl, heteroaryl or heterocyclyl, where the substituents are selected from the group consisting of: fluorine, chlorine, bromine, aryl, heterocyclyl, heteroaryl, -CF₃, -OCF₃, -NO₂, -CN, - C(O)R8, -NHC(O)(C₁-C₃-alkyl), -NH₂, hydroxy, C₁-C₃-alkyl, C₁-C₃-alkoxy, -0-heteroaryl, -O-aryl, - NH (C₁-C₃-alkyl), -N(C₁-C₃-alkyl)₂, -SO₂NH₂, -SO₂(C₁-C₃-alkyl) and -NH-SO₂(C₁-C₃-alkyl), and the aryl, heterocyclyl and heteroaryl fragments of these substituents may in turn be at least monosubstituted by fluorine, chlorine, bromine, hydroxy, C₁-C₃-alkyl or C₁-C₃-alkoxy;
R7 is selected from the group consisting of: hydrogen; unsubstituted or at least monosubstituted C₁-C₆-alkyl, phenyl and heteroaryl, where the substituents are selected from the group consisting of:
fluorine, chlorine, bromine, -CF₃, -OCF₃, -NO₂, - CN, -C(O)R8, -NHC(O) (C₁-C₃-alkyl), -NH₂, hydroxy, C₁-C₃-alkyl, C₁-C₃-alkoxy, -NH (C₁-C₃-alkyl), -N(C₁-C₃-alkyl)₂, -SO₂NH₂, -SO₂(C₁-C₃-alkyl) and -NH-SO₂(C₁-C₃-alkyl);
R8 is C₁-C₃-alkoxy, -O-phenyl, C₁-C₃-alkyl, -NH₂, - NH(C₁-C₃-alkyl), -N(C₁-C₃-alkyl)₂ or phenyl,
and the above phenyl fragments may in turn be at least monosubstituted by fluorine, chlorine, bromine, oxo, -CF₃, -OCF₃, -NO₂, -CN, aryl, heteroaryl, -NHC(O)(C₁-C₃-alkyl), -COOH, hydroxy, C₁-C₃-alkyl, C₁-C₃-alkoxy, -SO₂NH₂, -SO₂NH(C₁-C₃-alkyl), -SO₂N (C₁-C₃-alkyl)₂, -C(O)NH₂, -C(O)NH(C₁-C₃-alkyl), -C(O)N (C₁-C₃-alkyl )₂, -SO₂(C₁-C₃-alkyl), NH₂, -NH(C₁-C₃-alkyl) or -N(C₁-C₃-alkyl)₂;
R9 is selected from the group consisting of: hydrogen; unsubstituted or at least monosubstituted C₁-C₆-alkyl and phenyl, where the substituents are selected from the group consisting of:
fluorine, chlorine, bromine, aryl, heterocyclyl, heteroaryl, -CF₃, -OCF₃, -NO₂, -CN, -C(O)R8, - NHC(O) (C₁-C₃-alkyl), C₁-C₃-alkyl, C₁-C₃-alkoxy, - NH(C₁-C₃-alkyl), -N(C₁-C₃-alkyl)₂, -SO₂NH₂, -SO₂(C₁-C₃-alkyl) and -NH-SO₂(C₁-C₃-alkyl),
and, of these substituents, aryl, heterocyclyl and heteroaryl may in turn be at least monosubstituted by fluorine, chlorine, bromine, C₁-C₃-alkyl or C₁-C₃-alkoxy;
Ar is unsubstituted or at least monosubstituted aryl or heteroaryl, where the substituents are selected from the group consisting of: fluorine, chlorine, bromine, -CF₃, -OCF₃, -NO₂, -CN, -C(O)R8, -NH₂, - NHC(O) (C₁-C₆-alkyl), hydroxy, C₁-C₆-alkyl, C₁-C₆-alkoxy, -CH₂-CH₂-CH₂-, -CH₂-O-C(O)-, -CH₂-C(O)-O-, - CH₂-NH-C(O)-, -CH₂-N(CH₃)-C(O)-, -CH₂-C(O)-NH-, - NH(C₁-C₆-alkyl), -N(C₁-C₆-alkyl)₂, -SO₂(C₁-C₆-alkyl), heterocyclyl, heteroaryl, aryl and R3,
and, of these substituents, heterocyclyl, aryl and heteroaryl may in turn be at least monosubstituted by C₁-C₆-alkyl, C₁-C₆-alkoxy, fluorine, chlorine, bromine, trifluoromethyl, trifluoromethoxy or OH;
heteroaryl is a 5 to 10-membered, aromatic, mono- or bicyclic heterocycle which comprises one or more heteroatoms selected from N, O and S;
aryl is a 5 to 10-membered, aromatic, mono- or bicycle; heterocyclyl is a 5 to 10-membered, non-aromatic, mono-or bicyclic heterocycle which comprises one or more heteroatoms selected from N, Oand S;
or a physiologically tolerated salt thereof;
provided that Ar is not unsubstituted phenyl when X is a single bond and further provided that the compound 3-(3-methyl-1-oxo-1,2,5,6,7,8-hexahydroisoquinolin-4-yl)benzonitrile is excluded.

2. The compound of the general formula (I) as claimed in claim 1, in which R1, R2, R3 and X have the meanings given in claim 1 and
Ar is unsubstituted or at least monosubstituted phenyl or heteroaryl,
where the substituents are selected from the group consisting of: fluorine, chlorine, -CF₃, -OCF₃, - C (0) (C₁-C₃-alkyl), -NH₂, -NHC (O) (C₁-C₃-alkyl), hydroxy, C₁-C₃-alkyl, C₁-C₃-alkoxy, -CH₂-CH₂-CH₂-, -CH₂-O-C(O)-, -CH₂-C(O) -O-, -CH₂-NH-C(O) -, -CH₂-N(CH₃)-C(O)-, -CH₂-C (O) -NH-, -NH (C₁-C₃-alkyl), - N(C₁-C₃-alkyl)₂, -SO₂(C₁-C₃-alkyl), heterocyclyl, heteroaryl and R3,
and, of these substituents, heterocyclyl and heteroaryl in turn may be at least monosubstituted by C₁-C₃-alkyl, C₁-C₃-alkoxy, fluorine, chlorine, bromine, trifluoromethyl, trifluoromethoxy or OH;
or a physiologically tolerated salt thereof;
provided that Ar is not unsubstitued phenyl when X is a single bond.

3. A compound as claimed in claim 1 or 2, in which the meanings in the general formula (I) are:
X is a single bond, NH or N(C₁-C₃-alkyl) ;
R1 is hydrogen or C₁-C₃-alkyl which is optionally substituted by hydroxy, chlorine, methoxy or one, two or three fluorine atoms;
R2 is hydrogen, fluorine, -OCF₃, hydroxy, methoxy, -NH₂ or C₁-C₃-alkyl;
R3 is -CH₂-NR4R5, -SO₂NR4R5, -C(O)NR4R5, -CH₂-NHC(O)R6, -CH₂-NHSO₂R6 or -CH(OH)R7;
R4 and R5 are independently of one another selected from the group consisting of: hydrogen; unsubstituted or at least monosubstituted C₁-C₁₀-alkyl, C₂-C₆-alkenyl, phenyl, indanyl, heterocyclyl and heteroaryl,
where the substituents are selected from the group consisting of: phenyl, heteroaryl, heterocyclyl, -O-phenyl, fluorine, -CN, -C(O)NH2, -C(O) (C₁-C₃-alkyl), -C(O)-phenyl, - N(C₁-C₃-alkyl)₂, -NH(C₁-C₃-alkyl), -NH₂, -NH-heteroaryl, -NH-C(O)-heteroaryl, C₁-C₆-alkyl, C₁-C₃-alkoxy and hydroxy,
and the phenyl, heterocyclyl and heteroaryl fragments of these substituents may in turn be at least monosubstituted by fluorine, chlorine, bromine, oxo, -CF₃, -OCF₃, -NO₂, - CN, phenyl, pyrimidinyl, -NHC(O)(C₁-C₃-alkyl), -COOH, hydroxy, C₁-C₃-alkyl, C₁-C₃-alkoxy, - SO₂NH₂, -SO₂NH (C₁-C₃-alkyl) , -SO₂N (C₁-C₃-alkyl)₂, -C(O)NH₂, -C(O)NH(C₁-C₃-alkyl), -C(O)N(C₁-C₃-alkyl)₂, -SO₂(C₁-C₃-alkyl), -NH₂, - NH(C₁-C₃-alkyl) or -N(C₁-C₃-alkyl)₂; or
R4 and R5 form together with the nitrogen atom to which they are bonded unsubstituted or at least monosubstituted heterocyclyl, where the substituents are selected from the group consisting of: phenyl, heteroaryl, heterocyclyl, oxo, fluorine, chlorine, - C(O) (C₁-C₃-alkyl), -C(O)-phenyl and hydroxy,
and the phenyl, heterocyclyl and heteroaryl fragments of these substituents may in turn be at least monosubstituted by fluorine or C₁-C₃-alkyl;
R6 is unsubstituted or at least monosubstituted C₁-C₆-alkyl, phenyl or heteroaryl,
where the substituents are selected from the group consisting of: fluorine, chlorine, bromine, -CF₃, -OCF₃, -NHC (O) (C₁-C₃-alkyl) , hydroxy, C₁-C₃-alkyl, C₁-C₃-alkoxy, -0-heteroaryl, phenyl, -NH₂, -NH(C₁-C₃-alkyl), - N(C₁-C₃-alkyl)₂ and heterocyclyl,
and the phenyl, heteroaryl and heterocyclyl fragments of these substituents may in turn be at least monosubstituted by fluorine, chlorine, bromine, hydroxy, C₁-C₃-alkyl or C₁-C₃-alkoxy;
R7 is selected from the group consisting of: hydrogen; unsubstituted or at least monosubstituted C₁-C₆-alkyl, phenyl and pyridinyl,
where the substituents are selected from the group consisting of: fluorine, chlorine, bromine, hydroxy, C₁-C₃-alkyl and C₁-C₃-alkoxy;
Ar is unsubstituted or at least monosubstituted phenyl or heteroaryl,
where the substituents are selected from the group consisting of: fluorine, chlorine, -CF₃, -OCF₃, C(O) (C₁-C₃-alkyl) , -NH₂, -NHC(O)(C₁-C₃-alkyl), hydroxy, C₁-C₃-alkyl, C₁-C₃-alkoxy, -CH₂-CH₂-CH₂-, -CH₂-O-C(O)-, -CH₂-C (O) -O-, - CH₂-NH-C(O)-, -CH₂-N(CH₃)-C(O)-, -CH₂-C(O)-NH-, -NH(C₁-C₃-alkyl), -N(C₁-C₃-alkyl)₂, -SO₂ (C₁-C₃-alkyl), heterocyclyl, heteroaryl and R3;
and, of these substituents, heterocyclyl and heteroaryl may in turn be at least monosubstituted by C₁-C₃-alkyl, C₁-C₃-alkoxy, fluorine, chlorine, bromine, trifluoromethyl, trifluoromethoxy or OH;
heteroaryl is pyridinyl, thienyl, pyrimidinyl, imidazolyl, furanyl, indolyl, benzimidazolyl, pyrazolyl, 1,3-benzodioxolyl, triazolyl, thiazolyl, isoxazolyl, pyrrolyl, pyrazinyl, oxazolyl, pyridazinyl, quinolinyl, isoquinolyl, benzofuranyl, 3-oxo-1,3-dihydroisobenzofuranyl or 4,5,6,7-tetrahydrobenzothiazolyl;
heterocyclyl is morpholinyl, pyrrolidinyl, piperidinyl, tetrahydropyranyl, thiazolidinyl, dihydroisoxazolyl, piperazinyl or tetrahydrofuranyl;
or a physiologically tolerated salt thereof;
provided that Ar is not unsubstituted phenyl when X is a single bond.

4. A compound as claimed in claim 1 to 3, in which the meanings in the general formula (I) are:
X is a single bond, NH or N(C₁-C₃-alkyl);
R1 is hydrogen or C₁-C₃-alkyl which is optionally substituted by hydroxy, chlorine, methoxy or one, two or three fluorine atoms;
R2 is hydrogen, fluorine, -OCF₃, hydroxy, methoxy, -NH₂ or C₁-C₃-alkyl;
R3 is -CH₂-NR4R5, -SO₂-NR4R5, -C(O)NR4R5, -CH₂-NHC(O)3R6, -CH₂NHSO₂R6 or -CH(OH)R7;
R4 and R5 are independently of one another selected from the group consisting of: hydrogen; unsubstituted or at least monosubstituted C₁-C₁₀-alkyl, C₂-C₆-alkenyl, phenyl, indanyl, heterocyclyl and heteroaryl,
where the substituents are selected from the group consisting of: phenyl, heteroaryl, heterocyclyl, -O-phenyl, fluorine, -CN, -C(O)NH₂, -C (O) (C₁-C₃-alkyl), -C(O)-phenyl, -N(C₁-C₃-alkyl)₂, -NH(C₁-C₃-alkyl), -NH₂, -NH-heteroaryl, -NH-C(O)-heteroaryl, C₁-C₆-alkyl, C₁-C₃-alkoxy and hydroxy,
and the phenyl, heterocyclyl and heteroaryl fragments of these substituents may in turn be at least monosubstituted by fluorine, chlorine, bromine, oxo, -CF₃, -OCF₃, -NO₂, -CN, phenyl, pyridinyl, -NHC(O)(C₁-C₃-alkyl), -COOH, hydroxy, C₁-C₃-alkyl, C₁-C₃-alkoxy, - SO₂NH₂, -SO₂NH (C₁-C₃-alkyl), -SO₂N(C₁-C₃-alkyl)₂, -C(O)NH₂, -C(O)NH(C₁-C₃-alkyl), -C(O)N(C₁-C₃-alkyl)₂, -SO₂(C₁-C₃-alkyl), -NH₂, - NH(C₁-C₃-alkyl) or N(C₁-C₃-alkyl)₂; or
R4 and R5 form together with the nitrogen atom to which they are bonded unsubstituted or at least monosubstituted heterocyclyl,
where the substituents are selected from the group consisting of: phenyl, heteroaryl, heterocyclyl, oxo, fluorine, chlorine, -C (O) (C₁-C₃-alkyl), -C (O) -phenyl and hydroxy,
and the phenyl, heterocyclyl and heteroaryl fragments of the substituents may in turn be at least monosubstituted by fluorine or C₁-C₃-alkyl;
R6 is CF₃ or unsubstituted or at least monosubstituted C₁-C₆-alkyl, pyridinyl, furanyl or phenyl,
where the substituents are selected from the group consisting of: fluorine, -NHC(O)(C₁-C₃-alkyl), hydroxy C₁-C₃-alkyl, C₁-C₃-alkoxy and - O-pyridinyl;
R7 is selected from the group consisting of: hydrogen; unsubstituted or at least monosubstituted C₁-C₆-alkyl, phenyl and pyridinyl,
where the substituents are selected from the group consisting of: fluorine, chlorine, bromine, hydroxy, C₁-C₃-alkyl and C₁-C₃-alkoxy;
Ar is unsubstituted or at least monosubstituted phenyl, thienyl, furanyl or pyridinyl, where the substituents are selected from the group consisting of: fluorine, chlorine, -CF₃, -OCF₃, C(O) (C₁-C₃-alkyl), -NH₂, -NHC(O)(C₁-C₃-alkyl), hydroxy, C₁-C₃-alkyl, C₁-C₃-alkoxy, -CH₂-CH₂-CH₂-, -CH₂-O-C(O)-, -CH₂-C (0) -O-, - CH₂-NH-C(O)-, -CH₂-N(CH₃)-C(O)-, -CH₂-C(O)-NH-, -NH(C₁-C₃-alkyl), -N(C₁-C₃-alkyl)₂, -SO₂(C₁-C₃-alkyl) and R3;
heteroaryl is pyridinyl, thienyl, pyrimidinyl, imidazolyl, furanyl, indolyl, benzimidazolyl, pyrazolyl, 1,3-benzodioxolyl, triazolyl, thiazolyl, isoxazolyl, pyrrolyl, pyrazinyl, oxazolyl, pyridazinyl, quinolinyl, isoquinolyl, benzofuranyl, 3-oxo-1,3-dihydroisobenzofuranyl or 4,5,6,7-tetrahydrobenzothiazolyl;
heterocyclyl is morpholinyl, pyrrolidinyl, piperidinyl, tetrahydropyranyl, thiazolidinyl, dihydroxyisoxazolyl, piperazinyl or tetrahydrofuranyl;
or a physiologically tolerated salt thereof;
provided that Ar is not unsubstituted phenyl when X is a single bond.

5. A compound as claimed in any of claims 1 to 4, in which the meanings in the general formula (I) are:
X is a single bond, NH or N(C₁-C₃-alkyl) ;
R1 is hydrogen or C₁-C₃-alkyl;
R2 is hydrogen;
R3 is -CH₂-NR4R5, -CH₂-NHC(O)R6, -CH₂-NHSO₂R6, -C(O)NR4R5 or -CH(OH)R7;
R4 is selected from the group consisting of: hydrogen, unsubstituted or at least monosubstituted C₁-C₁₀-alkyl, C₁-C₆-alkenyl, phenyl, indanyl, heterocyclyl and heteroaryl, where the substituents are selected from the group consisting of: phenyl, heteroaryl, heterocyclyl, -O-phenyl, fluorine, -CN, -C(O)NH₂, -C(O) (C₁-C₃-alkyl), -C(O)-phenyl, - N(C₁-C₃-alkyl)₂, -NH (C₁-C₃-alkyl), -NH₂, -NH-heteroaryl, -NH-C(O)-heteroaryl, C₁-C₆-alkyl, C₁-C₃-alkoxy and hydroxy,
and the phenyl, heterocyclyl and heteroaryl fragments of these substituents may in turn be at least monosubstituted by fluorine, chlorine, bromine, oxo, -CF₃, -OCF₃, -NO₂, - CN, phenyl, pyridinyl, -NHC(O)(C₁-C₃-alkyl), - COOH, hydroxy, C₁-C₃-alkyl, C₁-C₃-alkoxy, -SO₂NH₂, -SO₂NH (C₁-C₃-alkyl), -SO₂N(C₁-C₃-alkyl)₂,
-C(O)NH₂, -C (O)NH (C₁-C₃-alkyl), -C(O)N (C₁-C₃-alkyl)₂,
-SO₂(C₁-C₃-alkyl), -NH₂, -NH(C₁-C₃-alkyl) or - N(C₁-C₃-alkyl)₂; and R5 is hydrogen; or
R4 and R5 form together with the nitrogen atom to which they are bonded unsubstituted
or at least monosubstituted heterocyclyl, where the substituents are selected from the group consisting of: phenyl, heteroaryl, heterocyclyl, oxo, fluorine, chlorine, -C(O)(C₁-C₃-alkyl), -C(O)-phenyl and hydroxy,
and the phenyl, heterocyclyl and heteroaryl fragments of these substituents may in turn be at least monosubstituted by fluorine or C₁-C₃-alkyl;
R6 is CF₃ or unsubstituted or at least monosubstituted C₁-C₆-alkyl, pyridinyl, furanyl or phenyl,
where the substituents are selected from the group consisting of: fluorine, -NHC(O)(C₁-C₃-alkyl), hydroxy, C₁-C₃-alkyl, C₁-C₃-alkoxy and -O-pyridinyl;
R7 is selected from the group consisting of: hydrogen; unsubstituted or at least monosubstituted C₁-C₆-alkyl, phenyl and pyridinyl,
where the substituents are selected from the group consisting of: fluorine, chlorine, bromine, hydroxy, C₁-C₃-alkyl and C₁-C₃-alkoxy;
Ar is monosubstituted phenyl, thienyl, furanyl or pyridinyl,
where the substituent is selected-from the group consisting of: fluorine, chlorine, -CF₃, -OCF₃, -C(O) (C₁-C₃-alkyl), -NH₂, -NHC(O)(C₁-C₃-alkyl), hydroxy, C₁-C₃-alkyl, C₁-C₃-alkoxy, -NH(C₁-C₃-alkyl), -N(C₁-C₃-alkyl)₂, -SO₂(C₁-C₃-alkyl) and R3;
and the substituent and X are in the meta position relative to one another;
heteroaryl is pyridinyl, thienyl, pyrimidinyl, imidazolyl, furanyl, benzimidazolyl, pyrazolyl, thiazolyl, isoxazolyl, pyrrolyl, pyrazinyl, 3-oxo-1,3-dihydroisobenzofuranyl or 4,5,6,7-tetrahydrobenzothiazolyl;
heterocyclyl is morpholinyl, pyrrolidinyl, piperidinyl, tetrahydropyranyl, thiazolidinyl, dihydroisoxazolyl, piperazinyl or tetrahydrofuranyl;
or a physiologically tolerated salt thereof.

6. A compound as claimed in any of claims 1 to 5, in which the meanings in the general formula (I) are:
X is a single bond or NH;
R1 is hydrogen or C₁-C₃-alkyl;
R2 is hydrogen;
R3 is -CH₂-NR4R5, -C(O)NR4R5 or -CH(OH)R7;
R4 is selected from the group consisting of; hydrogen; unsubstituted or at least monosubstituted C₁-C₁₀-alkyl, cyclohexenyl, indanyl, phenyl, pyrrolidinyl, pyrrolyl, pyrazolyl, furanyl and piperdinyl,
where the substituents are selected from the group consisting of: fluorine, -CN, -C(O)NH₂, -O-phenyl, -C(O)-phenyl, -N(CH₃)₂, C₁-C₃-alkyl, C₁-C₃-alkoxy, hydroxy, unsubstituted or at least monosubstituted phenyl, pyridinyl, thienyl, pyrimidinyl, imidazolyl, furanyl, indolyl, benzimidazolyl, pyrazolyl, morpholinyl, pyrrolidinyl, 1,3-benzodioxolyl, piperidinyl, tetrahydropyranyl, triazolyl, thiazolyl, thiazolidinyl, isoxazolyl and dihydroisoxazolyl, the substituents of which are in turn selected from the group consisting of: fluorine, chlorine, oxo, CF₃, - OCF₃, -NO₂, phenyl, pyridinyl, -NHC(O)CH₃, - COOH, hydroxy, C₁-C₃-alkyl, C₁-C₃-alkoxy, - SO₂NH₂, -C(O)NH₂ and -N(CH₃)₂; and R5 is hydrogen; or
R4 and R5 form together with the nitrogen atom to which they are bonded a radical selected from the group consisting of: unsubstituted or at least monosubstituted piperidinyl, pyrrolidinyl, morpholinyl and piperazinyl,
where the substituents are selected from the group consisting of: fluorine, -C(O)(C₁-C₃-alkyl), oxo, C₁-C₃-alkyl, hydroxy, unsubstituted or at least monosubstituted phenyl, imidazolyl, pyridinyl, pyrimidinyl, piperidinyl and pyrrolidinyl, the substituents of which are in turn fluorine or C₁-C₃-alkyl;
R7 is hydrogen;
Ar is monosubstituted phenyl, thienyl, furanyl
or pyridinyl,
where the substituent is selected from the group consisting of:
fluorine, chlorine, -CF₃, -OCF₃, -C(O)(C₁-C₃-alkyl), -NH₂, -NHC (0) (C₁-C₃-alkyl), hydroxy, C₁-C₃-alkyl, C₁-C₃-alkoxy, -NH(C₁-C₃-alkyl), - N(C₁-C₃-alkyl)₂, -SC₂(C₁-C₃-alkyl) and R3; and the substituent and X are in the meta position relative to one another;
or a physiologically tolerated salt thereof.

7. A compound as claimed in any of claims 1 to 6, in which the meanings in the general formula (I) are:
X is a single bond or NH;
R1 is hydrogen or C₁-C₃-alkyl;
R2 is hydrogen;
R3 is -CH₂-NR4R5;
R4 is unsubstituted or at least monosubstituted C₁-C₆-alkyl,
where the substituents are selected from the group consisting of: -N(CH₃)₂, hydroxy, unsubstituted or at least monosubstituted phenyl, pyridinyl, imidazolyl, indolyl, benzimidazolyl, pyrazolyl and pyrrolidinyl, the substituents of which are in turn selected from the group consisting of: -NHC(O)CH₃, C₁-C₃-alkyl, C₁-C₃-alkoxy, -SO₂NH₂ and -C(O)NH₂; and R5 is hydrogen; or
R4 and R5 form together with the nitrogen atom to which they are bonded a radical selected from the group consisting of: unsubstituted or at least monosubstituted pyrrolidinyl, piperidinyl and piperazinyl,
where the substituents are selected from the group consisting of: C₁-C₃-alkyl, hydroxy and pyrrolidinyl;
Ar is monosubstituted phenyl, thienyl, furanyl or pyridinyl,
where the substituent is selected from the group consisting of:
fluorine, chlorine, -OCF₃, -C(O)CH₃, - NHC(O)CH₃, hydroxy, -N(CH₃)₂, ethoxy, -SO₂CH₃ and R3;
and the substituent and X are in the meta position relative to one another;
or a physiologically tolerated salt thereof.

8. A compound as claimed in any of claims 1 to 6, selected from the group consisting of:
4-(3-methanesulfonylphenylamino)-3-methyl-5,6,7,8-tetrahydro-2H-isoquinolin-1-one, 4-(3-acetylphenylamino)-3-methyl-5,6,7,8-tetrahydro-2H-isoquinolin-1-one, 4-(5-butylaminomethylfuran-2-yl)-3-methyl-5,6,7,8-tetrahydro-2H-isoquinolin-1-one, 3-methyl-4-(5-pyrrolidin-1-ylmethylfuran-2-yl)-5,6,7,8-tetrahydro-2H-isoquinolin-1-one, 4-[5-(3-hydroxypyrrolidin-1-ylmethyl)furan-2-yl]-5,6,7,8-tetrahydro-2H-isoquinolin-1-one, 4-(5-{[(4-pyrrolidin-1-ylpiperidin-1-ylmethyl)amino]methyl}furan-2-yl)-5,6,7,8-tetrahydro-2H-isoquinolin-1-one, 4-{5-[(2-dimethylaminoethylamino)methyl]furan-2-yl)-5,6,7,8-tetrahydro-2H-isoquinolin-1-one, 4-{5-[(2-hydroxy-2-phenylethylamino)methyl]furan-2-yl}-5,6,7,8-tetrahydro-2H-isoquinolin-1-one, 4-(5-{[(4-methylpiperazin-1-ylmethyl)amino]methyl}furan-2-yl)-5,6,7,8-tetrahydro-2H-isoquinolin-1-one, 4-(5-{[(1-methyl-1H-pyrazol-4-ylmethyl)amino]methyl}furan-2-yl)-5,6,7,8-tetrahydro-2H-isoquinolin-1-one, 4-(5-butylaminomethylfuran-2-yl)-5,6,7,8-tetrahydro-2H-isoquinolin-1-one and 4-(5-hydroxymethylfuran-2-yl)-3-methyl-5,6,7,8-tetrahydro-2H-isoquinolin-1-one;
or a physiologically tolerated salt thereof.

9. A compound as claimed in any of claims 1 to 8 or a physiologically acceptable salt thereof for use as pharmaceutical.

10. The use of a compound as claimed in any of claims 1 to 8 or of a physiologically acceptable salt thereof for the manufacture of a medicament for the prophylaxis and/or treatment of a disease which is selected from the group consisting of: tissue damage resulting from cell damage or cell death owing to necrosis or apoptosis, neuronally mediated tissue damage or disorders, cerebral ischemia, head trauma, stroke, reperfusion damage, neurological disturbances and neurodegenerative disorders, vascular stroke, cardiovascular impairments, myocardial infarction, mycocardial ischemia, experimental allergic encephalomyelitis (EAE), multiple sclerosis (MS), ischemia related to heart surgery, age-related macular degeneration, arthritis, arterosclerosis, cancer, degenerative disorders of the skeletal muscles with subsequent replicative senescence, diabetes and diabetic myocardial disorders.

11. The use as claimed in claim 10, wherein the disease is selected from the group consisting of: cerebral ischemia, reperfusion damage, cardiovascular disorders, myocardial infarction, myocardial ischemia and ischemia related to heart surgery.

12. The use as claimed in claim 10 or 11, wherein the disease is myocardial infarction.

13. A pharmaceutical composition comprising an effective amount of at least one compound or of a physiologically acceptable salt thereof as claimed in any of claims 1 to 8 and a physiologically acceptable carrier.

14. A pharmaceutical composition as claimed in claim 13, where the pharmaceutical composition is in the form of a pill, tablet, coated tablet, suckable tablet, granules, capsule, hard or soft gelatin capsule, aqueous solution, alcoholic solution, oily solution, syrup, emulsion, suspension, suppository, pastille, solution for injection or infusion, ointment, tincture, cream, lotion, dusting powder, spray, transdermal therapeutic system, nasal spray, aerosol, aerosol mixture, microcapsule, implant, rod or patch.

15. A method for preparing a compound of the formula (I) as claimed in any of claims 1 to 8, where, provided that in the formula (I) X is a single bond and the substituent Ar is at least monosubstituted by R3 (formula Ib), 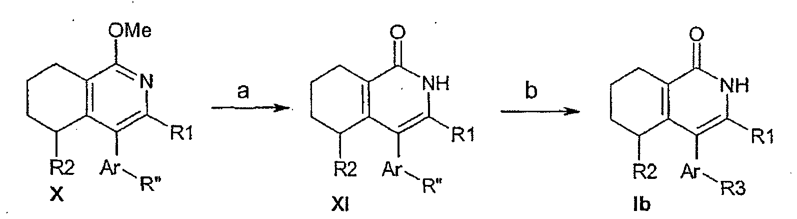
a) a compound of the formula (X) is reacted with a suitable reagent, with elimination of the methyl group, to give the compound of the formula (XI), and
b) the compound of the formula (XI)
1. is reacted with a suitable amine in the presence of a reducing agent when R" is -CHO and R3 is -CH₂-NR4R5, or
2. is reduced with a suitable reducing agent when R" is -CHO and R3 is -CH₂OH, or
3. is reacted by reductive amination with ammonium acetate, with subsequent coupling reaction with a suitable acid chloride, a suitable acid or a suitable sulfonyl chloride when R" is -CHO and R3 is -CH₂NHC(O)R6 or -CH₂NHSO₂R6, or
4. is reacted with a suitable amine in the presence of a condensing agent when R" is -COOH and R3 is -C(O)NR4R5.

16. A compound of the formula (XI) 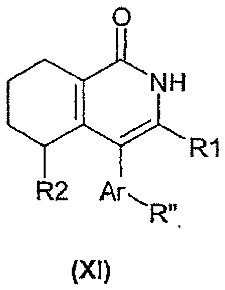 where
R1 is hydrogen, fluorine, chlorine, -CN, methoxy, -OCF₃ or C₁-C₃-alkyl which is optionally substituted by hydroxy, chlorine, methoxy or one, two or three fluorine atoms;
R2 is hydrogen, fluorine, -CN, hydroxy, methoxy, -OCF₃, -NH₂, -NH(C₁-C₃-alkyl), -N(C₁-C₃-alkyl)₂ or C₁-C₃-alkyl which is optionally substituted by hydroxy, chlorine, methoxy or one, two or three fluorine atoms;
R8 is C₁-C₃-alkoxy, -O-phenyl, C₁-C₃-alkyl, -NH₂, -NH(C₁-C₃-alkyl), -N(C₁-C₃-alkyl)₂ or phenyl, and the above phenyl fragments may in turn be at least monosubstituted by fluorine, chlorine, bromine, oxo, -CF₃,
-OCF₃, -NO₂, -CN, aryl, heteroaryl, - NHC (O) (C₁-C₃-alkyl),
-COOH, hydroxy, C₁-C₃-alkyl, C₁-C₃-alkoxy, - SO₂NH₂,
-SO₂NH (C₁-C₃-alkyl), -SO₂N (C₁-C₃-alkyl)2, - C(O)NH₂,
-C(O)NH (C₁-C₃-alkyl) -C(O)N (C₁-C₃-alkyl)₂, - SO₂ (C₁-C₃-alkyl), -NH₂, -NH (C₁-C₃-alkyl) or - N(C₁-C₃-alkyl)₂;
Ar is aryl or heteroaryl, where this aryl or heteroaryl is optionally substituted by at least one substituent selected from the group consisting of: fluorine, chlorine, bromine, -CF₃, -OCF₃, - NO₂, -CN,
-C(O)R8, -NH₂, -NHC(O) (C₁-C₆-alkyl), hydroxy, C₁-C₆-alkyl, C₁-C₆-alkoxy, -CH₂-CH₂-CH₂, -CH₂-O-C(O)-, -CH₂-C(O)-O-,
-CH₂-NH-C(O)-, -CH₂-N(CH₃)-C(O)-, -CH₂C(O)-NH-, -NH (C₁-C₆-alkyl), -N(C₁-C₆-alkyl)₂, -SO₂(C₁-C₆-alkyl), heterocyclyl, heteroaryl and aryl,
and, of these substituents, heterocyclyl, aryl and heteroaryl may in turn be at least monosubstituted by C₁-C₆-alkyl, C₁-C₆-alkoxy, fluorine, chlorine, bromine, trifluoromethyl, trifluoromethoxy or OH;
R" is -COOH, -CHO or -SO₂Cl;
heteroaryl is a 5 to 10-membered, aromatic, mono-or bicyclic heterocycle which comprises one or more heteroatoms selected from N, Oand S;
heterocyclyl is a 5 to 10-membered, nonaromatic, mono- or bicyclic heterocycle which comprises one or more heteroatoms selected from N, O and S;
aryl is a 5 to 10-membered, aromatic mono- or bicycle.

17. A compound as claimed in claim 16, in which the meanings in the general formula (XI) are:
R1 is hydrogen or C₁-C₃-alkyl;
R2 is hydrogen;
Ar is phenyl, furanyl, thienyl or pyridinyl, where R" and the tetrahydroisoquinolinone fragment are in the meta position relative to one another;
R2 is -CHO or -COOH.

## Revendications

1. Composé de formule générale (I) 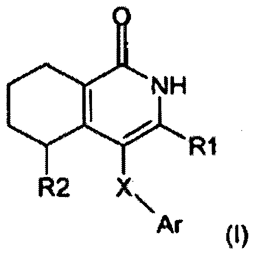 dans laquelle :
X signifie une simple liaison, 0, S, NH ou N(alkyle en C₁-C₃ ) ;
R1 signifie hydrogène, fluor, chlore, -CN, méthoxy, - OCF₃ ou alkyle en C₁-C₃, qui est éventuellement substitué avec hydroxy, chlore, méthoxy ou un, deux ou trois atomes de fluor ;
R2 signifie hydrogène, fluor, -CN, hydroxy, méthoxy, -OCF₃, -NH₂, -NH(alkyle en C₁-C₃), -N(alkyle en C₁-C₃)₂ ou alkyle en C₁-C₃, qui est éventuellement substitué avec hydroxy, chlore, méthoxy ou un, deux ou trois atomes de fluor ;
R3 signifie -(alkyle en C₁-C₃)-NR4R5, -SO₂NR4R5, - C(O)NR4R5, -C(H)=N-OR9, -C(O)R6, -NHC(O)R6, -(alkyle en C₁-C₃)-NHC(O)R6, -NHSO₂R6, -(alkyle en C₁-C₃)-NHSO₂R6 ou -CH(OH)R7 ;
R4 et R5 sont choisis indépendamment l'un de l'autre dans le groupe constitué par : hydrogène ; alkyle en C₁-C₁₀, alcényle en C₂-C₆, alcynyle en C₂-C₆, aryle, hétéroaryle et hétérocyclyle non substitués ou au moins monosubstitués,
les substituants étant choisis dans le groupe constitué par : aryle, hétéroaryle, hétérocyclyle, -0-aryle, fluor, chlore, brome, -CF₃, -OCF₃, -NO₂, -CN, -C(O)R8, - NHC(O)(alkyle en C₁-C₃), -NH₂, hydroxy, alkyle en C₁-C₆, alcoxy en C₁-C₃, -NH (alkyle en C₁-C₃), -N (alkyle en C₁-C₃)₂, -NH-aryle, -NH-hétéroaryle, -NH-C(O)-hétéroaryle, -SO₂NH₂, -SO₂(alkyle en C₁-C₃) et -NH-SO₂(alkyle en C₁-C₃),
et les fragments aryle, hétéroaryle et hétérocyclyle de ces substituants pouvant à leur tour être au moins monosubstitués avec fluor, chlore, brome, oxo, -CF₃, - OCF₃, -NO₂, -CN, aryle, hétéroaryle, -NHC(O)(alkyle en C₁-C₃), -COOH, hydroxy, alkyle en C₁-C₃, alcoxy en C₁-C₃, -SO₂NH₂, -SO₂NH(alkyle en C₁-C₃), -SO₂N (alkyle en C₁-C₃)₂, -C(O)NH₂, -C(O)NH(alkyle en C₁-C₃), -C(O)N(alkyle en C₁-C₃)₂, -SO₂(alkyle en C₁-C₃), -NH₂, -NH (alkyle en C₁-C₃) ou -N (alkyle en C₁-C₃)₂ ; ou
R4 et R5 forment ensemble avec l'atome d'azote auquel ils sont reliés un hétérocyclyle non substitué ou au moins monosubstitué,
les substituants étant choisis dans le groupe constitué par : aryle, hétéroaryle, hétérocyclyle, oxo, fluor, chlore, brome, -CF₃, -OCF₃, -NO₂, -CN, -C(O)R8, - NHC(O)(alkyle en C₁-C₃), -NH₂, hydroxy, alkyle en C₁-C₃, alcoxy en C₁-C₃, -NH (alkyle en C₁-C₃), -N (alkyle en C₁-C₃)₂, -SO₂NH₂, -SO₂(alkyle en C₁-C₃) et -NH-SO₂ (alkyle en C₁-C₃),
et, parmi ces substituants, aryle, hétérocyclyle et hétéroaryle pouvant à leur tour être au moins monosubstitués avec fluor, chlore, brome, hydroxy, alkyle en C₁-C₃ ou alcoxy en C₁-C₃ ;
R6 signifie alkyle en C₁-C₆, phényle, hétéroaryle ou hétérocyclyle non substitué ou au moins monosubstitué,
les substituants étant choisis dans le groupe constitué par : fluor, chlore, brome, aryle, hétérocyclyle, hétéroaryle, -CF₃, -OCF₃, -NO₂, -CN, -C(O)R8, - NHC(O)(alkyle en C₁-C₃), -NH₂, hydroxy, alkyle en C₁-C₃, alcoxy en C₁-C₃, -0-hétéroaryle, -0-aryle, -NH(alkyle en C₁-C₃), -N (alkyle en C₁-C₃)₂, -SO₂NH₂, -SO₂(alkyle en C₁-C₃) et -NH-SO₂(alkyle en C₁-C₃),
et les fragments aryle, hétérocyclyle et hétéroaryle de ces substituants pouvant à leur tour être au moins monosubstitués avec fluor, chlore, brome, hydroxy, alkyle en C₁-C₃ ou alcoxy en C₁-C₃ ;
R7 est choisi dans le groupe constitué par : hydrogène ; alkyle en C₁-C₆, phényle et hétéroaryle non substitués ou au moins monosubstitués,
les substituants étant choisis dans le groupe constitué par : fluor, chlore, brome, -CF₃, -OCF₃, -NO₂, -CN, - C(O)R8, -NHC(O)(alkyle en C₁-C₃), -NH₂, hydroxy, alkyle en C₁-C₃, alcoxy en C₁-C₃, -NH (alkyle en C₁-C₃), - N (alkyle en C₁-C₃)₂, -SO₂NH₂, -SO₂(alkyle en C₁-C₃) et - NH-SO₂(alkyle en C₁-C₃) ;
R8 signifie alcoxy en C₁-C₃, -O-phényle, alkyle en C₁-C₃, -NH₂, -NH (alkyle en C₁-C₃), -N (alkyle en C₁-C₃)₂ ou phényle,
les fragments phényle précédents pouvant à leur tour être au moins monosubstitués avec fluor, chlore, brome, oxo, -CF₃, -OCF₃, -NO₂, -CN, aryle, hétéroaryle, - NHC(O) (alkyle en C₁-C₃), -COOH, hydroxy, alkyle en C₁-C₃, alcoxy en C₁-C₃, -SO₂NH₂, -SO₂NH (alkyle en C₁-C₃), - SO₂N(alkyle en C₁-C₃)₂, -C(O)NH₂, -C(O)NH(alkyle en C₁-C₃), -C(O)N(alkyle en C₁-C₃)₂, -SO₂(alkyle en C₁-C₃), - NH₂, -NH (alkyle en C₁-C₃) ou -N (alkyle en C₁-C₃)₂ ;
R9 est choisi dans le groupe constitué par :
hydrogène ; alkyle en C₁-C₆ et phényle non substitués ou au moins monosubstitués,
les substituants étant choisis dans le groupe constitué par : fluor, chlore, brome, aryle, hétérocyclyle, hétéroaryle, -CF₃, -OCF₃, -NO₂, -CN, -C(O)R8, - NHC(O)(alkyle en C₁-C₃), alkyle en C₁-C₃, alcoxy en C₁-C₃, -NH(alkyle en C₁-C₃), -N(alkyle en C₁-C₃)₂, -SO₂NH₂, - SO₂(alkyle en C₁-C₃) et -NH-SO₂(alkyle en C₁-C₃),
et, parmi ces substituants, aryle, hétérocyclyle et hétéroaryle pouvant à leur tour être au moins monosubstitués avec fluor, chlore, brome, alkyle en C₁-C₃ ou alcoxy en C₁-C₃ ;
Ar signifie aryle ou hétéroaryle non substitué ou au moins monosubstitué,
les substituants étant choisis dans le groupe constitué par : fluor, chlore, brome, -CF₃, -OCF₃, -NO₂, -CN, - C(O)R8, -NH₂, -NHC(O)(alkyle en C₁-C₆), hydroxy, alkyle en C₁-C₆, alcoxy en C₁-C₆, -CH₂-CH₂-CH₂-, -CH₂-O-C(O)-, - CH₂-C(O)-O-, -CH₂-NH-C(O)-, -CH₂-N(CH₃)-C(O)-, -CH₂-C(O)-NH-, -NH (alkyle en C₁-C₆), -N (alkyle en C₁-C₆)₂, - SO₂(alkyle en C₁-C₆), hétérocyclyle, hétéroaryle, aryle et R3,
et, parmi ces substituants, hétérocyclyle, aryle et hétéroaryle pouvant à leur tour être au moins monosubstitués avec alkyle en C₁-C₆, alcoxy en C₁-C₆, fluor, chlore, brome, trifluorométhyle, trifluorométhoxy ou OH ;
hétéroaryle signifie un hétérocycle mono- ou bicyclique aromatique de 5 à 10 éléments, qui contient un ou plusieurs hétéroatomes choisis parmi N, 0 et S ; aryle signifie un mono- ou bicycle aromatique de 5 à 10 éléments,
hétérocyclyle signifie un hétérocycle mono- ou bicyclique non aromatique de 5 à 10 éléments, qui contient un ou plusieurs hétéroatomes choisis parmi N, O et S ;
ou un de ses sels physiologiquement acceptables ;
à condition qu'Ar ne signifie pas phényle non substitué lorsque X est une simple liaison, et également à condition que le composé 3-(3-méthyl-1-oxo-1,2,5,6,7,8-hexahydroisoquinolin-4-yl)benzonitrile soit exclu.

2. Composé de formule générale (I) selon la revendication 1, dans lequel R1, R2, R3 et X ont les significations données dans la revendication 1, et
Ar signifie phényle ou hétéroaryle non substitué ou au moins monosubstitué,
les substituants étant choisis dans le groupe constitué par : fluor, chlore, -CF₃, -OCF₃, -C(O)(alkyle en C₁-C₃), -NH₂, -NHC(O)(alkyle en C₁-C₃), hydroxy, alkyle en C₁-C₃, alcoxy en C₁-C₃, -CH₂-CH₂-CH₂-, -CH₂-O-C(O)-, -CH₂-C(O)-O-, -CH₂-NH-C(O)-, -CH₂-N (CH₃) -C(O)-, -CH₂-C (0) -NH-, -NH(alkyle en C₁-C₃), -N(alkyle en C₁-C₃)₂, - SO₂(alkyle en C₁-C₃), hétérocyclyle, hétéroaryle et R3, et, parmi ces substituants, hétérocyclyle et hétéroaryle pouvant à leur tour être au moins monosubstitués avec alkyle en C₁-C₃, alcoxy en C₁-C₃, fluor, chlore, brome, trifluorométhyle, trifluorométhoxy ou OH ;
ou un de ses sels physiologiquement acceptables ;
à condition qu'Ar ne signifie pas phényle non substitué lorsque X est une simple liaison.

3. Composé selon la revendication 1 ou 2, dans lequel, dans la formule générale (I) _{:}
X signifie une simple liaison, NH ou N(alkyle en C₁-C₃) ;
R1 signifie hydrogène ou alkyle en C₁-C₃, qui est éventuellement substitué avec hydroxy, chlore, méthoxy ou un, deux ou trois atomes de fluor ;
R2 signifie hydrogène, fluor, -OCF₃, hydroxy, méthoxy, NH₂ ou alkyle en C₁-C₃ ;
R3 signifie -CH₂-NR4R5, -SO₂NR4R5, -C(O)NR4R5, -CH₂-NHC(O)R6, -CH₂-NHSO₂R6 ou -CH(OH)R7 ;
R4 et R5 sont choisis indépendamment l'un de l'autre dans le groupe constitué par : hydrogène ; alkyle en C₁-C₁₀, alcényle en C₂-C₆, phényle, indanyle, hétérocyclyle et hétéroaryle non substitués ou au moins monosubstitués,
les substituants étant choisis dans le groupe constitué par : phényle, hétéroaryle, hétérocyclyle, -0-phényle, fluor, -CN, -C(O)NH₂, -C(O) (alkyle en C₁-C₃), -C(O)-phényle, -N (alkyle en C₁-C₃)₂, -NH (alkyle en C₁-C₃), - NH₂, -NH-hétéroaryle, -NH-C(O)-hétéroaryle, alkyle en C₁-C₆, alcoxy en C₁-C₃ et hydroxy,
et les fragments phényle, hétérocyclyle et hétéroaryle de ces substituants pouvant à leur tour être au moins monosubstitués avec fluor, chlore, brome, oxo, -CF₃, - OCF₃, -NO₂, -CN, phényle, pyridinyle, -NHC(O)(alkyle en C₁-C₃), -COOH, hydroxy, alkyle en C₁-C₃, alcoxy en C₁-C₃, -SO₂NH₂, -SO₂NH(alkyle en C₁-C₃), -SO₂N (alkyle en C₁-C₃)₂, -C(O)NH₂, -C(O)NH(alkyle en C₁-C₃), -C(O)N(alkyle en C₁-C₃)₂, -SO₂(alkyle en C₁-C₃), -NH₂, -NH (alkyle en C₁-C₃) ou -N(alkyle en C₁-C₃)₂ ; ou
R4 et R5 forment ensemble avec l'atome d'azote auquel ils sont reliés un hétérocyclyle non substitué ou au moins monosubstitué,
les substituants étant choisis dans le groupe constitué par : phényle, hétéroaryle, hétérocyclyle, oxo, fluor, chlore, -C(O) (alkyle en C₁-C₃), -C(O)-phényle et hydroxy,
et les fragments phényle, hétérocyclyle et hétéroaryle de ces substituants pouvant à leur tour être au moins monosubstitués avec fluor ou alkyle en C₁-C₃ ;
R6 signifie alkyle en C₁-C₆, phényle ou hétéroaryle non substitué ou au moins monosubstitué,
les substituants étant choisis dans le groupe constitué par : fluor, chlore, brome, -CF₃, -OCF₃, -NHC(O)(alkyle en C₁-C₃), hydroxy, alkyle en C₁-C₃, alcoxy en C₁-C₃, -0-hétéroaryle, phényle, -NH₂, -NH(alkyle en C₁-C₃), - N(alkyle en C₁-C₃) et hétérocyclyle,
et les fragments phényle, hétéroaryle et hétérocyclyle de ces substituants pouvant à leur tour être au moins monosubstitués avec fluor, chlore, brome, hydroxy, alkyle en C₁-C₃ ou alcoxy en C₁-C₃ ;
R7 est choisi dans le groupe constitué par :
hydrogène ; alkyle en C₁-C₆, phényle et pyridinyle non substitués ou au moins monosubstitués,
les substituants étant choisis dans le groupe constitué par : fluor, chlore, brome, hydroxy, alkyle en C₁-C₃ et alcoxy en C₁-C₃ ;
Ar signifie phényle ou hétéroaryle non substitué ou au moins monosubstitué,
les substituants étant choisis dans le groupe constitué par : fluor, chlore, -CF₃, -OCF₃, -C(O)(alkyle en C₁-C₃), -NH₂, -NHC(O) (alkyle en C₁-C₃), hydroxy, alkyle en C₁-C₃, alcoxy en C₁-C₃, -CH₂-CH₂-CH₂-, -CH₂-O-C(O) -, -CH₂-C(O)-O-, -CH₂-NH-C(O)-, -CH₂-N (CH₃) -C(O) -, -CH₂-C(O)-NH-, -NH(alkyle en C₁-C₃), -N(alkyle en C₁-C₃)₂, - SO₂(alkyle en C₁-C₃), hétérocyclyle, hétéroaryle et R3, et, parmi ces substituants, hétérocyclyle et hétéroaryle pouvant à leur tour être au moins monosubstitués avec alkyle en C₁-C₃, alcoxy en C₁-C₃, fluor, chlore, brome, trifluorométhyle, trifluorométhoxy ou OH ;
hétéroaryle signifie pyridinyle, thiényle, pyrimidinyle, imidazolyle, furanyle, indolyle, benzimidazolyle, pyrazolyle, 1,3-benzodioxolyle, triazolyle, thiazolyle, isoxazolyle, pyrrolyle, pyrazinyle, oxazolyle, pyridazinyle, quinolinyle, isoquinolyle, benzofuranyle, 3-oxo-1,3-dihydroisobenzofuranyle ou 4,5,6,7-tétrahydrobenzothiazolyle ;
hétérocyclyle signifie morpholinyle, pyrrolidinyle, pipéridinyle, tétrahydropyranyle, thiazolidinyle, dihydroisoxazolyle, pipérazinyle ou tétrahydrofuranyle ;
ou un de ses sels physiologiquement acceptables ;
à condition qu'Ar ne signifie pas phényle non substitué lorsque X est une simple liaison.

4. Composé selon les revendications 1 à 3, dans lequel, dans la formule générale (I) _{:}
X signifie une simple liaison, NH ou N(alkyle en C₁-C₃) ;
R1 signifie hydrogène ou alkyle en C₁-C₃, qui est éventuellement substitué avec hydroxy, chlore, méthoxy ou un, deux ou trois atomes de fluor ;
R2 signifie hydrogène, fluor, -OCF₃, hydroxy, méthoxy, -NH₂ ou alkyle en C₁-C₃ ;
R3 signifie -CH₂-NR4R5, -SO₂NR4R5, -C(O)NR4R5, -CH₂-NHC(O)R6, -CH₂NHSO₂R6 ou -CH(OH)R7 ;
R4 et R5 sont choisis indépendamment l'un de l'autre dans le groupe constitué par : hydrogène ; alkyle en C₁-C₁₀, alcényle en C₂-C₆, phényle, indanyle, hétérocyclyle et hétéroaryle non substitués ou au moins monosubstitués,
les substituants étant choisis dans le groupe constitué par : phényle, hétéroaryle, hétérocyclyle, -0-phényle, fluor, -CN, -C(O)NH₂, -C(O)(alkyle en C₁-C₃), -C(O)-phényle, -N(alkyle en C₁-C₃)₂, -NH(alkyle en C₁-C₃), - NH₂, -NH-hétéroaryle, -NH-C(O)-hétéroaryle, alkyle en C₁-C₆, alcoxy en C₁-C₃ et hydroxy,
et les fragments phényle, hétérocyclyle et hétéroaryle de ces substituants pouvant à leur tour être au moins monosubstitués avec fluor, chlore, brome, oxo, -CF₃, - OCF₃, -NO₂, -CN, phényle, pyridinyle, -NHC(O)(alkyle en C₁-C₃) , -COOH, hydroxy, alkyle en C₁-C₃, alcoxy en C₁-C₃, -SO₂NH₂, -SO₂NH(alkyle en C₁-C₃), -SO₂N(alkyle en C₁-C₃)₂, -C(O)NH₂, -C(O)NH(alkyle en C₁-C₃), -C(O)N(alkyle en C₁-C₃)₂, -SO₂(alkyle en C₁-C₃), -NH₂, -NH(alkyle en C₁-C₃) ou -N(alkyle en C₁-C₃)₂ ; ou
R4 et R5 forment ensemble avec l'atome d'azote auquel ils sont reliés un hétérocyclyle non substitué ou au moins monosubstitué,
les substituants étant choisis dans le groupe constitué par : phényle, hétéroaryle, hétérocyclyle, oxo, fluor, chlore, -C(O)(alkyle en C₁-C₃), -C(O)-phényle et hydroxy,
et les fragments phényle, hétérocyclyle et hétéroaryle de ces substituants pouvant à leur tour être au moins monosubstitués avec fluor ou alkyle en C₁-C₃ ;
R6 signifie CF₃ ; ou alkyle en C₁-C₆, pyridinyle, furanyle ou phényle non substitué ou au moins monosubstitué,
les substituants étant choisis dans le groupe constitué par : fluor, -NHC(O)(alkyle en C₁-C₃), hydroxy, alkyle en C₁-C₃, alcoxy en C₁-C₃ et -O-pyridinyle ;
R7 est choisi dans le groupe constitué par :
hydrogène ; alkyle en C₁-C₆, phényle et pyridinyle non substitués ou au moins monosubstitués,
les substituants étant choisis dans le groupe constitué par : fluor, chlore, brome, hydroxy, alkyle en C₁-C₃ et alcoxy en C₁-C₃ ;
Ar signifie phényle, thiényle, furanyle ou pyridinyle non substitué ou au moins monosubstitué,
les substituants étant choisis dans le groupe constitué par : fluor, chlore, -CF₃, -OCF₃, -C(O)(alkyle en C₁-C₃), -NH₂, -NHC(O)(alkyle en C₁-C₃), hydroxy, alkyle en C₁-C₃, alcoxy en C₁-C₃, -CH₂-CH₂-CH₂-, -CH₂-O-C(O)-, -CH₂-C(O)-O-, -CH₂-NH-C(O)-, -CH₂-N(CH₃)-C(O)-, -CH₂-C(O)-NH-, -NH(alkyle en C₁-C₃), -N(alkyle en C₁-C₃)₂, - SO₂(alkyle en C₁-C₃) et R3 ;
hétéroaryle signifie pyridinyle, thiényle, pyrimidinyle, imidazolyle, furanyle, indolyle, benzimidazolyle, pyrazolyle, 1,3-benzodioxolyle, triazolyle, thiazolyle, isoxazolyle, pyrrolyle, pyrazinyle, oxazolyle, pyridazinyle, quinolinyle, isoquinolyle, benzofuranyle, 3-oxo-1,3-dihydroisobenzofuranyle ou 4,5,6,7-tétrahydrobenzothiazolyle ;
hétérocyclyle signifie morpholinyle, pyrrolidinyle, pipéridinyle, tétrahydropyranyle, thiazolidinyle, dihydroisoxazolyle, pipérazinyle ou tétrahydrofuranyle ;
ou un de ses sels physiologiquement acceptables ;
à condition qu'Ar ne signifie pas phényle non substitué lorsque X est une simple liaison.

5. Composé selon l'une quelconque des revendications 1 à 4, dans lequel, dans la formule générale (I) _{:}
X signifie une simple liaison, NH ou N(alkyle en C₁-C₃) ;
R1 signifie hydrogène ou alkyle en C₁-C₃ ;
R2 signifie hydrogène ;
R3 signifie -CH₂-NR4R5, -CH₂-NHC(O)R6, -CH₂-NHSO₂R6, - C(O)NR4R5 ou -CH(OH)R7 ;
R4 est choisi dans le groupe constitué par : hydrogène ; alkyle en C₁-C₁₀, alcényle en C₁-C₆, phényle, indanyle, hétérocyclyle et hétéroaryle non substitués ou au moins monosubstitués,
les substituants étant choisis dans le groupe constitué par : phényle, hétéroaryle, hétérocyclyle, -O-phényle, fluor, -CN, -C(O)NH₂, -C(O)(alkyle en C₁-C₃), -C(O)-phényle, -N(alkyle en C₁-C₃)₂, -NH(alkyle en C₁-C₃), - NH₂, -NH-hétéroaryle, -NH-C(O)-hétéroaryle, alkyle en C₁-C₆, alcoxy en C₁-C₃ et hydroxy,
et les fragments phényle, hétérocyclyle et hétéroaryle de ces substituants pouvant à leur tour être au moins monosubstitués avec fluor, chlore, brome, oxo, -CF₃, - OCF₃, -NO₂, -CN, phényle, pyridinyle, -NHC(O)(alkyle en C₁-C₃), -COOH, hydroxy, alkyle en C₁-C₃, alcoxy en C₁-C₃, -SO₂NH₂, -SO₂NH(alkyle en C₁-C₃), -SO₂N(alkyle en C₁-C₃)₂, -C(O)NH₂, -C(O)NH(alkyle en C₁-C₃), -C(O)N(alkyle en C₁-C₃)₂, -SO₂(alkyle en C₁-C₃), -NH₂, -NH (alkyle en C₁-C₃) ou -N(alkyle en C₁-C₃)₂ ; et R5 signifie hydrogène ou R4 et R5 forment ensemble avec l'atome d'azote auquel ils sont reliés un hétérocyclyle non substitué ou au moins monosubstitué,
les substituants étant choisis dans le groupe constitué par : phényle, hétéroaryle, hétérocyclyle, oxo, fluor, chlore, -C(O)(alkyle en C₁-C₃), -C(O)-phényle et hydroxy,
et les fragments phényle, hétérocyclyle et hétéroaryle de ces substituants pouvant à leur tour être au moins monosubstitués avec fluor ou alkyle en C₁-C₃ ;
R6 signifie CF₃ ; ou alkyle en C₁-C₆, pyridinyle, furanyle ou phényle non substitué ou au moins monosubstitué,
les substituants étant choisis dans le groupe constitué par : fluor, -NHC(O)(alkyle en C₁-C₃), hydroxy, alkyle en C₁-C₃, alcoxy en C₁-C₃ et -O-pyridinyle ;
R7 est choisi dans le groupe constitué par :
hydrogène ; alkyle en C₁-C₆, phényle et pyridinyle non substitués ou au moins monosubstitués,
les substituants étant choisis dans le groupe constitué par : fluor, chlore, brome, hydroxy, alkyle en C₁-C₃ et alcoxy en C₁-C₃ ;
Ar signifie phényle, thiényle, furanyle ou pyridinyle monosubstitué,
le substituant étant choisi dans le groupe constitué par : fluor, chlore, -CF₃, -OCF₃, -C(O)(alkyle en C₁-C₃), -NH₂, -NHC(O) (alkyle en C₁-C₃), hydroxy, alkyle en C₁-C₃, alcoxy en C₁-C₃, -NH (alkyle en C₁-C₃), -N (alkyle en C₁-C₃)₂, -SO₂(alkyle en C₁-C₃) et R3 ;
et le substituant et X se trouvant en position méta l'un par rapport à l'autre ;
hétéroaryle signifie pyridinyle, thiényle, pyrimidinyle, imidazolyle, furanyle, benzimidazolyle, pyrazolyle, thiazolyle, isoxazolyle, pyrrolyle, pyrazinyle, 3-oxo-1,3-dihydroisobenzofuranyle ou 4,5,6,7-tétrahydrobenzothiazolyle ;
hétérocyclyle signifie morpholinyle, pyrrolidinyle, pipéridinyle, tétrahydropyranyle, thiazolidinyle, dihydroisoxazolyle, pipérazinyle ou tétrahydrofuranyle ;
ou un de ses sels physiologiquement acceptables.

6. Composé selon l'une quelconque des revendications 1 à 5, dans lequel, dans la formule générale (I) _{:}
X signifie une simple liaison ou NH ;
R1 signifie hydrogène ou alkyle en C₁-C₃ ;
R2 signifie hydrogène ;
R3 signifie -CH₂-NR4R5, -C(O)NR4R5 ou -CH(OH)R7 ;
R4 est choisi dans le groupe constitué par : hydrogène ; alkyle en C₁-C₁₀, cyclohexényle, indanyle, phényle, pyrrolidinyle, pyrrolyle, pyrazolyle, furanyle et pipéridinyle non substitués ou au moins monosubstitués,
les substituants étant choisis dans le groupe constitué par : fluor, -CN, -C(O)NH₂, -0-phényle, -C(O)-phényle, - N(CH₃)₂, alkyle en C₁-C₃, alcoxy en C₁-C₃, hydroxy ; phényle, pyridinyle thiényle, pyrimidinyle, imidazolyle, furanyle, indolyle, benzimidazolyle, pyrazolyle, morpholinyle, pyrrolidinyle, 1,3-benzodioxolyle, pipéridinyle, tétrahydropyranyle, triazolyle, thiazolyle, thiazolidinyle, isoxazolyle et dihydroisoxazolyle non substitués ou au moins monosubstitués, dont les substituants sont à leur tour choisis dans le groupe constitué par : fluor, chlore, oxo, CF₃, -OCF₃, -NO₂, phényle, pyridinyle, -NHC(O)CH₃, - COOH, hydroxy, alkyle en C₁-C₃, alcoxy en C₁-C₃, -SO₂NH₂, -C(O)NH₂ et -N(CH₃)₂ ; et R5 signifie hydrogène ou
R4 et R5 forment ensemble avec l'atome d'azote auquel ils sont reliés un radical choisi dans le groupe constitué par : pipéridinyle, pyrrolidinyle, morpholinyle et pipérazinyle non substitués ou au moins monosubstitués,
les substituants étant choisis dans le groupe constitué par : fluor, -C(O) (alkyle en C₁-C₃), oxo, alkyle en C₁-C₃, hydroxy ; phényle, imidazolyle, pyridinyle, pyrimidinyle, pipéridinyle et pyrrolidinyle non substitués ou au moins monosubstitués, dont les substituants sont à leur tour fluor ou alkyle en C₁-C₃ ;
R7 signifie hydrogène ;
Ar signifie phényle, thiényle, furanyle ou pyridinyle monosubstitué,
le substituant étant choisi dans le groupe constitué par : fluor, chlore, -CF₃, -OCF₃, -C(O)(alkyle en C₁-C₃), -NH₂, -NHC(O) (alkyle en C₁-C₃), hydroxy, alkyle en C₁-C₃, alcoxy en C₁-C₃, -NH (alkyle en C₁-C₃), -N (alkyle en C₁-C₃)₂, -SO₂(alkyle en C₁-C₃) et R3,
et le substituant et X se trouvant en position méta l'un par rapport à l'autre ;
ou un de ses sels physiologiquement acceptables.

7. Composé selon l'une quelconque des revendications 1 à 6, dans lequel, dans la formule générale (I) _{:}
X signifie une simple liaison ou NH ;
R1 signifie hydrogène ou alkyle en C₁-C₃ ;
R2 signifie hydrogène ;
R3 signifie -CH₂-NR4R5 ;
R4 signifie alkyle en C₁-C₆ non substitué ou au moins monosubstitué,
les substituants étant choisis dans le groupe constitué par : -N(CH₃)₂, hydroxy ; phényle, pyridinyle, imidazolyle, indolyle, benzimidazolyle, pyrazolyle et pyrrolidinyle non substitués ou au moins monosubstitués, dont les substituants sont à leur tour choisis dans le groupe constitué par : -NHC(O)CH₃, alkyle en C₁-C₃, alcoxy en C₁-C₃, -SO₂NH₂ et -C(O)NH₂ ; et
R5 signifie hydrogène ; ou
R4 et R5 forment ensemble avec l'atome d'azote auquel ils sont reliés un radical choisi dans le groupe constitué par :
pyrrolidinyle, pipéridinyle et pipérazinyle non substitués ou au moins monosubstitués,
les substituants étant choisis dans le groupe constitué par : alkyle en C₁-C₃, hydroxy et pyrrolidinyle ;
Ar signifie phényle, thiényle, furanyle ou pyridinyle monosubstitué,
le substituant étant choisi dans le groupe constitué par : fluor, chlore, -OCF₃, -C(O)CH₃, -NHC(O)CH₃, hydroxy, -N(CH₃)_{2,} éthoxy, -SO₂CH₃ et R3,
et le substituant et X se trouvant en position méta l'un par rapport à l'autre ;
ou un de ses sels physiologiquement acceptables.

8. Composé selon l'une quelconque des revendications 1 à 6, choisi dans le groupe constitué par :
la 4-(3-méthanesulfonyl-phénylamino)-3-méthyl-5,6,7,8-tétrahydro-2H-isoquinolin-1-one, la 4-(3-acétyl-phénylamino)-3-méthyl-5,6,7,8-tétrahydro-2H-isoquinolin-1-one, la 4-(5-butylaminométhyl-furan-2-yl)-3-méthyl-5,6,7,8-tétrahydro-2H-isoquinolin-1-one, la 3-méthyl-4-(5-pyrrolidin-1-ylméthyl-furan-2-yl)-5,6,7,8-tétrahydro-2H-isoquinolin-1-one, la 4-[5-(3-hydroxy-pyrrolidin-1-ylméthyl)-furan-2-yl]-5,6,7,8-tétrahydro-2H-isoquinolin-1-one, la 4-(5-{[(4-pyrrolidin-1-yl-pipéridin-1-ylméthyl)-amino]-méthyl}-furan-2-yl)-5,6,7,8-tétrahydro-2H-isoquinolin-1-one, la 4-{5-[(2-diméthylamino-éthylamino)-méthyl]-furan-2-yl}-5,6,7,8-tétrahydro-2H-isoquinolin-1-one, la 4-{5-[(2-hydroxy-2-phényl-éthylamino)-méthyl]-furan-2-yl}-5,6,7,8-tétrahydro-2H-isoquinolin-1-one, la 4-(5-{[(4-méthyl-pipérazin-1-ylméthyl)-amino]-méthyl}-furan-2-yl)-5,6,7,8-tétrahydro-2H-isoquinolin-1-one, la 4-(5-{[(1-méthyl-1H-pyrazol-4-ylméthyl)-amino]-méthyl}-furan-2-yl)-5,6,7,8-tétrahydro-2H-isoquinolin-1-one, la 4-(5-butylaminométhyl-furan-2-yl)-5,6,7,8-tétrahydro-2H-isoquinolin-1-one et la 4-(5-hydroxyméthyl-furan-2-yl)-3-méthyl-5,6,7,8-tétrahydro-2H-isoquinolin-1-one ;
ou un de leurs sels physiologiquement acceptables.

9. Composé selon l'une quelconque des revendications 1 à 8 ou un de ses sels physiologiquement acceptables destiné à être utilisé en tant que médicament.

10. Utilisation d'un composé selon l'une quelconque des revendications 1 à 8 ou d'un de ses sels physiologiquement acceptables pour la fabrication d'un médicament pour la prophylaxie et/ou le traitement d'une maladie choisie dans le groupe constitué par : les lésions tissulaires résultant de l'endommagement de cellules ou de la mort de cellules en raison d'une nécrose ou d'une apoptose, les lésions tissulaires ou les maladies à médiation neuronale, l'ischémie cérébrale, le traumatisme crânien, l'accident cérébral, les lésions de reperfusion, les troubles neurologiques et les maladies neurodégénératives, l'accident cérébral vasculaire, les troubles cardiovasculaires, l'infarctus du myocarde, l'ischémie du myocarde, l'encéphalomyélite allergique expérimentale (EAE), la sclérose en plaques (MS), l'ischémie relative à une chirurgie cardiaque, la dégénération maculaire liée à l'âge, l'arthrite, l'artérosclérose, le cancer, les maladies dégénératives du muscle squelettique avec sénescence réplicative consécutive, le diabète et les maladies diabétique du muscle cardiaque.

11. Utilisation selon la revendication 10, **caractérisée en ce que** la maladie est choisie dans le groupe constitué par : l'ischémie cérébrale, les lésions de reperfusion, les troubles cardiovasculaires, l'infarctus du myocarde, l'ischémie du myocarde et l'ischémie relative à une chirurgie cardiaque.

12. Utilisation selon la revendication 10 ou 11, **caractérisée en ce que** la maladie est l'infarctus du myocarde.

13. Composition pharmaceutique comprenant une quantité efficace d'au moins un composé ou d'un de ses sels physiologiquement acceptables selon l'une quelconque des revendications 1 à 8 et un véhicule physiologiquement acceptable.

14. Composition pharmaceutique selon la revendication 13, dans laquelle la composition pharmaceutique se présente sous la forme d'une pilule, d'un comprimé, d'un comprimé enrobé, d'une pastille à sucer, d'un granulat, d'une gélule, d'une gélule de gélatine dure ou souple, de solutions aqueuses, d'une solution alcoolique, d'une solution huileuse, d'un sirop, d'une émulsion, d'une suspension, d'une pastille, d'une solution pour injection ou perfusion, d'un onguent, d'une teinture, d'une crème, d'une lotion, d'une poudre, d'un spray, d'un système thérapeutique transdermique, d'un spray nasal, d'un aérosol, d'un mélange aérosol, d'une microgélule, d'un implant, d'une baguette ou d'un pansement.

15. Procédé de fabrication d'un composé de formule (I) selon l'une quelconque des revendications 1 à 8, selon lequel, à condition que, dans la formule (I), X représente une simple liaison et le substituant Ar soit au moins monosubstitué avec R3 (Formule Ib) 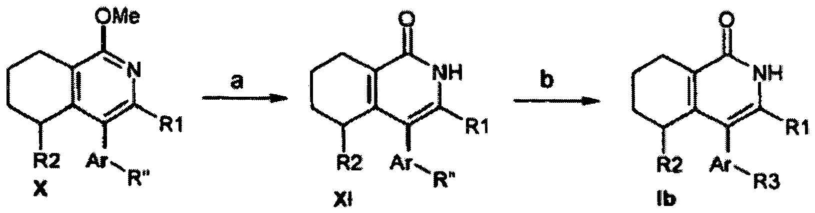
a) un composé de formule (X) est mis en réaction avec un réactif approprié avec clivage du groupe méthyle pour former le composé de formule (XI) et
b) le composé de formule (XI)
1. est mis en réaction avec une amine appropriée en présence d'un réducteur lorsque R'' = -CHO et R3 = - CH₂NR4R5, ou
2. est réduit avec un réducteur approprié lorsque R'' = -CHO et R3 = -CH₂OH, ou
3. est mis en réaction par amination réductrice avec de l'acétate d'ammonium avec une réaction de couplage ultérieure avec un chlorure d'acide approprié, un acide approprié ou un chlorure de sulfonyle approprié lorsque R'' = -CHO et R3 = -CH₂NHC(O)R6 ou - CH₂NHSO₂R6, ou
4. est mis en réaction avec une amine appropriée en présence d'un agent de condensation lorsque R'' = - COOH et R3 = -C(O)NR4R5.

16. Composé de formule générale (XI) 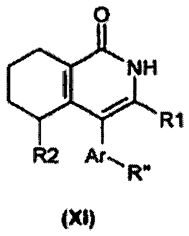 dans lequel
R1 signifie hydrogène, fluor, chlore, -CN, méthoxy, - OCF₃ ou alkyle en C₁-C₃, qui est éventuellement substitué avec hydroxy, chlore, méthoxy ou un, deux ou trois atomes de fluor ;
R2 signifie hydrogène, fluor, -CN, hydroxy, méthoxy, -OCF₃, -NH₂, -NH(alkyle en C₁-C₃), -N(alkyle en C₁-C₃)₂ ou alkyle en C₁-C₃, qui est éventuellement substitué avec hydroxy, chlore, méthoxy ou un, deux ou trois atomes de fluor ;
R8 signifie alcoxy en C₁-C₃, -0-phényle, alkyle en C₁-C₃, -NH₂, -NH (alkyle en C₁-C₃), -N (alkyle en C₁-C₃)₂ ou phényle,
les fragments phényle précédents pouvant à leur tour être au moins monosubstitués avec fluor, chlore, brome, oxo, -CF₃, -OCF₃, -NO₂, -CN, -aryle, hétéroaryle, - NHC(O)(alkyle en C₁-C₃), -COOH, hydroxy, alkyle en C₁-C₃, alcoxy en C₁-C₃, -SO₂NH₂, -SO₂NH (alkyle en C₁-C₃), - SO₂N(alkyle en C₁-C₃)_{2,} -C(O)NH₂, -C(O)NH(alkyle en C₁-C₃), -C(O)N(alkyle en C₁-C₃)₂, -SO₂(alkyle en C₁-C₃), - NH₂, -NH(alkyle en C₁-C₃) ou -N(alkyle en C₁-C₃)₂ ;
Ar signifie aryle ou hétéroaryle,
cet aryle ou hétéroaryle étant éventuellement substitué avec au moins un substituant choisi dans le groupe constitué par : fluor, chlore, brome, -CF₃, -OCF₃, -NO₂, -CN, -C(O)R8, -NH₂, -NHC(O) (alkyle en C₁-C₆), hydroxy, alkyle en C₁-C₆, alcoxy en C₁-C₆, -CH₂-CH₂-CH₂-, -CH₂-OC(O)-, -CH₂-C(O)-O-, -CH₂-NH-C(O)-, -CH₂-N(CH3)-C(O)-, - CH₂-C(O)-NH-, -NH(alkyle en C₁-C₆), -N(alkyle en C₁-C₆)₂, -SO₂(alkyle en C₁-C₆), hétérocyclyle, hétéroaryle et aryle,
et, parmi ces substituants, hétérocyclyle, aryle et hétéroaryle pouvant à leur tour être au moins monosubstitués avec alkyle en C₁-C₆, alcoxy en C₁-C₆, fluor, chlore, brome, trifluorométhyle, trifluorométhoxy ou OH ;
R" signifie -COOH, -CHO ou -SO₂Cl ;
hétéroaryle signifie un hétérocycle mono- ou bicyclique aromatique de 5 à 10 éléments, qui contient un ou plusieurs hétéroatomes choisis parmi N, O et S ; hétérocyclyle signifie un hétérocycle mono- ou bicyclique non aromatique de 5 à 10 éléments, qui contient un ou plusieurs hétéroatomes choisis parmi N, O et S ;
aryle signifie un mono- ou bicycle aromatique de 5 à 10 éléments.

17. Composé selon la revendication 16, dans lequel, dans la formule générale (XI) _{:}
R1 signifie hydrogène ou alkyle en C₁-C₃ ;
R2 signifie hydrogène ;
Ar signifie phényle, furanyle, thiényle ou pyridinyle, R'' et le fragment tétrahydroisoquinolinone se trouvant en position méta l'un par rapport à l'autre ;
R" signifie -CHO ou -COOH.
